# EUROPEAN PATENT APPLICATION

(11) **EP 4 818 337 A2**
(43) Date of publication of application: **07.10.2026**
(21) Application number: 26193622.3
(22) Date of filing: 31.05.2024
(51) Int. Cl.: C12R 1/01

(54) **BIFIDOBACTERIUM LONGUM TRANSITIONAL MICROORGANISM**

(30) Priority: 02.06.2023 EP 23177101
(62) Document number(s) of the earlier application(s) in accordance with Art. 76 EPC: 24733105.1 / 4 720 255
(71) Applicant: Société des Produits Nestlé S.A., 1800 Vevey (CH)
(72) Inventor: DUBOUX, Stéphane, 1162 St-Prex (CH); SAKWINSKA, Olga, 1009 Pully (CH); DELANNOY-BRUNO, Omar, 1007 Lausanne (CH); NGOM-BRU, Catherine Anne-Brigitte, 1083 Mézières (CH); SIEGWALD, Léa, 1066 Epalinges (CH)
(74) Representative: Ng, Yen Kar

(57) **Abstract**

The present invention relates to a *Bifidobacterium longum* transitional microorganism strain deposited with CNCM under number CNCM I-5942 or a *B*. *Longum* transitional strain having an identifying characteristic of the *B*. *Longum* transitional strain deposited under deposit number CNCM I-5942.

## Description

### FIELD OF THE INVENTION

The present invention relates to a *Bifidobacterium longum* transitional strain.

### BACKGROUND TO THE INVENTION

Nutrition plays a critical role in the development across all areas (including cognitive, motor sensory, dentition, musculo-skeletal, immunity, and social development) in infants and young children. Further, the gastrointestinal or "gut" microbiome during infancy can play a significant role in the health and development of the infant both during infancy and later on in life (see e.g., Tanaka and Nakayama. 2017. Allergol. Int. 66(4): 515-522). Various factors, including diet, can significantly influence the microbiome structure and thus influence the health and development of an infant both during infancy and later in life.

During infancy, a mammal, including a human, will transition from a diet that is composed of all or primarily a mother's milk to one of solid foods. This is referred to as the "transitional period", "transitional feeding period", or "weaning". As this occurs, significant changes in the gut microbiome structure can take place due the change in diet and other stressors during that time (see e.g., Vatanen et al., 2019. Nature Microbiology. 4:470-479; Dizzell et al., 2021. PLOS ONE. https://doi.org/10.1371/journal.pone.0248924; Moore and Townsend. 2019. Open Biol. Sep; 9(9): 190128; Magne et al. 2006. FEMS Microbiology Ecology, 58(3): 563-571; and Edwards C.A. Ann Nutr Metab 2017;70:246-250). The change in microbiome structure can in turn impact the physiologic, cognitive, anatomical, health or other state or characteristic of the mammal. Although several studies have and are currently investigating the gut microbiome during infancy and young childhood, the gut microbiome and its impact on the immediate and lifelong health and well-being of the infant is far from being well characterized. Paralleling the lack of characterization and understanding of the gut microbiome in infancy and young childhood is also a paucity of compositions and formulations capable of facilitating a healthy gut microbiome appropriate for infant or young child use. As such, there exists a need for improved characterization and understanding of the gut microbiome and compositions and methods to support and/or establish a healthy gut microbiome, particularly in infants and young children.

Transition between a fully milk-based diet, either breast-feeding or formula feeding, and solid foods rich in proteins and fibres results in an increase of bacterial numbers in the gut leading to the evolution in a microbial composition associated with adult individuals. Weaning is considered as a stressful and complex process and the disruption of gut microbiota can lead to microbiota dysbiosis that is linked to pathogenesis of both intestinal disorders, such as diarrhoea, IBD, IBS and coeliac disease and extra-intestinal disorders, such as allergies, asthma, metabolic syndrome, cardiovascular disease and obesity. It would be desirable to reduce the stress induced by weaning to develop and maintain a heathy gut microbiota.

Vatanen *et al.* have described *Bifidobacterium longum* subsp microorganisms of a clade that is present in the gut microbiome of the transitional feeding period of mammals, particularly humans, and demonstrated that this distinct *Bifidobacterium longum* clade expanded with introduction of solid foods and harbored enzymes for utilizing both breast milk and solid food substrates (Cell; 2022 Nov 10;185(23):4280-4297.e12). WO2023/278441 describes that this *Bifidobacterium longum* clade is greater in relative abundance during the transitional feeding period (e.g. weaning period) than either *B. longum* subsp*. infantis* (*B. infantis*) or *B. longum* subsp *longum.* Further, the relative abundance of *B. longum* subsp *infantis* decreases at the beginning of the transitional feeding period until the end of the transitional feeding period while *B. longum* subsp *longum* begins to increase in abundance.

However, there exists a need for compositions and methods that are particularly suited and advantageous for supporting the transition between milk-based diet and solid in infants and young children.

### SUMMARY OF THE INVENTION

The present invention is based, at least in part, on the provision of a novel *Bifidobacterium longum* transitional microorganism strain. This *B. longum* transitional strain is referred to herein as NCC 5025; and was deposited with the Collection Nationale de Cultures de Microorganisms (CNCM), Institute Pasteur by SOCIÉTÉ DES PRODUITS NESTLÉ S.A according to Budapest Treaty on the 29^{th} of March 2023 receiving the deposit number CNCM I-5942.

Accordingly, in a first aspect the present invention provides a *B. longum* transitional microorganism strain deposited with Collection Nationale de Cultures de Micro-organismes (CNCM) under deposit number CNCM I-5942 or a *B. longum* transitional strain having an identifying characteristic of the *B. longum* transitional strain deposited under deposit number CNCM I-5942.

In a further aspect the invention provides a *B. longum* transitional microorganism strain which has an Average Nucleotide Identity (ANI) of at least 99% compared to the *B. longum* strain deposited with the CNCM under deposit number CNCM I-5942. Suitably, the *B. longum* transitional microorganism strain has at least one identifying characteristic of the *B. longum* transitional strain deposited under deposit number CNCM I-5942, as described herein.

In another aspect, the present invention provides a composition comprising a *B. longum* transitional strain according to the present invention; suitably wherein the composition comprises at least one further probiotic and/or prebiotic.

The invention further provides use of a *B. longum* transitional strain according to the present invention to promote and/or assist the transition from a milk-based diet to solid food in an infant and/or in a young child.

In a further aspect, the invention provides a method of promoting and/or assisting the transition from a milk-based diet to solid food in an infant and/or in a young child, the method comprising administering to the infant and/or to the young child a *B. longum* transitional strain according to the present invention.

The present *B. longum* transitional strain is considered to have several advantageous characteristics which make it particularly suited for supporting the transition between a milk-based diet and solid in infants and young children, for example when used as a probiotic or as part of a synbiotic.

Without wishing to be bound by theory, the present *B. longum* transitional strain may provide one or more of the following advantages:
a) free of antibiotic resistance to the set of antibiotics considered relevant by EFSA;
b) a unique Carbohydrate Active EnZyme (CaZy) profile, including the presence of a GH43 subfamily 17 enzyme, that was not characterized to date in the *B. longum* species;
c) advantageous growth on 3-FL; without wishing to be bound by theory, this capacity is believed to render the present *B. longum* transitional strain competitive in the weaning infant gut environment;
d) advantageous growth on a set of food derived fibers (e.g. inulin and arabinan).

Overall, the present *B. longum* transitional strain is particularly adapted to the weaning period and may perform in this environment better than other *B. longum* transitional strains. In addition, the present *B. longum* transitional strain may perform better on a diet containing food derived fiber (e.g. in adulthood) than other *B. longum* transitional strains.

### FIGURES

**Figure 1** - UPGMA phylogenetic tree of *B. Longum* genomes
**Figure 2** - Carbohydrate-Active Enzymes (CAZymes) harbored by *B. longum* transitional strains, including NCC 5025.
**Figure 3** - The genetic region of NCC 5025 encompassing the unique GH43_17 encoding gene.
**Figure 4** - Growth profile of *B. longum* transitional strains, including NCC 5025 on 3-FL as sole carbon source. The final panel represents the obtained growth rates k for each tested strain.
**Figure 5** - Growth profile of different *B. longum* transitional strains on A) Inulin and B) Arabinan as a substrate

### DETAILED DESCRIPTION

Various preferred features and embodiments of the present invention will now be described by way of non-limiting examples. The skilled person will understand that they can combine all features of the invention disclosed herein without departing from the scope of the invention as disclosed.

Any reference to prior art documents in this specification is not to be considered an admission that such prior art is widely known or forms part of the common general knowledge in the field. All publications mentioned in the specification are herein incorporated by reference.

As used in this specification, the words "comprises", "comprising", and similar words, are not to be interpreted in an exclusive or exhaustive sense. In other words, they are intended to mean "including, but not limited to". The terms "comprises", "comprising", and similar words also include the term "consisting of".

The practice of the present invention will employ, unless otherwise indicated, conventional techniques which are within the capabilities of one of ordinary skill in the art. Such techniques are explained in the literature. Unless defined otherwise, all technical and scientific terms used herein have the same meaning as commonly understood by one of ordinary skill in the art.

Numeric ranges are inclusive of the numbers defining the range and all percentages disclosed herein are on a w/w basis, unless stated otherwise. As used herein the term "about" means approximately, in the region of, roughly, or around. When the term "about" is used in conjunction with a numerical value or range, it modifies that value or range by extending the boundaries above and below the numerical value(s) set forth. In general, the terms "about" and "approximately" are used herein to modify a numerical value(s) above and below the stated value(s) by 10%.

All percentages are by weight unless otherwise stated.

The terms "about" or "approximatively" as used herein when referring to a measurable value such as a parameter, an amount, a temporal duration, and the like, are meant to encompass variations of and from the specific value, such as the variation of 1/-10% or less, 1/-5% or less, 1/-1% or less, and +/0.1% or less of and from the specific value, insofar such variations are appropriate to perform in the disclosed invention. It is to be understood that the value to which the modifier "about" or "approximately" refers is itself also specifically, and preferably, disclosed.

Reference to a sequence which has a percent identity to any one of the SEQ ID NOs detailed herein may refers to a sequence which has the stated percent identity over the entire length of the SEQ ID NO referred to.

Identity comparisons can be conducted by eye or, more usually, with the aid of readily available sequence comparison programs. These commercially available computer programs can calculate percentage homology or identity between two or more sequences.

Percentage identity may be calculated over contiguous sequences, i.e., one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent identity when a global alignment is performed. Consequently, most sequence comparison methods are designed to produce optimal alignments that take into consideration possible insertions and deletions without penalising unduly the overall identity score. This is achieved by inserting "gaps" in the sequence alignment to try to maximise local identity.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percentage identity therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387), minimap or Burrows-Wheeler Aligner (BWA). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Suitably, the percentage identity may be calculated over a minimum coverage shared between the two sequences to be aligned using at least 80%, at least 85%, at least 90%, at least 95% or at least 99% coverage.

The alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result

The terms "subject", "individual" and "patient" are used interchangeably to refer to a vertebrate, preferably a mammal, more preferably a human. Mammals include but are not limited to murines, simians, humans, farm animals, sport animals and pets.

The term "infant" means a human subject under the age of 12 months or an age equivalent non-human animal.

The terms "young child" or "toddler" as used herein may mean a human subject aged between 12 months and 5 years of age. Suitably, a "young child" may refer to an age equivalent non-human animal.

The expressions "complementary feeding period", "complementary period", "transitional period", "transitional feeding period" and "weaning period" can be interchangeably used and refer to the period during which the milk, either breast milk or formula, is substituted by other foods in the diet of an infant or a young child. The infant or the young child is typically moved or transitioned gradually from exclusive milk-feeding, either breast feeding or formula feeding, to mixed diet comprising milk and/or solid foods. The transitional period depends on the infant or young child but typically falls between about 4 months and about 18 months of age, such as between about 6 and about 18 months of age, but can in some instances extend up to about 24 months or more. For humans, the weaning period typically starts between 4 and 6 months of age and is considered completed once the infant and/or the young child is no longer fed with breast milk or infant formula, typically at about 24 months of age. In some embodiments, the weaning period is between 4 and 24 months.

The expressions "composition" or "nutritional composition" refer to any kind of composition or formulation that provides a nutritional benefit to an individual and that may be safely consumed by a human or an animal. A nutritional composition may be in solid (e.g. powder), semi-solid or liquid form and may comprise one or more macronutrients, micronutrients, food additives, water, etc. For instance, the nutritional composition may comprise the following macronutrients: a source of proteins, a source of lipids, a source of carbohydrates and any combination thereof. Furthermore, the nutritional composition may comprise the following micronutrients: vitamins, minerals, fiber, phytochemicals, antioxidants, prebiotics, probiotics, bioactives, metabolites (e.g. butyrate, Docosahexaenoic acid (DHA), Eicosapentaenoic acid (EPA), Gamma-Linolenic acid (GLA)) and any combination thereof. The composition may also contain food additives such as stabilizers (when provided in liquid or solid form) or emulsifiers (when provided in liquid form). The amount of the various ingredients (e.g. the oligosaccharides) can be expressed in g/100 g of composition on a dry weight basis when it is in a solid form, e.g. a powder, or as a concentration in g/L of the composition when it refers to a liquid form (this latter also encompasses liquid composition that may be obtained from a powder after reconstitution in a liquid such as milk, water, e.g. a reconstituted infant formula or follow-on/follow-up formula or infant cereal product or any other formulation designed for infant or young child nutrition). Generally, a nutritional composition can be formulated to be taken enterally, orally, parenterally, or intravenously, and it usually includes one of more nutrients selected from: a lipid or fat source, a protein source, and a carbohydrate source. Preferably, a nutritional composition is for oral use.

In a particular embodiment, the nutritional composition is a "synthetic nutritional composition". The expression "synthetic nutritional composition" means a mixture obtained by chemical and/or biological means.

Suitably, the nutritional composition may comprise a fiber; for example as defined herein.

The expression "infant formula" as used herein refers to a foodstuff intended for particular nutritional use by infants during the first months of life and satisfying by itself the nutritional requirements of this category of person (Article 2(c) of the European Commission Directive 91/321/EEC 2006/141/EC of 22 December 2006 on infant formulae and follow-on formulae). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula".

A "follow-up formula" or "follow-on formula" is given from the 6th month onwards. It constitutes the principal liquid element in the progressively diversified diet of this category of person.

The expression "baby food" means a foodstuff intended for particular nutritional use by infants or young children during the first years of life.

The expression "infant cereal composition" means a foodstuff intended for particular nutritional use by infants or young children during the first years of life.

The expression "growing-up milk" (or GUM) refers to a milk-based drink generally with added vitamins and minerals, that is intended for young children or children.

The terms "fortifier" refers to liquid or solid nutritional compositions suitable for fortifying or mixing with human milk, infant formula, growing-up milk or human breast milk fortified with other nutrients. Accordingly, the fortifier can be administered after dissolution in human breast milk, in infant formula, in growing-up milk or in human breast milk fortified with other nutrients or otherwise it can be administered as a stand-alone composition.

The composition of the invention may be a supplement.

The supplement may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, the supplement may contain an organic or inorganic carrier material suitable for oral or parenteral administration as well as vitamins, minerals trace elements and other micronutrients in accordance with the recommendations of Government bodies such as the USRDA.

The term "metabolize" is used herein to mean that a substrate can by broken down, adsorbed and/or utilized by a microorganism. For example, the substrate may promote and/or contribute to the growth and/or survival of the microorganism.

Suitably, the term "capable of metabolizing the glycan substrate" may mean that the *B. longum* transitional strain encodes at least one CAZyme which is capable of utilizing the glycan substrate. For example, the CAZyme may be capable of catalyzing the hydrolysis of a glycosidic bond within the glycan substrate. Suitably, the *B. longum* transitional strain may encode at least one, at least two, at least three, at least four or at least five CAZymes that are capable of utilizing the glycan substrate. Suitably, the term "capable of metabolizing the glycan substrate" may mean that the glycan substrate (or a fiber or ingredient comprising the glycan substrate) is capable of promoting growth and/or survival of the *B. longum* transitional strain (e.g. when added to an anaerobic culture of the *B. longum* transitional strain). Growth and/or survival of the *B. longum* transitional strain through time may be determined by measuring the abundance using a strain-specific gene - for example using qPCR methods or by measuring growth via optical density of the cells at 580 nm. An illustrative assay for measuring growth of a *B. longum* transitional strain in the presence of glycan substrates (e.g. in the form of fiber) is provided in the present examples.

A glycan substrate capable of promoting growth and/or survival of the *B. longum* transitional strain may increase the number of *B. longum transitional* bacteria in an anaerobic culture by at least 20%, at least 30%, at least 40%, at least 50%, at least 75% or at least 100% compared to the number of *B. longum transitional* bacteria in a control anaerobic culture which does not comprise the HMO. Suitably, a glycan substrate capable of promoting growth and/or survival of the B*. longum transitional* strain may increase the number of *B. longum transitional* bacteria in an anaerobic culture by a statistically significant amount (e.g. p-value <0.05 as determined by one-way ANOVA) compared to the number of *B. longum transitional* bacteria in a control anaerobic culture which does not comprise the glycan substrate.

A "glycan substrate" refers to a glycan that can be metabolized by a microorganism. A glycan substrate may be, for example, a glycoconjugate, oligo- or polysaccharide. Glycoconjugate glycans may comprise N-linked glycans or O-linked glycans within glycoproteins and proteoglycans, or glycolipids. For example, an O-linked glycan may comprise a protein or peptide where the oxygen atom of a serine or threonine residue is linked to a monosaccharide, oligo- or polysaccharide as in the case with glycosaminoglycans (GAGs). Further examples of "glycan substrates" are cellulose, which is a glycan composed of β-1,4-linked D-glucose, and chitin, which is a glycan composed of β-1,4-linked N-acetyl-D-glucosamine. Glycans may be homo- or heteropolymers of monosaccharide residues and can be linear or branched. "Glycan substrate" as used herein encompasses, for example, oligosaccharides and polysaccharides.

The "oligosaccharide" may refer to a carbohydrate that has greater than 2 but relatively few monosaccharide units (typically 3, 4, 5, 6, and up to 10). Exemplary oligosaccharides include, but are not limited to, fructo-oligosaccharides, galacto-oligosaccharides (raffinose, stachyose, verbascose), maltooligosaccharides, gentio-oligosaccharides, cellooligosaccharides, milk oligosaccharides (e.g., those present in secretions from mammary glands), isomalto-oligosaccharides, lactosucrose, mannooligosaccharides, melibiose-derived oligosaccharides, pectic oligosaccharides, xylo-oligosaccharides.

The term "polysaccharide" may refer to a carbohydrate that has more than ten monosaccharide units. Exemplary polysaccharides include, but are not limited to, starch, arabinogalactan, arabinan, beta-glucan, laminarin, chrysolaminarin, xylan, arabinoxylan, mannan, fucoidan, rhamnogalacturonan and galactomannan. It is to be understood that there is not a precise cut-off or distinction between the terms oligosaccharide and polysaccharide, nor is such a distinction necessary to practice the invention.

The term "glycosaminoglycan" (GAG) or mucopolysaccharide refers to long linear polysaccharides consisting of repeating disaccharide units (i.e. two-sugar units). The repeating two-sugar unit consists of a uronic sugar and an amino sugar, with the exception of keratan, where in the place of the uronic sugar it has galactose. GAGs are classified into four groups based on core disaccharide structures.

"Mucins", as used herein, may refer to a family of high molecular weight, heavily glycosylated proteins (glycoconjugates). Mucins' key characteristic is their ability to form gels; therefore they are a key component in most gel-like secretions, serving functions from lubrication to cell signaling to forming mechanical and chemical barriers.

The term "HMO" or "HMOs" refers to human milk oligosaccharide(s). These carbohydrates are highly resistant to enzymatic hydrolysis, indicating they may display essential functions not directly related to their caloric value. It has been especially illustrated they play a vital role in the early development of infants and young children, such as the maturation of the immune system. Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy the terminal position at the non-reducing ends. Depending on the presence of fucose and sialic acid in the oligosaccharide structure, the HMOs can be divided as non-fucosylated (neutral) or fucosylated (neutral) and sialylated (acidic) and non-sialylated molecules, respectively.

The expression "fucosylated oligosaccharide" refers to an oligosaccharide having a fucose residue. It has a neutral nature. Some examples are 2'-fucosyllactose (2-FL), 3-fucosyllactose (3-FL), difucosyllactose (DiFL), lacto-N-fucopentaose (e.g. lacto-N-fucopentaose I, lacto-N-fucopentaose II, lacto-N-fucopentaose III, lacto-N-fucopentaose V), lacto-N-fucohexaose, lacto-N-difucohexaose I, fucosyllacto-N-hexaose, fucosyllacto-N-neohexaose, difucosyllacto-N-hexaose I, difucosyllacto-N-neohexaose II and any combination thereof. Fucosylated oligosaccharides represents the largest fraction of human milk with 2'-FL constituting up to 30% of the total HMOs. Fucosylated oligosaccharides are thought to reduce the risk of infections and inflammations and to boost growth and metabolic activity of specific commensal microbes reducing inflammatory response.

The expression "N-acetylated oligosaccharide(s)" encompasses both "N-acetyl-lactosamine" and "oligosaccharide(s) containing N-acetyl-lactosamine". They are neutral oligosaccharides having an N-acetyl-lactosamine residue. Suitable examples are LNT (lacto-N-tetraose), para-lacto-N-neohexaose (para-LNnH), LNnT (lacto-N-neotetraose), DSLNT (disialyllacto-N-tetraose), and any combinations thereof. Other examples are lacto-N-hexaose, lacto-N-neohexaose, para- lacto-N-hexaose, para-lacto-N-neohexaose, lacto-N-octaose, lacto-N-neooctaose, iso- lacto-N-octaose, para- lacto-N-octaose and lacto-N-decaose.

The expressions "at least one fucosylated oligosaccharide" and "at least one N-acetylated oligosaccharide" should be understood as "at least one type of fucosylated oligosaccharide" and "at least one type of N-acetylated oligosaccharide".

The term "sialylated oligosaccharide" refers to an oligosaccharide having a charged sialic acid residue. It has an acidic nature. Some examples are 3'-sialyllactose (3-SL), 6'-sialyllactose (6-SL), sialyllacto-N-tetraose (Lst - e.g. Lst-a, Lst-b or Lst-c).

Suitably, the term "capable of metabolizing the HMO" may mean that the *B. longum* transitional strain encodes at least one CAZyme which is capable of utilizing the HMO. For example, the CAZyme may be capable of catalyzing the hydrolysis of a glycosidic bond within the HMO. Suitably, the *B. longum* transitional strain may encode at least one, at least two, at least three, at least four or at least five CAZymes that are capable of utilizing the HMO. Suitably, the term "capable of metabolizing the HMO" may mean that the HMO is capable of promoting growth and/or survival of the *B. longum* transitional strain (e.g. when added to an anaerobic culture of the *B. longum* transitional strain). Growth and/or survival of the *B. longum* transitional strain through time may be determined by measuring the abundance using a strain specific genes, for example with qPCR methods or by measuring growth via optical density of the cells at 580 nm.

The term fibers is used herein to refer to carbohydrates that are indigestible by a human or animal. Such fibers are also discussed in relation to carbohydrates herein. Suitably, the fiber can be fermented by the present *B. longum* transitional strain. As used herein, the expressions "fiber" or "fibers" or "dietary fiber" or "dietary fibers" within the context of the present invention indicate the indigestible portion, in small intestine, that reaches the large intestine, of food derived from plants which comprises two main components: soluble fiber, which dissolves in water and insoluble fiber. Mixtures of fibers are comprised within the scope of the terms above mentioned. Soluble fiber is readily fermented in the colon into gases and physiologically active byproducts and can be prebiotic and viscous. Insoluble fiber does not dissolve in water, is metabolically inert and provides bulking, or it can be prebiotic and metabolically ferment in the large intestine. Chemically, dietary fiber consists of carbohydrate polymers with three or more monomeric units which are not hydrolyzed by endogenous enzymes in the small intestine or the upper tract of the digestive system, such as arabinoxylans, cellulose, and many other plant components such as resistant starch, resistant dextrins, inulin, lignin, chitins, pectins, arabinans, arabinogalactans, galactans, xylans, beta-glucans, and oligosaccharides. Non-limiting examples of dietary fibers are: prebiotic fibers such as Fructo-oligosaccharides (FOS), inulin, galacto-oligosaccharides (GOS), fruit fiber, vegetable fiber, cereal fiber, resistant starch such as high amylose corn starch.

As used herein, "added fiber" or "added dietary fiber" indicates an ingredient mainly or totally constituted by fiber which is added to the complementary nutritional composition and whose content in fiber contributes to the total fiber content of the composition. The total fiber content of the complementary nutritional composition is provided by the sum of amount of fiber naturally present in ingredients used in the recipe (for example from whole grain cereal flour) plus amount of added fiber.

Suitably, the present composition may be a probiotic composition.

The term "prebiotic" means non-digestible carbohydrates that beneficially affect the host by selectively stimulating the growth and/or the activity of healthy bacteria such as bifidobacteria in the colon of humans (Gibson GR, Roberfroid MB. Dietary modulation of the human colonic microbiota: introducing the concept of prebiotics. J Nutr. 1995;125:1401-12).

The term "probiotic" means microbial cell preparation or components of microbial cells with a beneficial effect on the health or well-being of the host (Salminen S, Ouwehand A. Benno Y. et al. "Probiotics: how should they be defined" Trends Food Sci. Technol. 1999:10 107-10). The microbial cells according to the present invention are generally bacteria.

The term "cfu" should be understood as colony forming unit.

The "gut microbiota" is the composition of microorganisms (including bacteria, archaea and fungi) that live in the digestive tract.

The term "gut microbiome" may encompass both the "gut microbiota" and their "theater of activity", which may include their structural elements (nucleic acid, proteins, lipids, polysaccharides), metabolites (signaling molecules, toxins, organic and inorganic molecules) and molecules produced by coexisting hosts and structured by the surrounding environmental conditions (Berg, G., et al., 2020. Microbiome, 8(1), pp.1-22).

### Bifidobacterium longum transitional microorganism strain

In a first aspect, the present invention provides a *Bifidobacterium longum* transitional microorganism strain deposited with Collection Nationale de Cultures de Micro-organismes (CNCM) under deposit number CNCM I-5942 or a *B. longum* transitional strain having the identifying characteristic of the *B. longum* transitional strain deposited under deposit number CNCM I-5942.

Suitably, *a Bifidobacterium longum* transitional strain may be referred to herein as *B. longum* subsp. *juvenis*.

Suitably, an identifying characteristic of the present *B. longum transitional* strain may refer to one or more of the phenotypic or genotypic characteristics described herein.

In another aspect, the present invention provides a *B. longum* transitional microorganism strain which has an Average Nucleotide Identity (ANI) of at least 99% to the *B. longum* transitional strain deposited with the CNCM under deposit number CNCM I-5942.

In some embodiments, the *B. longum* transitional strain has an ANI of at least at least 99.0%, at least 99.1%, at least 99.2%, at least 99.3%, at least 99.4%, at least 99.5%, at least 99.6%, at least 99.7%, at least 99.8%, or at least 99.9%, compared to the *B. longum* strain deposited with the CNCM under deposit number CNCM I-5942.

Preferably, the *B. longum* transitional strain has an ANI of at least 99.9% compared to the *B. longum* strain deposited with the CNCM under deposit number CNCM I-5942.

Suitably, the *B. longum* transitional strain has an ANI of at least 98.1%, at least 98.2%, at least 98.3%, at least 98.4%, of at least 98.5%, of at least 98.6%, of at least 98.6 %, of at least 98.7 %, of at least 98.8 %, of at least 98.9 %, of at least 99 %, of at least 99.1 %, of at least 99.2 %, of at least 99.3 %, of at least 99.4 %, of at least 99.5 %, of at least 99.6 %, of at least 99.7 %, of at least 99.8 %, or of at least 99.9 % compared to the *B. longum* strain deposited with the CNCM under deposit number CNCM I-5942 and has at least one identifying characteristics of the *B. Longum* transitional strain deposited under deposit number CNCM I-5942 - as described herein.

Suitably, the *B. longum* transitional strain has an ANI of at least 98.4%, of at least 98.5%, of at least 98.6%, of at least 98.6 %, of at least 98.7 %, of at least 98.8 %, of at least 98.9 %, of at least 99 %, of at least 99.1 %, of at least 99.2 %, of at least 99.3 %, of at least 99.4 %, of at least 99.5 %, of at least 99.6 %, of at least 99.7 %, of at least 99.8 %, or of at least 99.9 % compared to the *B. longum* strain deposited with the CNCM under deposit number CNCM I-5942 and has at least one identifying characteristics of the *B. longum* transitional strain deposited under deposit number CNCM I-5942 - as described herein.

Methods for sequencing microbial genomes are well known in the art (see e.g. Segerman; Front. Cell. Infect. Microbiol.; 2020; 10; Article 527102 & Donkor; Genes; 2013; 4(4); 556-572). By way of example, metagenomics methods may be used. Suitable metagenomics methods may be performed using shotgun sequencing data, for example. Suitable metagenomics methods are known in the art and include MetaPhlAn 3.0, for example (see Beghini et al.; eLife 2021;10: e65088; https://huttenhower.sph.harvard.edu/metaphlan).

The "Average Nucleotide Identity (ANI)" is a term of art that refers to a distance-based approach to delineate species based on pair-wise comparisons of their genome sequences and is an *in silico* alternative to the traditional DNA-DNA hybridization (DDH) techniques that have been used for phylogenetic definition of a species (Goris et al., 2007, "DNA-DNA hybridization values and their relationship to whole-genome sequence similarities", Int. J. Syst. Evol. Microbiol. 57: 81-91). Based on DDH, strains with greater than 70% relatedness would be considered to belong to the same species (see e.g., Wayne et al., 1987, Report of the Ad-Hoc-Committee on Reconciliation of Approaches to Bacterial Systematics. Int J Syst Bacteriol 37: 463-464). ANI is similar to the aforementioned 70% DDH cutoff value and can be used for species delineation. ANI has been evaluated in multiple labs and has become the gold standard for species delineation (see e.g., Kim et al., 2014, "Towards a taxonomic coherence between average nucleotide identity and 16S rRNA gene sequence similarity for species demarcation of prokaryotes", Int. J. Syst. Evol. Micr. 64: 346-351; Richter et al., 2009, "Shifting the genomic gold standard for the prokaryotic species definition", P Natl Acad Sci USA 106: 19126-19131; and Chan et al., 2012, "Defining bacterial species in the genomic era: insights from the genus Acinetobacter", Bmc. Microbiol. 12)).

The ANI of the shared genes between two strains is known to be a robust means to compare genetic relatedness among strains, and that ANI values of about 95% correspond to the 70% DNA-DNA hybridization standard for defining a species. See, e.g., Konstantinidis and Tiedje, Proc Natl Acad Sci USA, 102(7):2567-72 (2005); and Goris et al., Int Syst Evol Microbiol. 57(Pt 1):81-91 (2007). The ANI between two bacterial genomes is calculated from pair-wise comparisons of all sequences shared between any two strains and can be determined, for example, using any of a number of publicly available ANI tools, including but not limited to OrthoANI with usearch (Yoon et al. Antonie van Leeuwenhoek 110:1281-1286 (2017)); ANI Calculator, JSpecies (Richter and Rossello-Mora, Proc Natl Acad Sci USA 106:19126-19131 (2009)); and JSpeciesWS (Richter et al., Bioinformatics 32:929-931 (2016)). Other methods for determining the ANI of two genomes are known in the art. See, e.g., Konstantinidis, K. T. and Tiedje, J. M., Proc. Natl. Acad. Sci. U.S.A., 102: 2567-2572 (2005); and Varghese et al., Nucleic Acids Research, 43(14):6761-6771 (2015). In a particular embodiment, the ANI between two bacterial genomes can be determined, for example, by averaging the nucleotide identity of orthologous genes identified as bidirectional best hits (BBHs). Protein-coding genes of a first genome (Genome A) and second genome (Genome B) are compared at the nucleotide level using a similarity search tool, for example, NSimScan (Novichkov et al., Bioinformatics 32(15): 2380-23811 (2016)). The results are then filtered to retain only the BBHs that display at least 70% sequence identity over at least 70% of the length of the shorter sequence in each BBH pair. The ANI of Genome A to Genome B is defined as the sum of the percent identity times the alignment length for all BBHs, divided by the sum of the lengths of the BBH genes. These and ANI determination techniques are known in the art.

According to the present invention, a *B. longum* transitional strain deposited with the CNCM under deposit number CNCM I-5942 represents the reference genome to which a test genome is compared.

In some embodiments, the *B. longum* transitional strain of the present invention is isolated from a human.

In some other embodiments, the *B. longum* transitional strain is not of the subspecies *B. longum* subsp*. longum* or *B. longum* subsp. *infantis*.

Suitably, the *B. longum* transitional strain is provided as a probiotic. Suitably, the *B. longum* transitional stain is provided in a composition.

### Antibiotic Resistance

Suitably, the present *B. longum* transitional strain does not harbor transferable antibiotic resistance to one or more antibiotics, preferably one or more European Food Standard Agency (EFSA) relevant antibiotics (see European Food Safety Authority. 2012. Guidance on the assessment of bacterial susceptibility to antimicrobials of human and veterinary importance. EFSA J 10:2740).

Antibiotic resistance refers to the ability of microorganisms to withstand antibiotic treatments. The overuse or misuse of antibiotics has been linked to the emergence and spread of microorganisms which are resistant to them, rendering treatment ineffective and posing a serious risk to public health. In addition, the wide-spread use of antibiotics means that it is increasingly challenging to provide bacterial strains that do not have transferrable resistance to one or more EFSA relevant antibiotics.

It is known that a single gene may instill antibiotic resistance against a particular antibiotic, and that bacteria can transfer genes through horizontal gene transfer via conjugation, transduction or transformation. Accordingly, it is known that antibiotic resistance may be transferred between bacteria via horizontal gene transfer; including in the gut microbiome.

It is therefore advantageous that the present *B. longum* transitional strain does not harbor transferrable antibiotic resistance to one or more antibiotics as this reduces the risk of the antibiotic resistance being transferred to other components of the microbiome when the present *B. longum* transitional strain is used as a probiotic.

Antibiotics resistance has been well-described and antibiotic resistance may be determined using any suitable assay known in the art. By way of example, phenotypic and/or genetic methods may be used. Phenotypic methods typically involve measuring the growth of a test bacteria in the presence of a suitable concentration of the antibiotic under consideration. In addition, a number of genes mediating antibiotic resistance are known. Accordingly, genetic methods for determining antibiotic resistance comprise determining the presence of one or more antibiotic resistance genes in the genome of the test bacteria (for example by PCR, DNA microarray, whole-genome sequencing and metagenomics, and matrix-assisted laser desorption ionization-time of flight mass spectrometry). Suitably, phenotypic antibiotic testing of may be performed according to the recommendations made by EFSA (EFSA J 16, e05206, doi:10.2903/j.efsa.2018.5206 (2018)); for example following the official method ISO 10932. An illustrative method for determining antibiotic resistance is detailed in the present Examples.

Antibiotic resistance and underlying genes present in *Bifidobacterium* are known in the art (see e.g. Duranti et al.; Appl Environ Microbiol. 2017 Feb 1; 83(3): e02894-16.). As such, the skilled person is able to determine whether a test *Bifidobacterium* is resistant to one or more antibiotics.

Suitably, the present *B. longum* transitional strain is not resistant to at least 1, at least 2, at least 3, at least 4, at least 5, at least 6, or at least 7 EFSA relevant antibiotics.

Suitably, the *B. longum* transitional strain is not resistant to any one of tetracycline and erythromycin.

Suitably, the *B. longum* transitional strain is not resistant to any one of tetracycline, erythromycin, clindamycin and ampicillin.

Suitably, the *B. longum* transitional strain is not resistant to any of tetracycline, erythromycin, clindamycin, ampicillin, gentamycin, streptomycin, chloramphenicol and vancomycin.

Resistance to tetracycline may be afforded by *tet(W)* or tet(Q) genes which encode ribosomal protection proteins. Suitably, the present *B. Longum* transitional strain may lack a *tet(W)* gene. Suitably, the present *B. longum* transitional strain may lack a *tet(W)* gene encoding a polypeptide shown as SEQ ID NO: 1 or a variant which shares at least 80% sequence identity to SEQ ID NO: 1. Suitably, the variant may share at least 85%, at least 90%, at least 95%, at least 96%, at least 97%, at least 98% or at least 99% identity with SEQ ID NO: 1.

SEQ ID NO: 1

MKIINIGILAHVDAGKTTLTESLLYASGAISEPGSVEKGTTRTDTMLLERQRGITIQAAVTSFQWHRCKVNIVDT
PGHMDFLAEVYRSLAVLDGAILVISAKDGVQAQTRILFHALRKMNIPTVIFINKIDQAGVDLQSVVQSVRDKLSA
DIIIKQTVSLSPEIVLEENTDIEAWDAVIENNDKLLEKYIAGEPISREKLVREEQRRVQDASLFPVYYGSAKKGL
GIQPLMDAVTGLFQPIGEQGSAALCGSVFKVEYTDCGQRRVYLRLYSGTLRLRDTVALAGREKLKITEMRIPSKG
EIVRTDTAYPGEIVILPSDSVRLNDVLGDPTRLPRKRWREDPLPMLRTSIAPKTAAQRERLLDALTQLADTDPLL
RCEVDSITHEIILSFLGRVQLEVVSALLSEKYKLETVVKEPTVIYMERPLKAASHTIHIEVPPNPFWASIGLSVT
PLPLGSGVQYKSRVSLGYLNQSFQNAVRDGIRYGLEQGLFGWNVTDCKICFEYGLYYSPVSTPADFRSLAPIVLE
QALKESGTQLLEPYLSFTLYAPREYLSRAYHDAPKYCATIETVQVKKDEVVFTGEIPARCIQAYRTDLAFYTNGQ
SVCLTELKGYQAAVGKPVIQPRRPNSRLDKVRHMFSKIT

Suitably, the present *B. Longum* transitional strain may lack a *tet(Q)* gene. Suitably, the present *B. longum* transitional strain may lack a *tet(Q)* gene encoding a polypeptide shown as SEQ ID NO: 2 or a variant which shares at least 80% sequence identity to SEQ ID NO: 2. Suitably, the variant may share at least 85%, at least 90%, at least 95%, at least 96%, at least 97%, at least 98% or at least 99% identity with SEQ ID NO: 2.

SEQ ID NO: 2

MRFDNASNVVYYCLIQMNIINLGILAHIDAGKTSVTENLLFASGATEKCGRVDNGDTITDSMDIEKRRGITVRAS
TTSIIWNGVKCNIIDTPGHMDFIAEVERTFKMLDGAVLILSAKEGIQAQTKLLFNTLQKLQIPTIIFINKIDRAG
VNLERLYLDIKTNLSQDVLCMQTVVDGSVYPVCSQTYIKEEYKEFVCDHDDNILERYLADSEIPPTDYWNTIIAL
VAKAKVYPVLHGSAMFNIGINELMDAITSFILPPASVSDRLSAYLYKIEHDPKGHKRSFLKIIDGSLRLRDVVRI
NDSEKSIKIKNLKTIYQGREINVDEVGANDIAIVEDMEDFRIGDYLGAEPCLIQGLSHQHPALKSSVRPDKPEER
SKVISALNTLWIEDPSLSFSINSYSDELEISLYGLTQKEIIQTLLEERFSVKVHFDEIKTIYKERPIKKVNKIIQ
IEVPPNPYWATIGLTLEPLPLGAGLQIESDISYGYLNHSFQNAVFEGIRMSCQSGLHGWEVTDLKVTFTQAEYYS
PVSTPADFRQLTPYVFRLALQQSGVDILEPMLYFELQIPQEASSKAITDLQKMMSEIEDISCNNEWCHIKGKVPL
NTSKDYASEVSSYTKGLGIFMVKPCGYQITKDGYSDNIRMNEKDKLLFMFQKSMSLK

Resistance to erythromycin may be afforded by the *erm(49)* gene which encodes a rRNA methylase. Suitably, the present *B. Longum* transitional strain may lack a *erm(49)* gene. Suitably, the present *B. Longum* transitional strain may lack of *erm(49)* gene encoding a polypeptide shown in SEQ ID NO: 3or a variant which shares at least 80% sequence identity to SEQ ID NO: 3. Suitably, the variant may share at least 85%, at least 90%, at least 95%, at least 96%, at least 97%, at least 98% or at least 99% identity with SEQ ID NO: 3. 
SEQ ID NO: 3

MRNIKDTQNFLHSKELVRHLIGICNIKLDDVVIEIGPGKGIITNELAHKARKVVAIEFDEELYEKLKNKFQSNNK
VDIIYGDILNYTPRIPSYCVFSNIPFNITSEILNKFLSDKKNEKMFLIMQYEPFIKYAGNPYGAETLRSMLYKPF
FDMDLKYRFDPSDFKPAPQARIVLASFERKQFPDVKKEEEKLYKDFLAYIYTNKGETFFAKIKTLFSSNQIKRVW
GQIKIDKTTKISEVPYESILKVFKLFFLYGTDANKQLVVNSFNNMNKQNNKLQKNHRNNSKAKSWNSNRKRKPYH
RNNV
 Resistance to erythromycin and clindamycin may be afforded by th*e erm(X)* gene which encodes a ribosomal protection protein. Suitably, the present *B. Longum* transitional strain may lack an *erm(X)* gene. Suitably, the present *B. Longum* transitional strain may lack an *erm(X)* gene which encodes a protein comprising SEQ ID NO: 4 or a variant which shares at least 80% sequence identity to SEQ ID NO: 4. Suitably, the variant may share at least 85%, at least 90%, at least 95%, at least 96%, at least 97%, at least 98% or at least 99% identity with SEQ ID NO: 4.

SEQ ID NO: 4

MSAYGHGRHENGQNFLTNHKIINSIIDLVKQTSGPIIEIGPGSGALTHPMAHLGRAITAVEVDAKLAAKLTQETS
SAAVEVVHDDFLNFRLPATPCVIVGNIPFHLTTAILRKLLHAPAWTDAVLLMQWEVARRRAGVGASTMMTAQWSP
WFTFHLGSRVPRTAFRPQPNVDGGILVIRRVGDPKIPIEQRKAFQAMVHTVFTARGRGIGEILRRAGLFSSRSET
QSWLRSRGIDPATLPPRLHTNDWIDLFQVTGSSLPHHRPISPSGSSQRPPQQKNRSRRR

Resistance to streptomycin may be afforded by a mutation within the rpSL gene which encodes a ribosomal S12 protein. More specifically, a mutation at nucleotide position 128, replacing an A residue to a G residue was shown to provide streptomycin resistance (see Kiwaki & Sato; Int J Food Microbiol. 2009 Sep 15;134(3):211-5). Suitably, the present *B. Longum* transitional strain may have an A residue a position 128 of the rpSL gene. Suitably, the present *B. Longum* transitional strain does not comprise a G128A mutation in the rpSL gene. An illustrative rpSL gene sequence comprising an A at position 128 is shown as SEQ ID NO: 5.

SEQ ID NO: 5

TTGCCTACTATTGAACAGCTCGTCCGTAAGGGACGTCAGGCAAAGCCGAAGAAGTCCAAGACTTTGGCCCTGAAG
GGCAGCCCGCTGCGTCGCGGCGTGTGCACCCGTGTCTACACCACCACCCCGAAGAAGCCGAACTCGGCTCTGCGT
AAGGTCGCTCGTGTGCGCCTGTCCTCGGGCATCGAAGTCACCGCCTACATTCCGGGCGAGGGCCACAACCTGCAG
GAGCACTCCATCGTGCTCGTGCGCGGCGGCCGTGTGAAGGATCTCCCGGGTGTGCGTTACCACATCGTGCGTGGC
GCGCTCGATACCCAGGGTGTCAAGGACCGTAAGCAGGGTCGTTCCCTGTATGGAGCAAAGAAGGCGAAGTAA

Resistance to chloramphenicol may be afforded by the *crmX* gene which encodes a ribosomal protection protein. Suitably, the present *B. longum* transitional strain may lack a *crmX* gene. Suitably, the present *B. longum* transitional strain may lack a *crmX* gene encoding a polypeptide comprising SEQ ID NO: 6 or a variant which shares at least 80% sequence identity to SEQ ID NO: 6. Suitably, the variant may share at least 85%, at least 90%, at least 95%, at least 96%, at least 97%, at least 98% or at least 99% identity with SEQ ID NO: 6.

SEQ ID NO: 6

MPFALYMLALAVFVMGTSEFMLAGLLPAIATELDVSVGTAGLLTSAFAVGMVVGAPVMAAFARRWPPRLTLIVCL
LVFAGSHVIGAMTPVFSLLLITRVLSALANAGFLAVALSTATTLVPANQKGRALSILLSGTTIATVVGVPAGALL
STALGWRTTFWAIAILCIPAAVGVIRGVTNNVGRSETSATSPRLRVELSQLATPRLILAMALGALNNGGTFAAFT
FLAPIVTETAGLAEAWVSVALVMFGIGSFLGVTIAGRLSDQRPGLVLAVGGPLLLTGWIVLAVVASHPVALIVLV
LVQGFLSFGVGSTLITRVLYAASGAPTMGGSYATAALNIGAAAGPVLGALGLATGLGLLAPVWVASVLTAIALVI
MLLTRRALTKTAAEAN

### Glycan Substrate / Carbohydrate-Active Enzymes (CAZymes)

The present *B. longum* transitional strain encodes a specific profile of Carbohydrate-Active Enzymes (CAZymes).

Carbohydrate-active enzymes (CAZymes) are responsible for the synthesis and breakdown of glycoconjugates, oligo- and polysaccharides. They typically correspond to 1-5% of the genes in the living organism. Glycoconjugates, oligo- and polysaccharides play essential roles in many biological functions, for example as structure and energy reserve components and in many intra- and intercellular events. The Carbohydrate Active Enzyme (CAZy) classification is a sequence-based family classification system that correlates with the structure and molecular mechanism of CAZymes (www.cazy.org).

CAZymes include glycoside hydrolyases (GH), glycosyltransferases (GT), polysaccharide lyases (PL), carbohydrate esterases (CE), and carbohydrate-binding module families (CBM)
GHs catalyze the hydrolysis of glycosidic bonds between two or more carbohydrates or between a carbohydrate and a non-carbohydrate moiety. In most cases, the hydrolysis of the glycosidic bond is catalyzed by two amino acid residues of the enzyme: a general acid (proton donor) and a nucleophile/base. Depending on the spatial position of these catalytic residues, hydrolysis occurs via overall retention or overall inversion of the anomeric configuration.

A GH classification system is provided by the CAZy classification. Herein, GHs are divided into families based on molecular function (e.g., GH1, GH2, GH3, GH4, etc.). These families are then further divided into subfamilies based on subgroups found within a family that share a more recent ancestor and, typically more uniform in molecular function (e.g., GH13_1, GH13_2, GH13_3, GH13_4, etc.).

Suitably, the present *B. Longum* transitional strain encodes a glycosyl hydrolase family 43_17 (GH43_17) enzyme. GH43_17 comprises both α-L-arabinofuranosidase (EC 3.2.1.55) and endo-β-1,4-xylanase (EC 3.2.1.8) activities, with capacity to breakdown complex carbohydrates like arabinan, arabinogalactan, and arabinoxylan. Suitably, the GH43_17 gene comprises SEQ ID NO: 7 or a sequence with at least 60% sequence identity to SEQ ID NO: 7. Suitably, the GH43_17 gene comprises a sequence with at least 60%, at least 70%, at least 80%, at least 90%, at least 95%, at least 98% or at least 99% sequence identity to SEQ ID NO: 7.

SEQ ID NO: 7

ATGAAACGAACTGACATCCACCTGCGCGATCCGTTCGTCCTGCCTCACGACGGTGTCTATTACCTGTATGGCACC
CGCGCTGATAACGTGTGGGGCGCGATGGATGGTTTTGATTGCTACACCAGCCGCGACCTTGACAATTGGGAGGGT
CCGTTCGAGGTGTTCCACAAGCCGGATGAATTCACGGCCGACCGTGCTTACTGGGCGCCCGAATGCTACGAGCGA
GACGGTGTATTCCACCTGATTGCCACGCTCGGCGAGCCGGACGGGCGCAAAAGCGTGCACATGCTACGCGCTGAT
AGTCCGCTTGATCCGTTCGAATATGTCTGCCGGCTGACCGATCCGAATCAGTCCTGCATTGACGGAACTCTGCAT
GGTGAAGGTACCGATATGTGGCTTGTCTACTCGCATTCCTTGGAGGATGTGCCCGCCGGAGACATGGATGCCGTA
CGTCTGTCCTCCGACCTGACTCGGACGGTGGGGGAGAGCATGACATTGTTCCAGGCCTCGGATGCGCCGTGGGCG
GTGCCGGTGCCGTTCGCGAAAGCGGAATTCGGCATCGACGAGGACGCCTACTTCTCCGATGGTCCCTGCCTGTGC
AGGCTTTCCAACGGACGGCTGGCGATGCTGTGGTCGAGCTGGTCGACGGAAGGCGGATATGCAGTCGGCCAGGCC
ATCAGCGAATCAGGGTCGATTGCTGGGCCTTGGACGCAATGCCCCGAGCCTCTGCTTAGCCACGGCGGCCACGGC
ATGCTGTTCAACGGTCTCGATGGCGTGCTGCGTTACGCGGTCCACTCGCCCAACGACCCCGGCCAGGAACGGCCT
ACGTTTTTGTGCGTCGAAGAACAAGACGGGCTGCTGACGATTACGGAATAG

Suitably, the GH43_17 gene may encode a protein shown as SEQ ID NO: 8 or a sequence with at least 80% sequence identity to SEQ ID NO: 8. Suitably, the protein may comprise a sequence with at least 85%, at least 90%, at least 95%, at least 98% or at least 99% sequence identity to SEQ ID NO: 8.

SEQ ID NO: 8

MKRTDIHLRDPFVLPHDGVYYLYGTRADNVWGAMDGFDCYTSRDLDNWEGPFEVFHKPDEFTADRAYWAPECYER
DGVFHLIATLGEPDGRKSVHMLRADSPLDPFEYVCRLTDPNQSCIDGTLHGEGTDMWLVYSHSLEDVPAGDMDAV
RLSSDLTRTVGESMTLFQASDAPWAVPVPFAKAEFGIDEDAYFSDGPCLCRLSNGRLAMLWSSWSTEGGYAVGQA
ISESGSIAGPWTQCPEPLLSHGGHGMLFNGLDGVLRYAVHSPNDPGQERPTFLCVEEQDGLLTITE

Suitably, the present *B. Longum* transitional strain comprises a glycosyl hydrolase family 43_22 (GH43_22) gene. Suitably, the GH43_22 gene comprises SEQ ID NO: 9 and/or 10, or a sequence with at least 60% sequence identity to SEQ ID NO: 9 or 10. Preferably, the present *B. Longum* transitional strain comprises a GH43_22 gene with at least 60% sequence identity to SEQ ID NO: 9 and a GH43_22 gene with at least 60% sequence identity to SEQ ID NO: 10. Suitably, the GH43_22 gene comprises a sequence with at least 60%, at least 70%, at least 80%, at least 90%, at least 95%, at least 98% or at least 99% sequence identity to SEQ ID NO: 9 or 10.

SEQ ID NO: 9
GTGAAGCATTGGAAGAAGATGGCAGCATCGTTGGTTGCAATATCAACGATGATGGCAGTAGTTCCGACGACGTAT
GCCATGGAATCGGAAGATTCCCAACCACAGACAACCGATACCGCGACAGTGCAGACTACTAAGGCTGCTGAACCG
ACGCTGCTCGCCAGCTGGGACTTCACGGGCAAAAACGGCACCACGAACAGCGCGATTGCCGATTCGACCGGCAAG
TACAACCTGACGCTGAAGGACGGCGCCAAGATCGAACAGTACGGTGACCGCAGCACCAACGAGGCGCTCTCACTG
CGCGGCGATGGCCAGTACGCCCAGATCGATGACCAGCTGTTCAAGGATGCGGGCGACTCCTTCACTCTGGAGTTC
GCGTCCAAGACTCGTCACGACGACAGCGGCAAGTTCTTCTCGTTCATCGTCGGCAAGGACGGCTCGAACGACGCC
AACACCACCGATCAGGCCAACGCCAACAAGTACCTGATGTTCTACAACAGCAAGACCGCCATCAAGGGCGTTATC
TCAAACAACAACTGGGGTAACGAACAGGGATCCAAGGTCACCGTTTCCGGCAACGACAACAGCTGGGCCGATTAC
AAGATTGTCGTGGACGGCACCAACCTTGCCGTGTTCCGCAACAATGCCCTGATTATTTCAAGGCCAACACCGGC

ATCAAGATGAGCGATCTCGGTGCGACCACCGCCTACATCGGCAAGTCGTTCTACTCCGTCGATGAGTACTGGAAT
GGTGCAATGGATGATATCAAGGTCTACAGGGGCGCTGACCTGACCATGCCGACCGCCGTTGCGATTTCCGGTACC
GGTGTGGTGAACAACAAGCTCACCCTGATTGAGAAGGACTCCACCAAGCTCACCGCCACCGTCACTCCGGACGAC
GCCGTGAGCAAGAACGTCACCTGGTCCTCCTCCGATGAGTCCGTGGCCAAGGTCGCCGCAGACGGTACTGTAACC
GGCGTCAAGGCTGGTACTGCCACCATCACCGCCACCACTGAGCTGGGTGGTGTGAAGGCCGAACTGCCCGTCACC
GTTGAGCCGATGAACGCCCAGAACGCCGCCGCAGCCGACCTCGATGCCGCGATTGCTGCGCTGAAAGTTCCGGCG
GCCGAGAATCTGCCGCTAGTCGCCAAGGGCACCAAGAACGGCTCGGCGATTACGTGGAAGTCCTCGGACGAGAAG
CTCATTACGTCCACTAACGAGAAGTACGAAAACAAGACCACTGGTGCCGATGACCCGTATCGTGGTGCTGGCATC
ATCAATCGTCCGGCCTACGGCGACGGTGATTCCAAGCCGGTTACGCTGACCGCCACCGCTTCCTACAACGGCGGT
GAGAAGGTCACCAAGACCATCGAGGTCACTGTCAAGGAGAAGACCCGCATCGCGCCTGACACCGGCTATGCGGCC
GTCACTTTTGAGAGCGACAGCAACGGTGGAGAAAAGGCCTGGGTGGCTTCCACTGAGAAGAACGATTTCTTCACG
TTTAAGACTCGCAACAATGGCCAGGCGGTACTTACCAATGATGCAGACACGGGTGGCTTGCGTGACATGTTCGTG
CTGCGTTCCCACGAAGGCGACAAGTACTACCTGATTGCCACTGATCTCAAGGTCTCGTCAATGGGCTGGAGCCAG
AACCAGGTTAACGGTTCTCGGAAAGTTGAGGTCTACGAGTCCACCGATATGATGAACTGGACCCGTACCAACGGC
GACGGCAACGGCGGCATCACCATCAACACGCCGAACGCCGGTATGACCTGGGCGCCGGAAGCTTACTGGGATGAT
GACCTGAACGCTTACGTGGTGTTCTTCTCTTCCCGCATGTTCACTGATGACACCCGTACCACTCCGGTCAAGAAC
GACAAAACCGGCAATAGCTCCTATGCTCAGGTGCGTTACGCCATCACCCGCGACTTCGTGAACTTCACCGAGCCG
CAGATGTGGCAGGACACCGGCTACTCGCGCATTGATTCCACCGTGCGTAAGATCGGTGGCTACTACTACCGATTC
ACCAAGAATGAGCAGGGCGGTGCCGCTGGCGATTACATCACCACTGGTAAGAGCATCTTCCTTGAGCGTTCCAAG
GTGCTGACTGCACCGACCACCGAGGCATCTCCGGGTCAGGACCCGAACACCGGTTGGCAGTGCTCGAGCAGGCGT
TGCTGCCGTTCGAAGGACCAGAGACCATCAAGCTCAACAAGGATGACGAACTCAACACGAAGGACGACGACGGCT
ACATTCTGCTGTCCGACAACTTCGCCTACCGTGCATTTATGACCACGGGTGCCGAGCTTTCCAAGACCACGTGGG
ACAACCCGATGACCAAGCGTTACCCGGACTTCAACAACGAAAAGAAGCCGGTCAAAGCCGAGCCGGGCGCTCAGG
GCTACATCACTCAGGGTGCTAACGGCGGTCTGCCGGACAAGGTGCGTCACGGTGCGTTCGTGAACGTGCCTGAGT
CTGTGCTCAAGGTGACGAAGTCCTGGACCGCTGCCAACCCGACGCACATCGAGGCTGTTGACTCCACCACCAAGG
CCGTGTACAACGCCGGCACCCGCGAGCTCACCGCCACGGTGACCGCCGCCGATAAGGGCACGCTCGCCGGTTCGG
TGAAGTTCTCTGCTGGCGACTGGTCCAAGACCGTGAAGCTCGACGCCGAAGGCAAGGCCACTGTGACCCTCCCGG
CCAGCGTCTCTGGCACTGTTGCGGTTGCTTACGACGGCTACACCGATGGTTTGGTCAATCCATCCGATACTACGG
TTGACGGCATTGAACAGGGCAAGGTCGATTTGGCTGAGCTCAACAAGCAGATCGCTGCCGCCGAAGCGCTCAAGG
AATCCGACTACACGGCCGATTCCTGGGCCAAGCTTGCCGCCGCGCTGAAGACTGCCAAGGCCGCGCTCGCCGCTG
AGAATCAGGGCGAGGTCGATACCGCCGCAGCCGACCTTAAGACCGCAATCGAAGCCCTGCAGAAGGCTCCGACCA
ATCCGGGCGAAGGTGACGGAGATAAGGGCGACGGCAATAAGCCGACTACCCCGACCACCGGCGACAAGACCAACG
TCAACAAGCCCGGCAGCGCGCTGAGCAATACCGGTACGGCCGTGCTCGGCCTGGGTGGTGCCGTGGTAGTACTCG
CCATCGCCGGCATCTCCCTAACCCTCTGGCGCAAGCGTCGCGCCTGA

SEQ ID NO: 10
ATGGGAAAGCTGATACGAAAGGCAACCGGACTCACGGTCGGCGTGGCAACACTGCTCGCTGGTCTGGTGCTGCCG
ATGACGGCCAGTGCCGAGAGCGCATCGCCAATCGATGCCAGTCCGATCATCCACTATTCATTCGATAACGCACTG
ACGTCCAAGACCATCGCCAACGAGGGCAGCGCGGCCAACAGCGATGCCACCCTATCCGGCGACGCCACGGTGGCC
AATGGCCAGATCAACCTGACCGGCTCGCAAACCATTAGCGTGCCGACCACGGCCATCGCCGGTAAGAAGGACGTC
ACCGTCTCCATCTGGCTCAAGAACAATTACGGCAACGGCAATACCGCCGCCGCGTACATCGGCGCGGCCAAGACC
GGCAATTATCCGGCCAACGGTTACTGGCTGCTCAACCCGGCCAACCCGAGTGGCTACGCGAAATCCGTAATGACC
AATGCCACTGCGGCCGACCCGAATAACAGCCCGTGGGGCACCGAAGTCGGCCCTGGATCGACGAACGCCGCCATC
ACCGGCACCAAGGCCACCAGCGATTTGGCTCTGTACACCACCGTCATCAACGGCACCAACAGCACTATGAGCTTC
TACCTCAACGGCAAGCAGGTTGGAGACGCCACCTACGCCATTCCGGCCGGTGGCCTGACCAATTACGGCGATCTC
GTCGCCTACATTGGCAAGTCCTCCTACGCTGACCCGAACTCCAAGCTCGACGTGGACGATTACGCCGTATACGAC
ACTGCCATCAGCGCCGCAGACGTGACCAAGCTGTATGACGTTCAGGTGCTCGACAAGGCCGAGGCCGCTGTCAAG
GCCGCTGTGCCCGCATCCGCTACCGAGGACTTCACCCTGCCGACCAGCGCCGCTGGTGTGAGCGTCGCGTGGAAG
TCGGACAACGCAGCCATCGCCGTTGACAACGCCACCGGCAAGGCCACGGTCACTCGTCCGGCCGCAACCGCAGCT
GATGCCGAGGTGACCCTCACCGTCACGTTCGGCAACAACGCCAAAACCGCCGCCTACACGGTCCTCGTGCCGAAG
CAGCTCTCCGATGCCGAGCAAGCCAAGGCCGACCTTGACGCCATCACCATCGAGGACTCCGACGACATCCGTAGC
AACTTCTCCGTGCCCACCAAGGGCAACAATGGTTCGACCATCTCGTGGGGAGTGACCGGTGGCAAGGATATCGCC
ACACTAGGCGAAGGCGTGAGCGACAAATCTCGAACGGTCACTGTTAAGCGCCCTGCCGCCGGTAGCGATGCCGCC
ACTGTGACGCTCAAAGCCACTGCCAAGTACGATACCGCCACTGAAACTAAGACCTTCACCGTCACCATTCAGCCG
ATGCCTGCCGCCGAAGAGAAGGACGAGGCCTACGTGTGGGCGTTCTTCACCGGCGAGGGCGTGGGCGGCGAGAAA
ATCAGCCTCGCGGCCTCCAAGGGCAACGATGCGCTCGACTGGAACACGCTGAACAACGGCACGCCGATATTCACT
TCCGAGTTTGGCGAGAAGGGTTTGCGCGATCCGTTCATCATGAAGTCCAAGGACGGCGACAAGTTCTACATGCTC
GCCACCGATCTGAAGATTGACGGTCGTGCCCCCCTCAACGGGCTGAATGGCTTTGCTGGTGCACAGGCTAACGGT
TCCAAGTACATTGAGATCTGGAAGTCCGACGATCTGGTCAACTGGTCCAAGCAAAGCCACGTCAAAGTGAGCTCT
GATTACGCAGGCAACACTTGGGCGCCTGAGGCCTACTACGACGAGGAAATCGGCAAGTACGTGGTCTATTGGGCC

TCGAACCTGTACGACAACACCGACGAGAACAGCCGCAAGCAGCTGACCTACAACCGCATGGTGTACGTCACCACC
GATGACTTCGTCAACTTCTCCGACCCGACAGTGTGGATTGACGTTGATCGCCGAGGCGGTGCAGGCAGTGGATCC
ATCGATGTGACCGTGCAAAAGGTAGGGGATACCTACTACCGCATCTACAAAGATGAAAACACGATGTCTTTGCGT
CAGGAGAAGTCCACAGATTTGACTGCCGCAATTGGTGGTGCCGGCGTGAAGAACTACGCCGATGCGCTTAAGGGT
AGTGCATGGAGCGAAGTTGCCACGAACATCGGTAAAGGCCAGGCTAACGGTTACGGTAAAACCTTCACTTCCGGC
GAAGGTCCATCGCTATTCAAGGCCAACGATGGCGATGTGAACGGCTACCAGTACTACCTGTTCGCCGACCAGCCG
AGCTATCATCAAGGTCCAAACCACTATGTGCCGATGGCGACTGAGGATATCGCCAGCGGTCAGTGGACCGTTATC
GGCAATAAGATGCCTGAGGCGAACTTCCCGACCAACTCCGATGGCGGCAAGCCGCGCCACGGAACCGTGCTGCCC
GTGACCCGCGCCCAGTACCAGAAGGTGCTGGAGGCATACGCCCCGGCTGTGGCTGTGAAGTCCGTTGACGCGCTG
TCTGCCGAGACAACGGTTGGTGTGGCTCCGACGCTGCCGGAGACCGCGCATCTGACTCATGCGGACGGTTCCGTT
TCTGACGTTGCAGTTGAGTGGGATGCCATTGACGCATCTTTCTACGCCAAGACCGGCACCTTCACCGTCAAGGGC
ATCACCCAAGACGATTCCCGTATGCCGGTTGAGGCTACCGTCATTGTGAACGGCATCGACCTCTCCAAGGCGACC
GTCACCGTCGAACCCAACGAGTTCACCGCAGACGGCGCTGCCAAGGAACCAGCCGTGACCGTTGTACTCGATGGC
GCGACGCTCAAGGAAGGCGCCGACTATACGGTGGCCTATACGAACAACGTCGAACCTGGCACTGCCACAGTGACC
GTAACCGGCGCTGGCAAGTACTCCGGTACTGTCTCGGCAACGTTCACCATCAAGGCCGCCGAGCCCGGCTCCACG
CTGGACAAGTCCAAGTTGCAGGCGCTTGTCGATAAGGTGAAGGGCTATAACAAGGCTGATTACCAGTCTGGTTGG
GATGCTTTCGCCGTCGCGCTCGCCGACGCGCAGCAGGTGTTGCAGAACTCCACCGACCAGCAGGAAGTGGACAAG
GCGTTGTCTCGGCTCCAGTCCGCCGTCGACAAGCTGGTCAAGAAGTCCGGCGATTCCGGCAAGACCGATGGCAAG
GATGACGGCACGCAAAAGCCCGCCGCCAAGCCGGGCAGCGCTCTGTCCAACACCGGCGCCTCGGTGTTCGGTGTG
GGTATCACCGCGGTCATACTGCTCGCCGCCGCCGGCGCCGCCTACGCCTTCCGCAAGCGCCGCGCCTGA

Suitably, the GH43_22 gene may encode a protein shown as SEQ ID NO: 11 or 12, or a sequence with at least 80% sequence identity to SEQ ID NO: 11 or 12. Preferably, the *B. Longum* transitional strain comprises a GH43_22 gene encoding a protein shown as SEQ ID NO: 11 or a sequence with at least 80% sequence identity to SEQ ID NO: 11m and a GH43_22 gene encoding a protein shown as SEQ ID NO: 12, or a sequence with at least 80% sequence identity to SEQ ID NO: 12. Suitably, the protein may comprise a sequence with at least 85%, at least 90%, at least 95%, at least 98% or at least 99% sequence identity to SEQ ID NO: 11 or 12
SEQ ID NO: 11 
MKHWKKMAASLVAISTMMAVVPTTYAMESEDSQPQTTDTATVQTTKAAEPTLLASWDFTGKNGTTNSAIADSTGK
YNLTLKDGAKIEQYGDRSTNEALSLRGDGQYAQIDDQLFKDAGDSFTLEFASKTRHDDSGKFFSFIVGKDGSNDA
NTTDQANANKYLMFYNSKTAIKGVISNNNWGNEQGSKVTVSGNDNSWADYKIVVDGTNLAVFRNNALIIFKANTG
IKMSDLGATTAYIGKSFYSVDEYWNGAMDDIKVYRGADLTMPTAVAISGTGVVNNKLTLIEKDSTKLTATVTPDD
AVSKNVTWSSSDESVAKVAADGTVTGVKAGTATITATTELGGVKAELPVTVEPMNAQNAAAADLDAAIAALKVPA
AENLPLVAKGTKNGSAITWKSSDEKLITSTNEKYENKTTGADDPYRGAGIINRPAYGDGDSKPVTLTATASYNGG
EKVTKTIEVTVKEKTRIAPDTGYAAVTFESDSNGGEKAWVASTEKNDFFTFKTRNNGQAVLTNDADTGGLRDMFV
LRSHEGDKYYLIATDLKVSSMGWSQNQVNGSRKVEVYESTDMMNWTRTNGDGNGGITINTPNAGMTWAPEAYWDD
DLNAYVVFFSSRMFTDDTRTTPVKNDKTGNSSYAQVRYAITRDFVNFTEPQMWQDTGYSRIDSTVRKIGGYYYRF
TKNEQGGAAGDYITTGKSIFLERSKVLTAPTTEASPGQDPNTGWQLLEQALLPFEGPETIKLNKDDELNTKDDDG
YILLSDNFAYRAFMTTGAELSKTTWDNPMTKRYPDFNNEKKPVKAEPGAQGYITQGANGGLPDKVRHGAFVNVPE
SVLKVTKSWTAANPTHIEAVDSTTKAVYNAGTRELTATVTAADKGTLAGSVKFSAGDWSKTVKLDAEGKATVTLP
ASVSGTVAVAYDGYTDGLVNPSDTTVDGIEQGKVDLAELNKQIAAAEALKESDYTADSWAKLAAALKTAKAALAA
ENQGEVDTAAADLKTAIEALQKAPTNPGEGDGDKGDGNKPTTPTTGDKTNVNKPGSALSNTGTAVLGLGGAVVVL
AIAGISLTLWRKRRA
SEQ ID NO: 12

MGKLIRKATGLTVGVATLLAGLVLPMTASAESASPIDASPIIHYSFDNALTSKTIANEGSAANSDATLSGDATVA
NGQINLTGSQTISVPTTAIAGKKDVTVSIWLKNNYGNGNTAAAYIGAAKTGNYPANGYWLLNPANPSGYAKSVMT
NATAADPNNSPWGTEVGPGSTNAAITGTKATSDLALYTTVINGTNSTMSFYLNGKQVGDATYAIPAGGLTNYGDL
VAYIGKSSYADPNSKLDVDDYAVYDTAISAADVTKLYDVQVLDKAEAAVKAAVPASATEDFTLPTSAAGVSVAWK
SDNAAIAVDNATGKATVTRPAATAADAEVTLTVTFGNNAKTAAYTVLVPKQLSDAEQAKADLDAITIEDSDDIRS
NFSVPTKGNNGSTISWGVTGGKDIATLGEGVSDKSRTVTVKRPAAGSDAATVTLKATAKYDTATETKTFTVTIQP

MPAAEEKDEAYVWAFFTGEGVGGEKISLAASKGNDALDWNTLNNGTPIFTSEFGEKGLRDPFIMKSKDGDKFYML
ATDLKIDGRAPLNGLNGFAGAQANGSKYIEIWKSDDLVNWSKQSHVKVSSDYAGNTWAPEAYYDEEIGKYVVYWA
SNLYDNTDENSRKQLTYNRMVYVTTDDFVNFSDPTVWIDVDRRGGAGSGSIDVTVQKVGDTYYRIYKDENTMSLR
QEKSTDLTAAIGGAGVKNYADALKGSAWSEVATNIGKGQANGYGKTFTSGEGPSLFKANDGDVNGYQYYLFADQP
SYHQGPNHYVPMATEDIASGQWTVIGNKMPEANFPTNSDGGKPRHGTVLPVTRAQYQKVLEAYAPAVAVKSVDAL
SAETTVGVAPTLPETAHLTHADGSVSDVAVEWDAIDASFYAKTGTFTVKGITQDDSRMPVEATVIVNGIDLSKAT
VTVEPNEFTADGAAKEPAVTVVLDGATLKEGADYTVAYTNNVEPGTATVTVTGAGKYSGTVSATFTIKAAEPGST
LDKSKLQALVDKVKGYNKADYQSGWDAFAVALADAQQVLQNSTDQQEVDKALSRLQSAVDKLVKKSGDSGKTDGK
DDGTQKPAAKPGSALSNTGASVFGVGITAVILLAAAGAAYAFRKRRA

Suitably, the present *B. Longum* transitional strain comprises a glycosyl hydrolase family 43_27 (GH43_27) gene. Suitably, the GH43_27 gene comprises SEQ ID NO: 13 or a sequence with at least 60% sequence identity to SEQ ID NO: 13. Suitably, the GH43_27 gene comprises a sequence with at least 60%, at least 70%, at least 80%, at least 90%, at least 95%, at least 98% or at least 99% sequence identity to SEQ ID NO: 13.

SEQ ID NO: 13
ATGACAACCAAACCATCGATAGGCAAACGCCTGCTCGGCGCGATGCTGGCAGTGCCGATGGCGCTCGCCGGCATG
GGAATCGGCGCGACCACGGCGGTCGCGGCCGATACCGTTCCGACCAATAATCTCATCGCCGCCTACGACTTCACC
ACGAAGCCAAGTGACGGCAAGACCGTGGCCAACAGTGCGCCGAACGCTACGCTTGGCGCGGCCGAAGTACAGAAC
TCCGCCGACTCGCTTTGGGCCGATGATGCCCTCACCCTTTCCGGCGGTGCCAAGACCGGCACCGGCGACTGGGTC
AAGCTGCCCTCGAATCTGCTGTCCGGCAAGGACGCCGCCACGGTGCAGTTGGAGGTCAAAGCGGATTCCAGCATG
CTCAATGCTTTCCATTTCCTGTGGAACATCGGTAACGACAGCTCCGATACGGAGTATTTCTTCGCCACGCTCAAC
TGCGGCAGTTCGCGTAACCCGCTCGTCGGCCTGAAATCGGGCGGTACGGAGACGCTCGTGCAGTCCAGCTCCTGC
GTGGCCAAGGCCGACCAATGGTTGTCGGTGACCGCCACCATTGATGGCACCGCCGCGAAACTGTACATCGACGGC
ACGCAGGTGGCATCCGGCACCGTGCCGGCCAAACTGTCCAGCGTCAAGGACCAGTCGCTCAACACCATCGGCCGT
TCGCCGTGGCCCGACAACCTGTTCAAGGGCGCGGTCTCGAACTTCCGCGTATACGATGCCGCGCTCACCGCCGAT
CAGGTCGCCGCGATCAGCACTGCCGATGCCTCAATTCATGCCGGTGAACTCACCGGTTCCGTGCTGAACGGCATC
ATCATCCCCACGACGGTCGACGATCCGTTCATTTCGCTGCCCACTGCGAACGGCGTGACGTGGGCGTCCTCCGAT
AGCAGCGTCATCGCGACTGACGGCACGGTCAACCAGCCCGCCAAGGGCGAGGCAGCCAAGACTGTCACGCTGACC
GCCGCCGTCACGATCCGTGGCCAGACCGCTACGAAGGAATTCACGGTCACAGTCAACCCGACCACGAAAACTGCC
GCTGAACAGCTCAAGGAAGCCGCGGCCGGCTACGTGATCCCGTCCGTCGTGCGTTCCGGAGACGCCCTCCCGGCG
GCTGTGAATGGCACTACCGTCACGGTTACGTCCACTAAGGACGTAGCCGTCGAGGATGGCAAGATCACCATCGAT
GGCGACGAGGCCACGACCGGTACCATCACCGTCGAGTTCTCCAAGAACGGTCTCGCCGGCATCGAGCCCATTACC
AAGGTCTTCACCGTAAAGGTGCTGCCCGCCGCGAAGTCCGCGACCATCGCCGCCTATGATTGCAACGCCACCAGC
GCCGACGAGGCCAACAACGGCGACATCGCCTACAGCATGCACCTCGCGTTGCAGAACGCTGACGGTTCGTACACC
CCGTACAACGAGAATTACGGTATCTTCTTCGCACGTTCGCCGAAGGCGCAGAATCTCAACGAGAACCTCGACGGC
AATGATTACCGCAGTCTCAAGGATCCGAGCCTGCTCCGCATGGCCGACGGCACCTATGGCGTGATTTCCGTGCGT
ACCAACCGCGGCACCGCCACCGGTGACTCCACCGCGAAGTCCAGCGTGCTCATCGCCACCTCCGAAGACCTGCTC
ACCTATAGCGAACAGGAGAACTCCGGTTCCATCGTCGACCTTGGCGAGACCAACGGCGTCAACGCTCCGTACGCC
GTGTACGACACCGCCAGCAAGCAGTATGTTGTCGGCTGGGCCGATGACAACGGCGTGGCCAAGTACACCACGTTC
GATTCGCTCAAGGGCTCCGCGTCCAAGCATGGCAGCGTACTGTACGGTTCCATCGCCAAGTCCGGCGTACTCGAT
GCCGACGGCGTGCAGGGCATCGCGAACTTCCGCTCCGGTGCCACCATCGCGGTGGACGAGGCGACCGTCAAGGCG
CTCAACACCCGTTACGGCCGCTCTGAGAACACCGGCACGAGCAATCTCACTGACATCACCGTCGAGAAAGGTTCC
TCGATTGATGAGATGACCTCGCAGCTGCCGAAGAACGTGGACCTCACTTACTCCGACGGTTCTACCGGCTCCCTG
CCGATTTCCTCATGGAACACTGAGGGTATAGATCTGACGAAGGTGGGTGATTACACTGTCACCGGCACCGTCAAG
CAGACCGAATACCAGATTCCGTTCGCCGAGGACCGCGCCGATCCATCGGTGTATAAGTGGCAGTGGACGCATGAG
GTCGACGGCAAGGAAGTCACCGAAACCAAGTTCCTGATGATCGCTTCCAACGACATCCAAGGTGATGTCACTTGG
CAGCATGGTTCGCCCCACATGCCGTTCCGCATGGCCGACACGATTTCCGGTCTCGCCGACGAGCCGGGCAACCCG
AATGCCCTGATTCAGTCGAACGGCTACAACAACAAGGAGGTGTCGCTGCTCAAGGCTGGCGACAAGGACTCCGAG
GGTAATGCCATCATGCACAGCTTCTGGGCTCCAGAAATTCATGAGATTGATGGTAGGCTCACGATTCTGTTCATG
GCCGGATACGGCAACACATGGTCCAACGGCAAGTCGGTGTACATGCAGCTCAAGCAGGATGCCGACGGTCATGAC
CTCGACCCGACCGACCCCGATAACTGGACTGTGCCGACACCGATCTACCGCAATGACGCCTCGCTGCTCAACGGT
AACAAGCAGCTCGCAGCCACAGCGTCCGGCGGAGTGGGCATGTCGCTCGACATGACCTATTTCCAGGATGCCGAC
GGCAGGTCCTACTACGCCTGGCAGCAGCTCGGCGCCACCTACATCGCCACGATGGATCCGAAGGACCCGGCCCAT
GTGACCAGCTCCCCGGTGCGCATCGTCACCCCGGAGTATGCGTGGAACGCCGCCATAGCCGAAGGTCCGAACGTG
ACCCTGCGCGACGGCAAGCTGTACCTCATGTTCTCCGGTTCCGGCGTGGGTAAGACATACACCACTGGGCTGGCC

GTAGCGGATGCCTCCGGTACTGACCTGACCGACCCGGCCAGTTGGACGGTGCTCAACTACCCGATTCAGAAGTCC
GGTCCGTTCAACGGTGAGATGCAGCTCGGCACCGGTCACGGCATGTGGAGCGAGGACGAAGATGGCAACCAGATC
TACGTGTTCCACGCCTATGCCACGAAGAATCTCGGATCCGTGAATGCTGCCGGCCGCGACATGTTCGTGCGCCGT
GTGCACTGGGCCGCCGACGGCATGCCGGTGTTCGACATGAGCTCTTCCGAGGAGCTGGCGAACAAGATCGTTTCC
GTTACGGTGCATGTGGTTGACGATGCGGTTGCGGTCGATAAGTCTGGTTTGTCCAAGGCGCTTGCGTCCGCCAAG
CAGCTGCACGGGTCCGACTACACCGCCGCCTCGTGGAAGGCGTTTGCCACGATGCTGGCCTCCGCTGAGAAGGTC
TATGCCGACGATACTGCTACGCAGAAGGACGTCGATGACACGACCGTCGCGTTGGTCAAGGCGCAGGCTGCGTTA
GTGAAGATTGATGGTTCCGATTCAGGCGATGGCTCGGGCGATTCGACTAAGCCGAGCGACGGTTCGAGCGTCGAT
GCGGGAGATAAGACGTGCAACAATCTTGGTTTGTCCAAGACCGGTGCGGCTGTGCTTAGTCTTAGCGGCGTAGCC
GTGGCGCTTGCTGTCGCCGGTATCGCTCTGACTCTCCAGCGCAAGCGTCGCGCCTGA

Suitably, the GH43_27 gene may encode a protein shown as SEQ ID NO: 14 or a sequence with at least 80% sequence identity to SEQ ID NO: 14. Suitably, the protein may comprise a sequence with at least 85%, at least 90%, at least 95%, at least 98% or at least 99% sequence identity to SEQ ID NO: 14.

SEQ ID NO: 14

MTTKPSIGKRLLGAMLAVPMALAGMGIGATTAVAADTVPTNNLIAAYDFTTKPSDGKTVANSAPNATLGAAEVQN
SADSLWADDALTLSGGAKTGTGDWVKLPSNLLSGKDAATVQLEVKADSSMLNAFHFLWNIGNDSSDTEYFFATLN
CGSSRNPLVGLKSGGTETLVQSSSCVAKADQWLSVTATIDGTAAKLYIDGTQVASGTVPAKLSSVKDQSLNTIGR
SPWPDNLFKGAVSNFRVYDAALTADQVAAISTADASIHAGELTGSVLNGIIIPTTVDDPFISLPTANGVTWASSD
SSVIATDGTVNQPAKGEAAKTVTLTAAVTIRGQTATKEFTVTVNPTTKTAAEQLKEAAAGYVIPSVVRSGDALPA
AVNGTTVTVTSTKDVAVEDGKITIDGDEATTGTITVEFSKNGLAGIEPITKVFTVKVLPAAKSATIAAYDCNATS
ADEANNGDIAYSMHLALQNADGSYTPYNENYGIFFARSPKAQNLNENLDGNDYRSLKDPSLLRMADGTYGVISVR
TNRGTATGDSTAKSSVLIATSEDLLTYSEQENSGSIVDLGETNGVNAPYAVYDTASKQYVVGWADDNGVAKYTTF
DSLKGSASKHGSVLYGSIAKSGVLDADGVQGIANFRSGATIAVDEATVKALNTRYGRSENTGTSNLTDITVEKGS
SIDEMTSQLPKNVDLTYSDGSTGSLPISSWNTEGIDLTKVGDYTVTGTVKQTEYQIPFAEDRADPSVYKWQWTHE
VDGKEVTETKFLMIASNDIQGDVTWQHGSPHMPFRMADTISGLADEPGNPNALIQSNGYNNKEVSLLKAGDKDSE
GNAIMHSFWAPEIHEIDGRLTILFMAGYGNTWSNGKSVYMQLKQDADGHDLDPTDPDNWTVPTPIYRNDASLLNG
NKQLAATASGGVGMSLDMTYFQDADGRSYYAWQQLGATYIATMDPKDPAHVTSSPVRIVTPEYAWNAAIAEGPNV
TLRDGKLYLMFSGSGVGKTYTTGLAVADASGTDLTDPASWTVLNYPIQKSGPFNGEMQLGTGHGMWSEDEDGNQI
YVFHAYATKNLGSVNAAGRDMFVRRVHWAADGMPVFDMSSSEELANKIVSVTVHVVDDAVAVDKSGLSKALASAK
QLHGSDYTAASWKAFATMLASAEKVYADDTATQKDVDDTTVALVKAQAALVKIDGSDSGDGSGDSTKPSDGSSVD
AGDKTCNNLGLSKTGAAVLSLSGVAVALAVAGIALTLQRKRRA

Suitably, the present *B. Longum* transitional strain comprises a glycosyl hydrolase family 43_29 (GH43_29) gene. Suitably, the GH43_29 gene comprises SEQ ID NO: 15 or a sequence with at least 60% sequence identity to SEQ ID NO: 15. Suitably, the GH43_29 gene comprises a sequence with at least 60%, at least 70%, at least 80%, at least 90%, at least 95%, at least 98% or at least 99% sequence identity to SEQ ID NO: 15.

SEQ ID NO: 15

ATGAGTTTCCATGTATCCGCGCAATCGGTTCGCGCGGTGGCCGGTGGACTCGTCGCCGCAGCGACATTGCTGTCA
GGCCTTGCCCTTGCGCCGACCGCAATGGCCGCCGATTCAGCCACCGCTGACAACGCGCCCAGCGTTGCCGGTCAC
GCGTATAACGAACTGCCGTATAACAATCCTGATGTCACCGTCACCCAAATCGACAATAGCGCACTGCCCAGCTAC
ATGCGCAACCCCATCGGGCAGAACGAGGGTATTGACACCCCGAACGATCTTTCGCAGAACTACTACTCTGCAGAT
GCATCCGCGCTGAGCTATGACGGCAAACTCTTCGTCTTCACCGGTCACGATGAGGCTTCGCCCGACTACGGCTCC
TTCAACATGAAGGACTGGGGCGTATACGTCACCGATGAAGACGGCCTGAATCAAGGCAAATGGACACATTACAAG
ACCATCGCCAAGGCAGACCTGTTCAGCTGGGCCACCGGCGATGGCGCGTACGCCGGCCAAGTCGTAGCCGACGAT
AACGGCACCCCGAGCGACACTTCCGATGATTGGTTCTACTACTACGTGCCGGTGAAGGACAAGGCTTCTGAGGCG
GCTGGACAGGACCCGTTCGCCATCGGCGTGGCCAAGTCGAAGAGTCCGCTCGGCCCGTGGAAGGATACCATCGGC
AAGCCGCTGCTCACCACATCGCAAACCCAGATTGAAACCATCGATCCGGCATTCTTTGTGGACGAGGATGGCACC
GGATATTTGCACTTTGGTACGTTCGGCACTCAGCTCGCCATCAAGATGAAGAAGGACGCCACAACCGGCCGCACC

TCATACACCGAGGTGGAAACCAAGGCTGATGGCACCACGCCGAACCTCCACACCATGAAGGACGCGGACAGCAAC
GCGAACGGCCCGAAGGGATTCTTCGAGGCGGCGTGGGTGTTCCGTAAGGGCGATACCTATTACAACGTGTACGAC
GGCGGTAAGCCCGGTTCGGGCACGGCCACCTGCGTGGAATCGAACTATCAAGCTTGCATCCAGTACTCCACTTCC
GACAGCCCGCTCGGCCCATGGAAGTACCAAGGCGTAATCGTGCCTTCTGGCTCGGCCACCACGATGCACCCCTCG
GTGCTCCAGTTCGGCGACAAATGGTATGTGACCTATCACACCGGCGACAAGGAAGGCGGCACCGATTTCCGCCGT
GCCGTGTGCATTGATGAAGTCGATTGGACCGCCGACGGCCAGATGGTTTCCACCGCCCATCCAACCAAGGCCGAG
AAAACGCAGCCCTCCACCAACGTGGCTCCGTACGCAAAGGTGAGCGCCACGTTCACTGAAACGCCTGCTTGGAAG
GGTTCGGTGAACGACGGCCGTGTGTTGCAAACCGCTGTGGTCCCGCCGAATCACTGGACCAACTACCGTTCTATC
CCGCAATCGCAGTCCGGCGATTCTCTGGTCTACCAATGGGATGGCACTGTGCGCGTCAACTCGTCTAAGGTTTGG
TTCGACGTGGATTCCAACGCTCTGCGCGCGCCCGCCTCGTGGAAGATTCAGTACTTGGACGCGGACGGCACATGG
AAGGATGTCATCAACCCGAGTGCCTATACAACGACCACAGGCAAGGCCAACCCCAACGCCGTCACCTTCGATGCG
GTGACCACTACTGCCTTAAAGCTCGACATGACCGGTCAAGCTGTGGATGGCGGCTATGCCTCCGTGGCCGTTGCT
GAATGGGAAGTCGGCTCCGACTCCAGCGAATCGCCGGCAATCACTGCGCCGAAGAGCGTGACCACCGCCACCGGT
ACTGCGCCTACTCTGCCGGCCACAGTGGATGTGAAGTACGGGAACCCAACCGTTGCCTCCCCAGTAATTTGGCGT
CCAGTTGATGCTTCCTCGTATGCCAAGGTCGGTTCGTTTACGGCCTACGGCGTGGTCGCCGGCGTGCCCGGTGAG
GCAAGCGAGCAGGGCAATGTGTCGGTAAATGTCACCGTGCAGGACGGCTACCAGCCTGCCGCTGATACCACGAAG
CCGACTGTAACCGTTGCCGTTACTGCTAACGCAGGCAATAGCGAGTGGCTCACCACCGCTCCGTTCGCCACCGTG
CAGGCCACGGACGACACCGCACCTATCGCCAAGCTGGAGATTTCCGCTGATCAAGGCAAGAGCTGGACCACCATC
GCCGCGAATGCAAACGCGGCCATTGCCACGCTTTCCCAGCAGGGCGATGTCGAAGTGTGGGCTCGCGCCACCGAT
CAGGCCGGCAACGTTTCCGACGTGGCCAAGGCCGGCGGCAAGGTGGACTCCGCCGCGCCAACCGTGACCGCCGCC
GCCGATAAGGAGGAGCGCACGCTGACCTTGACCGCTGATGACGGCACCGGTTCCGGTGTCGCATCAATTGAATAC
CGCATTGGCACAGACGGTCAATGGGCCACGTACAGCAAGCCGATTGCTGCACCGAGCGCGTCGCGCGCCACCGTG
TACTACCGCGCCACCGATAAGGCCGGCAACGTGTCCGCTTCGGCGAAAACCGACATTCCATCCGACACTTCCGTG
CCGCTGACCGGCTACATTGAGGGCGATGCCACCGCCACCGATGTGGACGGCAAGGCATCCGGCTGGGTCAAGGGT
GCCGCCGCGTTGAACGACGGCAAGATCATTCCCGATATCACCATTGCCAACGAGGATGTCTGGGGCACTTGGCCC
AACACCGGTGAGATGCGCCTCGACTACGAGTGGGACCGTGAAGTGACTATCGACTCTAGCCGCGTGCAATTCACC
TCGGATGATGGCGGATTGGGTATTCCGGCATCGTGGGAATTGCAGTACTGGGACGCCTTGGCGAACAACGGTGCC
GGCAACTTCGTGGATATTCCCGACGCCACCTACACTGTGACCGCCAATTCACCGTCTGCTGGCTGGGCCACCGGC
GATGCCAAGGGGTGGTCTGATGGCACGTGGAACACTCCGGTCAAGACTACCAAGTTGCGTATGGTTATCACGTCC
GGCTCGGCTTCTCCGGCTGTTGCCGAATGGCAGGTTCATGCCATTGACGACAGTACGCCTGAGCCGCCTGAGCCC
ACACCGATCGACAAGACCGAGCTCAAGCAGGCGCTCGCTGACTCGCCTAAGGCTGACGATGCCTCCAAGTACACC
GAGACTTCATGGGCGGAGTACGCGGCGGTATTGGATTCGGCGCAGCAGGTGTATAAGGCTGAGGATGCCACCGAA
GCTGCGGTGGTGGATGCCGCAACCCAGCTGAAGCAGGCAGCGAAGAAGCTGGTGCTTGTAGCTACGGTGCAAGAT
CGTGCCGCGCTGAGCGCTCAGCTCGATGCCGCTGCTGCCGTGGATCGCACAAAGTGGACTGATGAATCGCTGGCC
GTGCTTGATTCGGCAGTCGCTACGGCGAATGCGCTGACGAGTGATGGTCAGGCCGCCCAGTCTGACGTACAGGCT
GCGACTGAGGCAATCAGCGATGCCATCGCGGGTCTGGTTGAGAAGAGCACCACGAAGCCTGGCCAGGGTGGCGAT
AAGCCCGGTTCCGGCACGGACAAGCCCAACCAAGGCAACGATTCCAACCAGAACAAGGGTGATGCAGACTCCGGC
AAGCACAAGAAGATACCTGACACCGGTGCAGCCGTGCTTGGTGTTGGCATCCTCGCCGTGGTACTTGCTGTTGCG
GGTGTAATCATCCTCAAGCGCCGCAAGTCCGGTACCTGCTAG

Suitably, the GH43_29 gene may encode a protein shown as SEQ ID NO: 16 or a sequence with at least 80% sequence identity to SEQ ID NO: 16. Suitably, the protein may comprise a sequence with at least 85%, at least 90%, at least 95%, at least 98% or at least 99% sequence identity to SEQ ID NO: 16.

SEQ ID NO: 16

MSFHVSAQSVRAVAGGLVAAATLLSGLALAPTAMAADSATADNAPSVAGHAYNELPYNNPDVTVTQIDNSALPSY
MRNPIGQNEGIDTPNDLSQNYYSADASALSYDGKLFVFTGHDEASPDYGSFNMKDWGVYVTDEDGLNQGKWTHYK
TIAKADLFSWATGDGAYAGQVVADDNGTPSDTSDDWFYYYVPVKDKASEAAGQDPFAIGVAKSKSPLGPWKDTIG
KPLLTTSQTQIETIDPAFFVDEDGTGYLHFGTFGTQLAIKMKKDATTGRTSYTEVETKADGTTPNLHTMKDADSN
ANGPKGFFEAAWVFRKGDTYYNVYDGGKPGSGTATCVESNYQACIQYSTSDSPLGPWKYQGVIVPSGSATTMHPS
VLQFGDKWYVTYHTGDKEGGTDFRRAVCIDEVDWTADGQMVSTAHPTKAEKTQPSTNVAPYAKVSATFTETPAWK
GSVNDGRVLQTAVVPPNHWTNYRSIPQSQSGDSLVYQWDGTVRVNSSKVWFDVDSNALRAPASWKIQYLDADGTW
KDVINPSAYTTTTGKANPNAVTFDAVTTTALKLDMTGQAVDGGYASVAVAEWEVGSDSSESPAITAPKSVTTATG
TAPTLPATVDVKYGNPTVASPVIWRPVDASSYAKVGSFTAYGVVAGVPGEASEQGNVSVNVTVQDGYQPAADTTK
PTVTVAVTANAGNSEWLTTAPFATVQATDDTAPIAKLEISADQGKSWTTIAANANAAIATLSQQGDVEVWARATD
QAGNVSDVAKAGGKVDSAAPTVTAAADKEERTLTLTADDGTGSGVASIEYRIGTDGQWATYSKPIAAPSASRATV

YYRATDKAGNVSASAKTDIPSDTSVPLTGYIEGDATATDVDGKASGWVKGAAALNDGKIIPDITIANEDVWGTWP
NTGEMRLDYEWDREVTIDSSRVQFTSDDGGLGIPASWELQYWDALANNGAGNFVDIPDATYTVTANSPSAGWATG
DAKGWSDGTWNTPVKTTKLRMVITSGSASPAVAEWQVHAIDDSTPEPPEPTPIDKTELKQALADSPKADDASKYT
ETSWAEYAAVLDSAQQVYKAEDATEAAVVDAATQLKQAAKKLVLVATVQDRAALSAQLDAAAAVDRTKWTDESLA
VLDSAVATANALTSDGQAAQSDVQAATEAISDAIAGLVEKSTTKPGQGGDKPGSGTDKPNQGNDSNQNKGDADSG
KHKKIPDTGAAVLGVGILAVVLAVAGVIILKRRKSGTC

Suitably, the present *B. Longum* transitional strain comprises a glycosyl hydrolase family 121 (GH121) gene. Suitably, the GH121 gene comprises SEQ ID NO: 17 or a sequence with at least 60% sequence identity to SEQ ID NO: 17. Suitably, the GH121 gene comprises a sequence with at least 60%, at least 70%, at least 80%, at least 90%, at least 95%, at least 98% or at least 99% sequence identity to SEQ ID NO: 17.

SEQ ID NO: 17

ATGCATCAATCAACACGAAAGCGGTGGCTTGCGTCAATCGGCGCGGTTGCAGCGGTCGCCACACTGGCCACCGGC
GGTGCAGTCACCGCGCAGGCAGCCGATGCGCCCGTCATCAAGAATGCGGATGTGGCATATCCGTCGTTCAAGGGA
TCTGATGATCCGATGAAGACGGCGGCGAACAACACCACATATAACCCTGCCGTCAGCTATCTGCAGGAGACATTC
GATAACGACGTGAAGAACCTGGCCGGCATCGACACCGACCATGACTTCTGGATCGATAAGATTCTCACCCGTACT
GGTGCACAGCCAACTGGTAAAGGCACGAACGACAAGGGTGCTTACTCGTATGAAGGCTCCGACGGCAACAACTAC
CTGTTCACCCGTGGTCGCGCCGCCTACATGTACACGCACACGCCTAATCAGCTCGGTTTTGTGGGTGATACCGCC
TACTGGGACCAGACCAGCAGGAGCGGCTTCACCGTTACCGTAAACGCTGATGGATCAAACCAGACCCTTAACGAA
GACGCCTCCCAGCGCAAGCAGACGCCGAGCTACTTCACCTCCCTGTTCCAGACCGGTGGCAAGAGCCTCAAGATC
AAGGAAGTCAAGTACATCACCTACAACAACGTGATGGTTGCGAACCTCACCGTGGAAAGCACGCAGGACCGCGAT
GTCACACTGACCACGGCCTCGCCGTTCGCCGCCGAGGGTGCTGATGGTGCCACCGAACTTACTGGCCGCGTGAAC
GTCAAGAACAACCTGACGACCATCTATCCGCGCTTCTCCGCCAACAACCAGGACGGTTCCAACTGGATCGTCAGC
GGTGGCAAACTCACCAGCACGTTGAGCCTCAAGGCCAACGAACCGCAGACCGTCAAGATTCAGCTCGGCCTGATC
GCCAACGAACTGCCTGACTCCACCAAGGAATATGAGGCCCGTTACACCGGCGACCTTAAGGATGCTGCCGCCTCC
TACAAGGATTCCGTGACCACCTACAACAAGTGGTGGGTCGATAACGCTCCCTACGTGGACACTCCGGAAGACAAT
ATCGATAAGACCGTGGTCTACCGCTGGTGGCTGAGCCGTTTCAACATGCTCGACGCCAACATGCCTGGCAACACC
TTCCAGTACCCGACCTCCATCGAGGGTGTGCTCGGCTACAACAACCAAATCGTGCTCACCTCCGGCATGTTCATG
ATGGACCCCAAGTGGTTCCGCAACCCCGAGTACTCCTACGGCACCTGGCTTTCCGCCGGCGATACCGCCAAGAAG
AGCAAGGCGGGCTATTACTACCACGACAATCCGGGCGACCCGGCCAACTGGAACCATAGCTACACGCAGTAC
ATCACGCGCGCCGGCTGGGACTCCTACAAGGTGCACGGCGGTCCGTCCACCGTGGCCGAGGAGCTGGCCGACCAG
GGTGCCGAGGACGTGCAAGGTCTACTCGCTTCCAAGAGCGAGCCGGACAACAACGACAACCAGAACAACAATGAC
AACAGCTTGATTGACTGGTCCTGGTGGTCGATGACCGGTAACGATGCCGACGCCGTTTCCTTCTCTGAGCCGGGT
CGCTCCGGCCAGCGCATGGATCGCGCCGATGGTTCCGCCAATATGTGGGCCAACGCCAATGCGGCTGCTCAGGCC
TACAAGGCCGCTGGCGATACCGCCAACGCCGAGAAGATGCAGGCCATCGCCGACAAGATCCAGAAAGAAGTCACC
ACTGAACTGTGGGACAAGTCCGACAACCTGCTCAAGCACAAGTGGCTGAACGACGGTGCTTTCGCCAAGTACAAG
GAGATCAATAACTACTACCCGTACTCCGAAGGCCTGATGCCTACCGGCAACGAAGATTACAACAAGGCTCTGCGC
CTGTTCGAGGATTCCAACGAGTTCCCGATCTTCCCGTTCTTCACCGCCAACCAGGCGGACAAGGCGGCGCTGAAC
TTCCCCGGTTCCAACAACTTCTCCATTATCAACGCACAGCCGCTGCTGCAGGTCTATTCAGCCGGCATCCGCAAT
TACGATGCAGCCAAGAACGGTTACATCACCAATGAGCAGTTCAAGAAACTGCTGTACTGGGTGGCGTTCGCGCAC
TATCAGGGCGGCGATAACAACTACCTTGATCAAAACGAGTTCTGGAACGAGGATAACAACAACGTCGGCGATGTA
AACGGTGACGGCGTGATCAACAACCTCGACAAGAACCTTGACGCCGCACAGAACGGCGGCAAGATCACCTACCGC
TCCTGGATCCACCACACCCAGCTCGGCACCACGAACTGGACGATGGTCGAGGACGTAGCCGGTATGGTGCCGCGC
GAGGATAACAAGATTGAGCTGAACCCGATTGAGATCCCCGGCTGGAACTACTTCACGGTGAACAACCTGAGCTAC
CACGGTCAAGATGTTTCCATCGTGTGGGATAAGGACGGCAGCCACTATGGTGGACCTGCTGGCTACAGCCTGTAC
GTGGGGGGCAAGCTCGCCTTCACTTCCGACAAGCTCGCACACCTCATTTACGATCCGTCCACGGGCACCGTTGAG
GATGCCGACAAGGCCGGCGTAACCATCACCAATGCCGCTGGTTCTGATATCAAGGCCGCCAACCAGGTTGCCTTC
ACCGCCGACCAGCGTGTGACCGACCTGTTCGCCAAGTCCGGTGCCAACGTCGACTCCGCTTCCAAGTCCACCACG
AATGTGGCCAAGGACGCGGACGTGACCGGTACCACCTACGCCGAGAAGGACACCAACTACCCGGCCAAGAACGCG
GTGGACGGCAAGACCGTGATGGAATCGTTCTGGGGTACCAAGGGTTCTGAGAACAAGACCGACACGCTCAATATC
AAGTTCAAGGACGGCAAGCAGAAGATCGACGACCTCCGCTTGTACTTCTACCAGAGCTCGTCCAGCCAGACCATC
TCCGGCTATGCCGAGCCCGCCAACTACAAGTTGGAGTACCAGAAGGATGACGGCACATGGGCCCCGATTGCGGAT
CAGGTGCGCACCCCGAACTACGCGGGCGCGAACTACAACCGTATCCAGTTCACTCCGGTGGAGACCACGACTATC
CGCGTCACCTTCACGCCGCAGGCCGGCATGGCCGTCGGTGTCAAGGAGATCGAAGCCTACAACACCGGTATCAAG
GCTGACGGCACTTCCGAGAACCAGGCTCCGCAGGTGGATGCTTACGTGTCTTCCAGCACCTCATCCGGTGCCAAG

CTCGTCGGTACGGTGAAGGATGACGGTCTGCCCGCAGAAGGCGACGTCACCACCAAGTGGGAGCTGGTTTCCGGC
CCCGAGGGCGGTACCGCGAAGTTCGTGGACGATACTGCTGCCAGCACCACCGTCACCTTCAACAAGGAAGGCGAC
TACGTTCTGAAGCTCACCGCTTCCGATGGCGAGAAGGAAGGCTCCAAGGAAATCACCGTTCACGGCATCCCCTCT
GACGGTACCGTGAACGTAGCCCCGCAGTCGAGCGCCTCTGCCAGCTACACCAACGGCTACCAGCCGAAGGACAAC
GCCAAGAAGGTCATCGACGGTCAGGTGGTATACACCAACACGCCGAACGAGACCTGGAACAACTGGGGCGACAAC
ACTGGTGTGGAGCCGTGGCTGCAACTGAAGTGGGCCGGCAAGGTGCCACTGAAGAAGGCCAAGGTCTTCTTCTGG
ACCGATGGCGGTGGCGTGCCGATGGCCTCATCTTGGAAGCTCCAGTACGCTGACGCTGACGGTAACTGGCAGGAT
GTGAAGCTGGCTGACGGCCAGTCCTACACGGTCAATCAGAACGAAGGCAACGAAGTGAAGTTCGCCGACACCGTC
GAAACCGACAAGCTGCGCGTGGTCTTCCCGAAGGGCGCCATCGTGGGTGCTTCCGAGTTCGAGGCGTACGCCATC
GAGCCGGTGAGCGTGGACGAAGTCAACCGACTGGTGCAGACCGGTTCCAAGGCCGATGATCTGAAGCTGCCCTCC
ACCGTGAGCGCCGTATACACCGACGGTTCTCGCCGTGACCTCGCCGTCACGTGGGATAAGGTGACCGACGCTCAG
CTGGCCGCCGATGCCGTATTCGATGTCAAGGGCATCGTCGCTGGTGCGCTGAGCGGTACGGTTGCACACATCGCA
GCTCGTTCCGATACCGCATTGCAGACCGTGGGTAATGCGCAGCCGGTTGAGCAGACCGTCTACCAGAACGCCAAG
TCCATCGACCTGCCCGCCACGGTTCCGGTGAAGTTCCCGAACGGATACAACGACGACCGCAAGGTCACGTGGAAG
GATGCCGACATCAAGGCCATCGACCTGACCAAGGTTGGTGACTACGAGGTGGCTGGTACCGTCGACGACGGTTCG
TCTTCCGCAGCTGCCAAGCTCACTGTCCACGTGGTTGCCGACCCGAACGGTTCCTCCACTCCTGAGCCTGAGCCT
GAGCCGTTGGTCGGTTGGATTGAAGGCAAGGCGACCAAGACCACCATTTCGCCTGATTCCGAGGCGACCTGGTCA
CCGGCCGAAGGCAAGCTCAACGACGGCGTAGTCGTCGATGATACTTGGCCGACCACGGATGATCAGAACGTCAAC
GACAAGGTCTGGGGTTCTTGGGGCAAGGCAAAGGACGGCATGTACGCCCAGTACGACTTCGGTCAGTCCGTGACC
GTTGACCAGAGCCGCGCCCAGTTCTGGGCCAACTTCGCTGAGACTGACGATTCGAAGGGTGGTCTGGAAGTCCCG
GACGCTTGGAAGATTCAGTACCTCGCCGAGGATGGTTCTTGGAAGGATGTCGAGCCCACCGAGGATTACACCATT
GTGCGTAACTCGCCGGCTTCTCGTGCGGATACCGATGCCAAAGGTTGGAGCACTGTGACCTTCAAGCCGGTCGCC
ACCAAGTCGCTGCGACTCGTGCTCACTCCGCACACCGGCAGCAGCACCTTCGGGGCCGCCGTGGCCGAGTGGGGC
GTGCATGGTATTGACGGCACCGAGCCTGAACCTACCCCGGTCGACAAGACCGCGCTCGAGTCGGCTCTTGACACA
GCCAACGGCCTCGATGCAAGCCGCTACACCGCCGCTTCATGGGCTGAGTTCCAGCAAATCATTGACGCTGCCCAG
GCTGTGTACGACGATGCCAACGCCACCGCAGAACAGGTCGCCGAGCAGGTGACCAAGCTCGAGGACGGCCAGAAG
GCACTCGTTGCGCTCGCCACCGACGTGGAGAAGTCCACGTTGCAGGCGGCCATCGATGCGGCCAAAGCCGAGGCC
GCTTCCGGCAAGTACACGGATAAGAGTGTCGAGGCCTTGAACAAGGCCATCGAGGCTGCGGAAGGTGTGCTCAAG
GTCGGTGAGGTCGGTGAGGTCACTCAGGCCGCCGTCCAGGAAGCGTCCGCTTCGCTGAACAAGGCCGTCAAGGCC
TTGGAAGAGAAGCCCGCCGCCGAAACGGTGAAGAAGGAGTCCCTCGAGGCTTCCATCGAGCAGGCCAAGAAGGCT
GACAAGTCGAAGTACACCGAGGAGGCATGGCAGGCTCTGCAGAGCCAGATTGCCGCCGCTCAGAAGGTGTACGAC
GACAAGGATGCCAAGCAGGCCGATGTCGATGCCGCACAGGATGCCCTTGACAAGGCATTTTGGGCCACCAAGGTT
GAGCAGAAGCCCGGCTCCCAGCAGCCTGGTGTGACCGACACTGATAAGGATGATAAGGACAACAAGGGTGATCGT
GTGCCTCCGACTGGTGCCGCGGTTTCCGTAGTTGCTGCGGCTGCCGTGCTGCTCACCGCCGCAGGCGTGACCATC
CTGAAGCGTCGCCAGTCCGGCGACCACGGTTCGGCTCGCCACTCGGCCTGA

Suitably, the GH121 gene may encode a protein shown as SEQ ID NO: 18 or a sequence with at least 80% sequence identity to SEQ ID NO: 18. Suitably, the protein may comprise a sequence with at least 85%, at least 90%, at least 95%, at least 98% or at least 99% sequence identity to SEQ ID NO: 18.

SEQ ID NO: 18
MHQSTRKRWLASIGAVAAVATLATGGAVTAQAADAPVIKNADVAYPSFKGSDDPMKTAANNTTYNPAVSYLQETF
DNDVKNLAGIDTDHDFWIDKILTRTGAQPTGKGTNDKGAYSYEGSDGNNYLFTRGRAAYMYTHTPNQLGFVGDTA
YWDQTSRSGFTVTVNADGSNQTLNEDASQRKQTPSYFTSLFQTGGKSLKIKEVKYITYNNVMVANLTVESTQDRD
VTLTTASPFAAEGADGATELTGRVNVKNNLTTIYPRFSANNQDGSNWIVSGGKLTSTLSLKANEPQTVKIQLGLI
ANELPDSTKEYEARYTGDLKDAAASYKDSVTTYNKWWVDNAPYVDTPEDNIDKTVVYRWWLSRFNMLDANMPGNT
FQYPTSIEGVLGYNNQIVLTSGMFMMDPKWFRNPEYSYGTWLSAGDTAKKSKAGYYYYHDNPGDPANWNHSYTQY
ITRAGWDSYKVHGGPSTVAEELADQGAEDVQGLLASKSEPDNNDNQNNNDNSLIDWSWWSMTGNDADAVSFSEPG
RSGQRMDRADGSANMWANANAAAQAYKAAGDTANAEKMQAIADKIQKEVTTELWDKSDNLLKHKWLNDGAFAKYK
EINNYYPYSEGLMPTGNEDYNKALRLFEDSNEFPIFPFFTANQADKAALNFPGSNNFSIINAQPLLQVYSAGIRN
YDAAKNGYITNEQFKKLLYWVAFAHYQGGDNNYLDQNEFWNEDNNNVGDVNGDGVINNLDKNLDAAQNGGKITYR
SWIHHTQLGTTNWTMVEDVAGMVPREDNKIELNPIEIPGWNYFTVNNLSYHGQDVSIVWDKDGSHYGGPAGYSLY
VGGKLAFTSDKLAHLIYDPSTGTVEDADKAGVTITNAAGSDIKAANQVAFTADQRVTDLFAKSGANVDSASKSTT
NVAKDADVTGTTYAEKDTNYPAKNAVDGKTVMESFWGTKGSENKTDTLNIKFKDGKQKIDDLRLYFYQSSSSQTI
SGYAEPANYKLEYQKDDGTWAPIADQVRTPNYAGANYNRIQFTPVETTTIRVTFTPQAGMAVGVKEIEAYNTGIK
ADGTSENQAPQVDAYVSSSTSSGAKLVGTVKDDGLPAEGDVTTKWELVSGPEGGTAKFVDDTAASTTVTFNKEGD
YVLKLTASDGEKEGSKEITVHGIPSDGTVNVAPQSSASASYTNGYQPKDNAKKVIDGQVVYTNTPNETWNNWGDN

TGVEPWLQLKWAGKVPLKKAKVFFWTDGGGVPMASSWKLQYADADGNWQDVKLADGQSYTVNQNEGNEVKFADTV
ETDKLRVVFPKGAIVGASEFEAYAIEPVSVDEVNRLVQTGSKADDLKLPSTVSAVYTDGSRRDLAVTWDKVTDAQ
LAADAVFDVKGIVAGALSGTVAHIAARSDTALQTVGNAQPVEQTVYQNAKSIDLPATVPVKFPNGYNDDRKVTWK
DADIKAIDLTKVGDYEVAGTVDDGSSSAAAKLTVHVVADPNGSSTPEPEPEPLVGWIEGKATKTTISPDSEATWS
PAEGKLNDGVVVDDTWPTTDDQNVNDKVWGSWGKAKDGMYAQYDFGQSVTVDQSRAQFWANFAETDDSKGGLEVP
DAWKIQYLAEDGSWKDVEPTEDYTIVRNSPASRADTDAKGWSTVTFKPVATKSLRLVLTPHTGSSTFGAAVAEWG
VHGIDGTEPEPTPVDKTALESALDTANGLDASRYTAASWAEFQQIIDAAQAVYDDANATAEQVAEQVTKLEDGQK
ALVALATDVEKSTLQAAIDAAKAEAASGKYTDKSVEALNKAIEAAEGVLKVGEVGEVTQAAVQEASASLNKAVKA
LEEKPAAETVKKESLEASIEQAKKADKSKYTEEAWQALQSQIAAAQKVYDDKDAKQADVDAAQDALDKAFWATKV
EQKPGSQQPGVTDTDKDDKDNKGDRVPPTGAAVSVVAAAAVLLTAAGVTILKRRQSGDHGSARHSA

Suitably, the *B. Longum* transitional strain comprises a GH43_17 gene and one or more genes selected from a GH43_22, GH43_27, GH43_29 and GH121 gene as defined herein.

Suitably, the *B. Longum* transitional strain comprises a GH43_17, GH43_22, GH43_27, GH43_29 and GH121 gene as defined herein.

Suitably, one or more of the arabinan-degrading GHs described herein comprises a signal peptide. A 'signal peptide' may refer to a short amino acid sequence, typically present at the N-terminus of a polypeptide, which allows the polypeptide to be secreted out of abacterial cell. Without wishing to be bound by theory, this may advantageously allow the present *B. longum* transitional strain to act as a primary degrader of complex structures of arabinan when present in high molecular weight, usually in the diet. Suitably, a 'primary degrader' may refer to a bacterium that is capable of depolymerizing specific polysaccharides to mono-, di-, and oligosaccharides that they can take up and ferment themselves to acidic end products such as acetate or lactate. Suitably, the GH43_22, GH43_27, GH43_29, GH_121, GH43_24 and/or GH30_5 enzyme may comprise a signal peptide. Suitably, each of the GH43_22, GH43_27, GH43_29, GH_121, GH43_24 and GH30_5 enzymes may comprise a signal peptide.

Suitably, the present *B. longum* transitional strain comprises a glycosyl hydrolase family gene that encodes a CAZyme that targets arabinogalactans.

Suitably, the present *B. longum* transitional strain comprises a glycosyl hydrolase family 43_24 (GH43_24) gene. Suitably, the GH43_24 gene comprises SEQ ID NO: 19 or a sequence with at least 60% sequence identity to SEQ ID NO: 19. Suitably, the GH43_24 gene comprises a sequence with at least 60%, at least 70%, at least 80%, at least 90%, at least 95%, at least 98% or at least 99% sequence identity to SEQ ID NO: 19.

SEQ ID NO: 19
ATGAAGATAAACAATAAGGGCAAGGGCGCTCTTATCGCGGCAATTACCGCCGCGGCAACGCTATTGTCATGCGGG
CTGGCCGCTGCAAGTGCCAGTGCGGCAGGTGTGAATTACCTGCCTACCATCGGCCAAGTGCCGACATACACCAAG
TTCCAGCCCACAGCCGATCCGGGCAAGAACGCTAGCGATTACTTCCAGCCATATTGGTATGCCAAGAACGCCAAT

GATAATGGCGGCACACACATCCAAGCGCACGGTGGCCAAGTGGTCAAGGTTGGCGACGCCTACTACTGGTATGGC
GAAGACCGTTCTAACGGTTACGACAACAGCCCCGGTGTTCATGCTTATATGTCGACAGATCTATACAACTGGACC
GATCTTGGTGTGGCGCTGCGTGCGGTGACCAGCAAATCTCAGTTGACGGATAAGAGCAATGCCGATTACGCCTAC
TTCGACAAGGCCTACAACCTGACCAAGTCCGACGGCAGTGTGGACGCTGCCAAGGCCGACGCAATCTTCCCGTAC
CTCAACACCAACCCCGATCAGGATGGTGATGGCGCGGTTGATTCCGTACAGGGCATTTTCGAGCGTCCGAAGATC
ATCTACAACAAGAAGAACAAGCAATACGTGCTGTGGTGGCATTCCGATGGAAGCACCACGCCGGGCGGTTCCAAC
TATGCACGTGCACTTGCGGGCGTGGCTGTTTCCGACAATCCGGCGGCCCGTTCACTATGGTGGGTGCCTATCGT
TTGCCTAACCAGAACAATTGGAAAGAAGCCGCAGGTAACCCCAGCTGGGGTGAGAACGGTGACAGCCGCGATATG
ACTGTGTTCGTGGACCCGAAGGACGACAGTGCCTATGTACTGTATTCTTCCGAAGCCAATGCCACGCTGTACATC
GCCAAGCTCAACGATGATTACACCAATGTAGTCAAGACCACGAATGTGGACCAGTCCGAGGGACAAAAGCAGTAC
TCTGCTGACGGGCAGTACCCATACATTCTTGCAGACGCTACTACGGATGCCCCGGTGCGTGGCGAAGATTTCCAA
ATCGTCAAACAAAATGGTTCGCTGGAAGCTCCTGCCGTATTCCAATATGACGGGCGTTACAACATCATCGCATCT
GGTGCAACCGGCTGGGCCCCGAACAAGCAGACCTACTACACCGCCGACTCCATGCTGGGAAGCTGGACCCGTGGC
GTGGAAAAGGACGATATCAACGAGAACACGTGGTACAACAACATGCCGGAAGGCGCGGATGGTCTGTTGTCCGTG
GGCGATACCCGCGGCACCACATTCGGTTCGCAGTCGGCTAGTGTGCTCGCAGTAGACCAGGAGAAAGGTCACTTC
ATCTACCTTGGTGACCGTTGGGATTCCGGTAAAGCCGATTCCACCTATGTTTGGCTGCCGCTGACCATCGGTGAG
AACGGCACCATCGAAATGCACAATCCTGCTCAAGAAGGCGAGCCCGACGGTTGGGATCTGAGCTATTGGGGCAAC
CATGGTAGCGCCAAGGGCAAGCTGGTCAACTGGACTGTGGAAACCGGCGATGATCTCCCGAAGACCGTGAACACG
GGCGGAACCGTTACTCTGCCGGACACCGTCAACGTCAAGGAAGGCGACGATACCATTGCTACCAAGGTGACATGG
AATGTGGAAGGCGGTACGGCAGTCAGCAAGTCGACCAAGGCTGCTGGTAACACCTACGCATTCAATGTGCCGGGA
ACCTACACCATTACGGGCACTCTTGCCGAGAGCAGTAACTTCAATCCGGGCCGTACATTCCGTAGAACCATCGAT
GTTTCCTGCTCCAACCCAATTTCCGGAAGTTGGAAGGAAGCTCATTGGAAGGGCGGCAGCGCGTGCCAGGTTTCT
GCGTCCGGCGGTGCTTATGACTTCACGATTACGGACAACGCCAATCGGGGCGTCTGGACGGATCGCAACGAGGGC
AGTGCGGTGTACCAGCCTGATGCCCTGGACGTGAACGAAATGCTGGAAACCACGGTCAAGCCGCTCGACTTGGGC
GGTAATGGCGATCCGCGCGCCGGTCTGGTGGTCCGTAACGGTCTTACTGGCGCTAACGGCGGCAAGGATATGCC
ACGTTACTTGCCAGCCCAAGCGGCGTTTACATGCAGTACGATTCCAATGCCGATGGCTACATCGATAAGGAAACA
TCGCATGTTGGTACCGGCTTCGGCGACCAAGTGCAGCTCAAGCTGGAGCGCACCTCAACCGATACTCTGAAAGGC
TACTGGCGTGCTTCCGCGAACGATGAATGGCAGGATGTCGCTACGGTAACGCTGACCGGTGCGGACGTAACCGGG
CTCGATGCCGGTGCTTTCGCCACGTCGAACAGCAATGCCGGCGCATTCACCGTGGCCTTCAACGGCACTGCGTTC
GGTTCGCAGACTGCTGCTGTGGAGTCCATCGCGGCCAAGGGCCCTGAAGCCACTATCGCCAAGAGGCAGACGCTC
GCGCATAAGGACGTGACGGTTACCGCTACGCTCACCAATGGCAAGACGCGTGTACTGGAGCCAGATGAATACACG
TTGGAAGGCTTCGACACCACCAAATTGGGCGAGCAAACCGTGACGGTACGCCTTGTCACTGATTCTTCAGTAACT
GCCACGCTCACCGTGACTGTGGAAAGCAACCTTGCCCGGTTGTTCTGCTCGTCCGCCGCAGCCTCGAAGTATGAG
CCGGCCAGCAGCTGGGCCTCCGCTTCTACGGCCGACCTGACTTGCGACAACAATCTGAGCACCAACTGGTCGAAC
TGGGGCACCGGCGACACCTCGCCGTGGCTCAGCTACACCTTCGATAAGGCATATCAGCTGGGCAAGCTCAGCGTT
GCGGTGGATAAGGCCAAGGGCGAGGCCGCTCCGAAGAGCTTCACTGTATCGTACCTAGCTGAAGACAACGCCACG
TGGACTGATGCCACGCTGCCGGCAGTCACTGTGAATGGTGCTGCTGGAGCCGTGACGGAAGCCGATGTGAGCGCT
CTGCCCGCCACCAAGGGCATTCGCCTCAACTTCACCTACGCCGATGGCAATGACTATGCCAAGATCGCTGAAGTA
CGCATCGCCGAAGGTGAAGCAACGCCAAAGCCGCAGCCGTCTAGTAACGCCAATCTTGCTGATCTGACTGTGGAT
GGCAAGACGGTTGACGGATTCTCCGCGGATATCACCGAATATGCCGGTGCGCTGGCCGGAGACGCTGCTTCTTAC
CCGACGGTGGAGGCGACTGCTGCTGACGCGAAGGCTACGGTGCAGGTGGAGCAGGCTTCGACCGAGAACAGCGGC
GTGGCCACGGTGACTGTAACTGCTGAGGATGGCACGGCGGAAACCTACACAGTGACATTCGGCGAACTGCCTCAG
TTGGCCGAGCTTGCTGTGGAAGTGACCAAGGATTCCTATCAGGTAGGCGATAAGTTCAACGCTGCCGATGTGAAG
GTATCCGCCATTTACAAAGTCGGCGATACCGAAACGCTGCGCAAGCTGATTGATCCAACTGATGGTGATCTGAAG
TTCACTGGCTTTGATTCTGCCACCGCAGGCACGAAGACCATCACCGTCTCTTATCGTGGCGTGAACGCGACGTTC
GAAGTCACGGTCACGGCCACGGAGGTCACTCCCGGCCCTGGAGAGCAGAAGCCCGGCGATACCAACAATCCTGGC
AACACTGCTAAGCCCGGTAACACTGCCACGAATAAGCCGGCTGCTAATGGCGCTGCGCCCCTTTCGAATACGGGT
GTTGCCGTGGCTGCCATTGCGGTCGTGGTTGTGGTGCTGACAGCTGCGGCTGGTGCCTTGCTCGTCATCCGCAAA
CGCCGCGCATAA

Suitably, the GH43_24 gene may encode a protein shown as SEQ ID NO: 20 or a sequence with at least 80% sequence identity to SEQ ID NO: 20. Suitably, the protein may comprise a sequence with at least 85%, at least 90%, at least 95%, at least 98% or at least 99% sequence identity to SEQ ID NO: 20.

SEQ ID NO: 20

MKINNKGKGALIAAITAAATLLSCGLAAASASAAGVNYLPTIGQVPTYTKFQPTADPGKNASDYFQPYWYAKNAN
DNGGTHIQAHGGQVVKVGDAYYWYGEDRSNGYDNSPGVHAYMSTDLYNWTDLGVALRAVTSKSQLTDKSNADYAY
FDKAYNLTKSDGSVDAAKADAIFPYLNTNPDQDGDGAVDSVQGIFERPKIIYNKKNKQYVLWWHSDGSTTPGGSN
YARALAGVAVSDNPAGPFTMVGAYRLPNQNNWKEAAGNPSWGENGDSRDMTVFVDPKDDSAYVLYSSEANATLYI
AKLNDDYTNVVKTTNVDQSEGQKQYSADGQYPYILADATTDAPVRGEDFQIVKQNGSLEAPAVFQYDGRYNIIAS
GATGWAPNKQTYYTADSMLGSWTRGVEKDDINENTWYNNMPEGADGLLSVGDTRGTTFGSQSASVLAVDQEKGHF
IYLGDRWDSGKADSTYVWLPLTIGENGTIEMHNPAQEGEPDGWDLSYWGNHGSAKGKLVNWTVETGDDLPKTVNT
GGTVTLPDTVNVKEGDDTIATKVTWNVEGGTAVSKSTKAAGNTYAFNVPGTYTITGTLAESSNFNPGRTFRRTID
VSCSNPISGSWKEAHWKGGSACQVSASGGAYDFTITDNANRGVWTDRNEGSAVYQPDALDVNEMLETTVKPLDLG
GNGDPRAGLVVRNGLTGANGGKGYATLLASPSGVYMQYDSNADGYIDKETSHVGTGFGDQVQLKLERTSTDTLKG
YWRASANDEWQDVATVTLTGADVTGLDAGAFATSNSNAGAFTVAFNGTAFGSQTAAVESIAAKGPEATIAKRQTL
AHKDVTVTATLTNGKTRVLEPDEYTLEGFDTTKLGEQTVTVRLVTDSSVTATLTVTVESNLARLFCSSAAASKYE
PASSWASASTADLTCDNNLSTNWSNWGTGDTSPWLSYTFDKAYQLGKLSVAVDKAKGEAAPKSFTVSYLAEDNAT
WTDATLPAVTVNGAAGAVTEADVSALPATKGIRLNFTYADGNDYAKIAEVRIAEGEATPKPQPSSNANLADLTVD
GKTVDGFSADITEYAGALAGDAASYPTVEATAADAKATVQVEQASTENSGVATVTVTAEDGTAETYTVTFGELPQ
LAELAVEVTKDSYQVGDKFNAADVKVSAIYKVGDTETLRKLIDPTDGDLKFTGFDSATAGTKTITVSYRGVNATF
EVTVTATEVTPGPGEQKPGDTNNPGNTAKPGNTATNKPAANGAAPLSNTGVAVAAIAVVVVVLTAAAGALLVIRK
RRA

Suitably, the present *B. longum* transitional strain comprises a glycosyl hydrolase family 127 (GH127) gene. Suitably, the GH127 gene comprises SEQ ID NO: 21 or a sequence with at least 60% sequence identity to SEQ ID NO: 21. Suitably, the GH127 gene comprises a sequence with at least 60%, at least 70%, at least 80%, at least 90%, at least 95%, at least 98% or at least 99% sequence identity to SEQ ID NO: 21.

SEQ ID NO: 21
ATGAACGTTACAATCACTTCCCCGTTCTGGAAGCGGCGTCGCGACCAGATTGTCGAATCCGTCATCCCCTACCAG
TGGGGCGTGATGAACGACGAAATCGACACCACAGTGCCCGACGACCCGGCCGGTAACCAGCTGGCTGACAGCAAA
AGCCACGCGGTCGCCAATCTGAAGGTTGCCGCCGGCGAATTGGACGACGAATTCCACGGCATGGTGTTCCAGGAT
TCCGACGTCTACAAGTGGCTTGAGGAAGCCGCTTATGCGCTGGCCTACCATCCGGATCCCGAACTCAAGGCGCTG
TGCGATCGCACGGTCGATCTCATCGCCCGCGCTCAGCAGCCGGACGGCTACTTGGACACTCCGTACCAGATCAAG
TCCGGCGTATGGGCCGACCGCCCGCGCTTCAGCCTGATTCAGCAAAGCCACGAGATGTATGTGATGGGTCACTAC
ATCGAAGCCGCCGTCGCCTACCATCAGGTGACCGGCAACGAGCAGGCCCTTGAAGTCGCCAAGAAGATGGCCGAC
TGCCTGGATGCCAACTTCGGGCCCGAAGAAGGCAAGATTCATGGCGCCGACGGCCACCCGGAAATCGAACTCGCC
CTCGCCAAACTGTACGAGGAAACCGGCGAAAAGCGTTACCTGACGCTCTCCCAATACCTCATCGACGTGCGCGGC
CAAGACCCTCAGTTCTACACCAAGCAGCTGAAGGCCCTGAACGGCGACAACATCTTCCCCGACCTCGGCTTCTAC
AAGCCCACCTACTTCCAGGCCGCCGAACCTGTGCGCGACCAGCAGACCGCGGATGGCCACGCCGTGCGCGTCGGC
TACCTGTGCACTGGTGTGGCCCATGTGGCCGACTGCTCGGCGATCGGGGACTGATCGACACCGCCAAGCGTTTC
TGGACGAACATCGTCGCCCGTCGTATGTATGTCACCGGCGCGATTGGTTCCACCCACGTGGGCGAGTCGTTCACC
TACGACTATGATCTGCCGAACGACACGATGTACGGTGAGACCTGTGCTTCCGTGGCTATGAGCATGTTCGCCCAG
CAGATGCTCGACCTCGAGCCCAAGGGCGAATACGCCGACGTGCTGGAGAAGGAACTGTTCAACGGTTCCATTGCC
GGCATCTCGCTCGACGGCAAGCAGTACTACTACGTCAATGCACTGGAGACCACGCCTGACGGACTGGATAACCCG
GACCGTCACCACGTGCTCTCCCACCGCGTCGACTGGTTCGGCTGCGCCTGCTGCCCGGCCAACATCGCCCGACTC
ATCGCCTCCGTGGACCGCTACATCTACACCGAGCGCGACGGCGGCAAGACCGTGCTGAGCCACCAGTTCATCGCC
AACACAGCCGAATTCGCTTCCGGCCTGACGGTCGAGCAGCGTTCGAACTTCCCGTGGGATGGCCATGTGGAATAC
ACGGTGAGCCTGCCCGCCAGCGCCACTGACAGCTCGGTCCGTTTCGGACTGCGCATCCCCGGCTGGTCGCGGGGC
TCCTACACGCTGACCGTGAACGGCAAGCCCGCAGTGGGTTCGCTGGAAGACGGCTTCGTATACCTTGTGGTCAAC
GCCGGCGATACGTTGGAGATTGCGCTCGAGCTCGACATGTCCGTGAAGTTCGTGCGCGCCAACTCCCGCGTGCGC
TCCGATGCCGGTCAGGTGGCCGTGATGCGCGGACCGCTGGTCTACTGCGCCGAACAGGTCGATAATCCCGGTGAT
TTGTGGAACTATCGTCTGGCCGATGGCGTCACCGGTGCGGATGCCGCTGTGGCTTTCCAGGCCGACTTGCTGGGT
GGAGTCGATACCGTTGATTTGCCGGCAGTGCGCGAGCACGCCGACGAGGATGACGCGCCGCTGTACGTGGATGCC
GACGAACCGCGTGCGGGTGAGCCCGCGACGCTGCGCTTGGTGCCGTACTACTCGTGGGCCAACCGCGAGATAGGC
GAGATGCGTGTCTTCCAGCGTCGATAA

Suitably, the GH127 gene may encode a protein shown as SEQ ID NO: 22 or a sequence with at least 80% sequence identity to SEQ ID NO: 22. Suitably, the protein may comprise a sequence with at least 85%, at least 90%, at least 95%, at least 98% or at least 99% sequence identity to SEQ ID NO: 22.

SEQ ID NO: 22

MNVTITSPFWKRRRDQIVESVIPYQWGVMNDEIDTTVPDDPAGNQLADSKSHAVANLKVAAGELDDEFHGMVFQD
SDVYKWLEEAAYALAYHPDPELKALCDRTVDLIARAQQPDGYLDTPYQIKSGVWADRPRFSLIQQSHEMYVMGHY
IEAAVAYHQVTGNEQALEVAKKMADCLDANFGPEEGKIHGADGHPEIELALAKLYEETGEKRYLTLSQYLIDVRG
QDPQFYTKQLKALNGDNIFPDLGFYKPTYFQAAEPVRDQQTADGHAVRVGYLCTGVAHVGRLLGDRGLIDTAKRF
WTNIVARRMYVTGAIGSTHVGESFTYDYDLPNDTMYGETCASVAMSMFAQQMLDLEPKGEYADVLEKELFNGSIA
GISLDGKQYYYVNALETTPDGLDNPDRHHVLSHRVDWFGCACCPANIARLIASVDRYIYTERDGGKTVLSHQFIA
NTAEFASGLTVEQRSNFPWDGHVEYTVSLPASATDSSVRFGLRIPGWSRGSYTLTVNGKPAVGSLEDGFVYLVVN
AGDTLEIALELDMSVKFVRANSRVRSDAGQVAVMRGPLVYCAEQVDNPGDLWNYRLADGVTGADAAVAFQADLLG
GVDTVDLPAVREHADEDDAPLYVDADEPRAGEPATLRLVPYYSWANREIGEMRVFQRR

Suitably, the present *B. longum* transitional strain comprises a glycosyl hydrolase family 30_5 (GH30_5) gene. Suitably, the GH30_5 gene comprises SEQ ID NO: 23 or a sequence with at least 60% sequence identity to SEQ ID NO: 23. Suitably, the GH30_5 gene comprises a sequence with at least 60%, at least 70%, at least 80%, at least 90%, at least 95%, at least 98% or at least 99% sequence identity to SEQ ID NO: 23.

SEQ ID NO: 23
ATGAAGGTACTGAGCAAATCGCTTGCTGCAATGGTTGCGGCGGCAACACTAGTGGGAGGAGGGGCGTTTGCGGTT
GCCGGCACTGCGTATGCGGCTGATAACGATGCCATTACCGTGACCCCGAACCCGTGGTATGCCAACAGTTTCGAT
GGCTGGGGCACCTCGCTGGCTTGGTTCGCCAACGCCACCGGCAGCCTCGGCGAGGAATCGGCCATCACCACCAAT
CTCGGCGATGACGCTTCCAAGGCTAAGGCTGTGGAATACGGCAAACAGCTGCGCGAACAGTTCTACCAGTCCATC
TTCGGTGATGAAGGACTGGACCTGAACATGGCCCGCTACAACGTGGGCGGCGGCAATGCCTCCGATGTTGCCTAC
GGCTACCCATTCATGCGCCAAGGCGCTGCCGTGCCTGGCACGTGGAAAGATGACGCCACCGGCTCCGGCACGTAT
GGCAATGGCGTAACCACCAAGCAGGCCGACAAAGACAAGCTGGCTGCGGCATTCGACCCGACTGACGACAACCAG
TATGACTTCTCCAAGTCCGCCGCCCAAGACTGGTGGATTGAGCGCGGTGCCACCGGCGATAACCCTGACATCACC
GACGTAGAGGCCTTCGCCAACTCCGCTCCGTGGTTCCTGACCAACAGCGGTTACGCCACTGGTGGACGTAACTCC
GGTAGCAATAATCTTGCAAACCCTGAGAAATTCGCTCAGTACATGGCCAAGAACGTCGAGCACCTCGAAAGCCTT
GGCGCAAACGTTGACACGGTCGAGCCGTTCAACGAGTCCGAGACCAGTTACTGGGGCACTCCGGGCGACATGGCT
TCGAAGTACACCGATGAGAGCGATGACAACACCAAGCTCATTAACAACTACTGGGATAAGTACTACTCCGACAAA
GATAAGTCCGTCACCCCATACGCCAACGCGCTGAAGAAGCCGCAGGAGGGTATGCATGTCAGCAACGCCCAGCAG
CAGCAGACGATTACCGCACTCGCTGAGGCGCTCAAGGACAATGATGACACCATCATCGCAGCCACCGATGCCACG
AACTCCGCCGACTTCGTCAAGTCGTACAACCAGTACCCGCAGGCGATCAAGGACCTTATCGGCCAGTACAACGTT
CACGCCTACTCCGACAGCAACCAGATGCAGTCGCGCGATATCGCTCAGGCAGACGGCAAGAAGCTGTCGATGAGC
GAGGTGGACGGCTCCTGGCAGTCTGGCTCCTACAACCCGTACGGTTTCGACAACGCGCTGGGCATGATGAGCAAG
ATCAGCTCCAACGTCACCCGCCTGCAGTCCAAGGACTTCACCTTCTGGCAGGTGGTCGAGGACCTCTACAACATG
CAGATGGGCTCGAATGTGAATCCGGCCGGTGAGAACACCAACTGGGGCACCGTGCTCATCGACTTCGACTGCACC
GTGGCTGGCATGGACGGCAAGCTCTACTCCGAGCGCCGCGTGAACAACAACGGCGGTACCACCGATGGACTTGAA
CCGTGCACGGTTATTGCAAACGCCAAGTACAACGGCGTCAAGGCCATCACCCACTTCATCCACGCGGGCGACAAG
GTCATCGCCAACAACGATGAAGACAACAACATGACTGCCACCTCCGACGATGGCAAGACACAGACCGTCATCCAC
CGCAACTCCGGCACCTCTGACCAGACCTTCGTCATCGACCTGTCGAAGTACGGCGAGATTGCCGACAACGCTTAC
GGTGAGCTCTACCTGACCACCGAAACCTCTGCCGAAGACAAGAACGCGGGTGTCGATTCCGCCACTCCGGAAGTC
TTCGCCAAGACCAGCAACGTCAAGCAAGCTGAAGGCTCTGTGATGATTGACAAGGCTGCCAAGACCGCTACGGTC
ACTGTGCCCGCCCGTTCTATCGCCTCCATCCAGCTCACTGGCGTGACCGGCTACGCCAAGGATGCTGCCGTCGAG
ACCGGCGACACTTACCAGCTCGTTGGTAAGCAGTCCGGCAAGGCCGTGGCTGATACCACTTCTGGTGATTCCGCG
CTGTCCCTGGCCAACGTCGCTTCCGATGCCGAGAACGCCAAGAAGCAGACTTGGACCTTTACCCAGATCGAGCAG
CCCGCCGACTCCGAGCGCCCTGATCTCAAGGTTTATGTGATTACTAACGCCGAAGGCAAGGTGCTGGTGTCCAAG
GATGGCACGAACGCGCTTTCCAACGAAACGGTTGAGGCCGCTAAGTCCGACCCGGCTGCCAAGTGGATTCTCAAC

ACTTCCGATGGTTCGACCTACCAGCTGCTCAATGCCGCGACTAAGACGAACCTCGATGTGGATAACTCTGGTACC
ACAGTCGGCACGAAGGTTGGCTTGTGGCAGTCACCGAGCGGCACTTCGCCGTCCGCCAACCAGACATGGACTCTA
CGCAATGTAACGCCGACCAGCCAGAAGACCGTGAACGTGCAGACCGCCGTTAACGAGAAGGCCGCGCTGCCGACC
GAAGTCACGCTCTACTACACCTGGGGCGAAGGCAAGGCCACGGTTGCCAACTGGGATACTTCCAAGGTCGATGTG
GCCAAGGAAGGCACCTACGAAGCCACCGCTACCGCCACCGATGTGTACGGCAACGAGTTCAATGTCGCCGCTACG
GTCTACGTTGGCGCGCTCACCGTTTCCGATCCGGTATCGGCTACAGTGCTGGCCGGCACCAGTGCGAGCGAGGCG
AAGGCCGCGCTTGAGGCTGCGCCGGTGTATCTGCACGTCAAGGCATCGCCTGCATTCGAGGGCGATGCGGCTAAG
GTTACGTGGAACTTCGATGGGCTTGATACCAAGCTCGCCGATGCCAAGGCTGGCGACAACATTGCCGTGACCGGT
ACTTACCAGCTGGACGACGCGACCACGATTGCGCTGAAGGGCGCGATCTATGTCACCGCCGCCACGCCTGAGAAT
GTGGCCGACACTGCTTCCAGCCTGACCGTGACCAACCAGCAGACGGAATACAGCAAGGGCGATCAGTGGAAGAAG
CTCACCGATGGTGACACGTCAGCTGAAGCCTGGGTGACGTGGAACTCTGCTGGTGACTATTCCGCCAGCCCGACC
GCCACGATTGACTTCGGCTCTGAGTGTGAGCTTAGCAGCGTGACCATTACGTATGGTGACAAGGCTCCGGCTTCC
GCCAAGGCCGAGTACACCACTGATGGCGAGACGTGGATGCAATTCGGTAGCGATGTTAAGCCTGCCGCAGGCCAG
ACGGTGACGTTCAAGGCCGATAAGGGCACAGTGAATGCCACGAAGGTGCGCATTGTGAACACCGTGAACAACGAC
TACATGAACGCCACCGAAATTCAGGCATTCGTGACGCCGGTTCAGGGTGCTGCGAAGAACATCGCCGCGGCCTCT
GGCACGAACTTCTCGGTGAACTTCCAGGAGGGTGCCTCCGCTTCCAAGGCCATCGATGGTGACACTACGTCAAAG
GGTTGGTCCACTTGGGCTTCCACCGCCTCGACGGTGGACCCGGTCGCCACGTTCACCTTCGACGAAGCTCAGACC
ATCACCGAAGTGAAGACCTTCTTCTACTACGATGGTCGTGCGTCTTGGCCGAAGAGCCAGACGCTGGAATACCAG
GATGAGGCTGGCGAATGGCATGGAGTCGGTACCAAGGATGGCTGGAAGATACAGGCCGGCGATGCCGGCTCTGGC
TCCGACGGCATCACCGCCGCCGACACCCCGACCGTTGACTTCGTGCTCGGCACCCCGGTAAAGGCCAAGGCCATC
CGCCTGACTAACACATTGCAGGACACCAAGGTGTACATCAACGTGGCTGAGATCCAGGTGTTCGCACAAGACAGC
ACGGTACTCACCCCGCAGCCAGCATCCGATGCCACGCTGGGCGACCTGCGTCTTGACGGCGAAACCGTTGAAGGC
TTCGACCCGGCCAAGACCGACTACACGGTTGATCTGCCGGTCGACGCCGAGGCAAACCCGGTGCTGCAGGCCTTC
GCCACCGACAATGCCGCCGCCGTCAAGGTGACTGGCGACGCGGTTGAGAACGGCCAGCTTGGCGGCAAGGCCGCC
ATTACGGTGACCTCAGCCGACGAGTCTGAGACGAAGACCTACACGGTGACCTTCAACGCCTTCACTTTGGCTTCG
CTCAAGGTGATCGGACCCACGAAGACCGAGTACGCCATCGGCGACAAGCTCGATACCGCCGGTCTGAAGGTGACT
GCCGTCTACCAGAGTGGCGACAAGACCAAGGAAGTGCCGGTCGCTCTTGACGACCCGCAGCTTGCGATTGGCTCG
TTCGACTCCACCACCGCAGGCAAGAAGGCGATTACCGTCTCCTACCGTGGTGTGACCGCGACCTTCAACGTCACG
GTCAAGGCCAACGCAGTCGCCCCTGGCCCTGAAGAACAGAAGCCCGGCAACACCAACAAGCCCGGTGCCACCGGC
AGCGGCAACAAGAACACGGTGGCCAACACCGGTTCCAGTGTTGCCGCCATCGCTGGCGCTGTCGCTCTGCTGGCC
GCTGCCGCGGGTGCACTGTTCATGCTGCGCAAGCGTGCATAG

Suitably, the GH30_5 gene may encode a protein shown as SEQ ID NO: 24 or a sequence with at least 80% sequence identity to SEQ ID NO: 24. Suitably, the protein may comprise a sequence with at least 85%, at least 90%, at least 95%, at least 98% or at least 99% sequence identity to SEQ ID NO: 24.

SEQ ID NO: 24

MKVLSKSLAAMVAAATLVGGGAFAVAGTAYAADNDAITVTPNPWYANSFDGWGTSLAWFANATGSLGEESAITTN
LGDDASKAKAVEYGKQLREQFYQSIFGDEGLDLNMARYNVGGGNASDVAYGYPFMRQGAAVPGTWKDDATGSGTY
GNGVTTKQADKDKLAAAFDPTDDNQYDFSKSAAQDWWIERGATGDNPDITDVEAFANSAPWFLTNSGYATGGRNS
GSNNLANPEKFAQYMAKNVEHLESLGANVDTVEPFNESETSYWGTPGDMASKYTDESDDNTKLINNYWDKYYSDK
DKSVTPYANALKKPQEGMHVSNAQQQQTITALAEALKDNDDTIIAATDATNSADFVKSYNQYPQAIKDLIGQYNV
HAYSDSNQMQSRDIAQADGKKLSMSEVDGSWQSGSYNPYGFDNALGMMSKISSNVTRLQSKDFTFWQVVEDLYNM
QMGSNVNPAGENTNWGTVLIDFDCTVAGMDGKLYSERRVNNNGGTTDGLEPCTVIANAKYNGVKAITHFIHAGDK
VIANNDEDNNMTATSDDGKTQTVIHRNSGTSDQTFVIDLSKYGEIADNAYGELYLTTETSAEDKNAGVDSATPEV
FAKTSNVKQAEGSVMIDKAAKTATVTVPARSIASIQLTGVTGYAKDAAVETGDTYQLVGKQSGKAVADTTSGDSA
LSLANVASDAENAKKQTWTFTQIEQPADSERPDLKVYVITNAEGKVLVSKDGTNALSNETVEAAKSDPAAKWILN
TSDGSTYQLLNAATKTNLDVDNSGTTVGTKVGLWQSPSGTSPSANQTWTLRNVTPTSQKTVNVQTAVNEKAALPT
EVTLYYTWGEGKATVANWDTSKVDVAKEGTYEATATATDVYGNEFNVAATVYVGALTVSDPVSATVLAGTSASEA
KAALEAAPVYLHVKASPAFEGDAAKVTWNFDGLDTKLADAKAGDNIAVTGTYQLDDATTIALKGAIYVTAATPEN
VADTASSLTVTNQQTEYSKGDQWKKLTDGDTSAEAWVTWNSAGDYSASPTATIDFGSECELSSVTITYGDKAPAS
AKAEYTTDGETWMQFGSDVKPAAGQTVTFKADKGTVNATKVRIVNTVNNDYMNATEIQAFVTPVQGAAKNIAAAS
GTNFSVNFQEGASASKAIDGDTTSKGWSTWASTASTVDPVATFTFDEAQTITEVKTFFYYDGRASWPKSQTLEYQ
DEAGEWHGVGTKDGWKIQAGDAGSGSDGITAADTPTVDFVLGTPVKAKAIRLTNTLQDTKVYINVAEIQVFAQDS
TVLTPQPASDATLGDLRLDGETVEGFDPAKTDYTVDLPVDAEANPVLQAFATDNAAAVKVTGDAVENGQLGGKAA
ITVTSADESETKTYTVTFNAFTLASLKVIGPTKTEYAIGDKLDTAGLKVTAVYQSGDKTKEVPVALDDPQLAIGS

FDSTTAGKKAITVSYRGVTATFNVTVKANAVAPGPEEQKPGNTNKPGATGSGNKNTVANTGSSVAAIAGAVALLA
AAAGALFMLRKRA

Suitably, the present *B. longum* transitional strain comprises a glycosyl hydrolase family 43_32 (GH42_32) gene. Suitably, the GH42_32 gene comprises SEQ ID NO: 25 or a sequence with at least 60% sequence identity to SEQ ID NO: 25. Suitably, the GH42_32 gene comprises a sequence with at least 60%, at least 70%, at least 80%, at least 90%, at least 95%, at least 98% or at least 99% sequence identity to SEQ ID NO: 25.

SEQ ID NO: 25
ATGACCGCAACCATCAGCAACGGTGTATCCGCCAGCTACAGCCCTGCGGAAGACGAGCTCGGCGCAGCTGACCCC
ACCGCCTTGCTTGCCGAATCTGGCGATTTGAAGCCGCTGGCCGAACGCACTTATACGAATCCGGTTCCATATGCG
GACGGTAAGTCCCATACCGCGCCCGACCCGTTCGTGCTCAAATACCGCGACCTCTACTACTGCTATGCCACCGAC
GAGCACGGCATTCTGGTCTCCACCTCACCGGACATGGTGCACTGGACCTCACATGGATTCTGCTACACCGAAGCC
GGACGCAGAAACTTCTGGGCCCCATCGGTGATTCTCATCAACGGCGTCTTTCACATGTACTTCTCGAATATGCCG
GCCGAGGAGACCGACACCCACACGGAAATCATGCGTGTGGCCGTGAGCGAGGATCCGCTCGGCCCGTTCGAAAAG
AAAGCGGAGCTGTTCAACACCTTCGCCATCGACTCCCAAGTGGTCTATGGCGATGACGGCCAGTTGTACTTGCTT
TACGCCGACAATCAGGTCACCGGCCTGAGCGATGACCGGCCCGGAACCTCCGTGATGATCGATCGCCTTGTGACC
CCGTATTCGCGTGAGAACAAACCGCGCCCGCTCATCGTGCCCACCATGGACGAGGAGATCTTTGCCCGCAACCGT
TTCGGCGATGGCCGCGACTGGCACACCGTAGAAGGCGCCACATACTTCGCCTACCGTGACCGCGCGTTCATCACC
TACTCGGCCAACGCCTACGAGCATGAGGACTACTTCGTCGGATACTCGTACGCACAGCTGCCGAATAAGCAGGCC
GACGCCCACATCGATCAGCTCGATTGGACGAAACAGCTCAACGAGAACCGCTTCGATCCGCTGCTTATCCGCAGC
CCAAAGGTTGAAGGCACGGGCCACAACTCCATAGTCAAAGCGCCCAATGCCGTTGATGACTGGATTGTCTACCAC
GGCCGTAACGCCGATGACGAGCTGTATGTGGGCACCGAACAGCGCGTAATGCGCATCGACCCGCTGTACTACGCC
GAAGGAGGGCTCGACACCCCAGGACCTACCGCCGCCGCTCAAAGCGCACCGCTGTATGGCACTGTGCATGATGAT
TTTGCGGATGGCCTGAACGCCGGATGGTCGGTTATTTCCGGTGCGGCCCACACCGAATCCGATGTGGACGGTCAC
GCGCTTGTTGCCGACGAATCCAGTGTATTCATCGCTGTGTCGGGCAAATCGTCCGCAACCCAAGTGATTGACGTC
TGGGCCAAAGCTCCCGTCACCCCACTGGGCGCACGATTCGGTATCGTGGTGCGGTACCAGGATGCCAACAACCTC
ACCAAACTCGAGGTGGATGCTGGCCGTCAGGTAATTAGCGTGGTCGATGTGATCGGCGGCGTTGCCTCCGAACGC
GTGACCAATGCCGACCTCCATGACTTCGATTCCCATGCCTGGCATGAGTACCGGCTTGAGCGCCGCTACTGCAGG
CTGGAGATCCGCATTGATGGCCGTTTCGCCGCGTCCTGCACCATCAGTGATAAGCCCGGTCGGGCGGGATTGTTC
TCGTTGCGAACGGGGGCCGCGTTCAGCGCATATGCGGCCACTGAACATGTGAATCTGTGGGGTGCCGGATTGCGG
GATCTCGGTCGAGAATTGCATGCTGACCGCCGACTCGTCATCGACGGCGGCGTGAGGTCCAGCGGCGTGTGTCCG
GTAACACTCGAACTGGCATACCCGCTGGTCAGCAACCGTTTCGTCCTTGATTTCGCTGGGCAGACGAGCCGTGGG
CAGGCGCTGTTGTCTCTTGGCGAATACCGTTTGTCCGGCACGGCATCATCCGTGGAGTTCATGCGCAACGGCAAG
TCTCTGCCTTCCACCCCGGAGCCGGCCAGGCTGCGTGTCTTTGAAGACAACGTCCGCCGTGACCGTTCGGGCCGA
GCCGTGCTCACCATCCGTATCGAAGCTCTGAACGGCACGATGCGACTGCACCTACGTGGCAAAACCTGGCAGGTG
CCGTTTGCGGACAATGCGGCCCGTGCCCGTATCACTCTTGATCGCGCATCCCTGACCGGATACGAGAGGACATCG
CTGGAATCCAGCATCGAGGAAAGGAGTGCGTCCGGCAATTGA

Suitably, the GH42_32 gene may encode a protein shown as SEQ ID NO: 26 or a sequence with at least 80% sequence identity to SEQ ID NO: 26. Suitably, the protein may comprise a sequence with at least 85%, at least 90%, at least 95%, at least 98% or at least 99% sequence identity to SEQ ID NO: 26.

SEQ ID NO: 26

MTATISNGVSASYSPAEDELGAADPTALLAESGDLKPLAERTYTNPVPYADGKSHTAPDPFVLKYRDLYYCYATD
EHGILVSTSPDMVHWTSHGFCYTEAGRRNFWAPSVILINGVFHMYFSNMPAEETDTHTEIMRVAVSEDPLGPFEK
KAELFNTFAIDSQVVYGDDGQLYLLYADNQVTGLSDDRPGTSVMIDRLVTPYSRENKPRPLIVPTMDEEIFARNR
FGDGRDWHTVEGATYFAYRDRAFITYSANAYEHEDYFVGYSYAQLPNKQADAHIDQLDWTKQLNENRFDPLLIRS
PKVEGTGHNSIVKAPNAVDDWIVYHGRNADDELYVGTEQRVMRIDPLYYAEGGLDTPGPTAAAQSAPLYGTVHDD
FADGLNAGWSVISGAAHTESDVDGHALVADESSVFIAVSGKSSATQVIDVWAKAPVTPLGARFGIVVRYQDANNL
TKLEVDAGRQVISVVDVIGGVASERVTNADLHDFDSHAWHEYRLERRYCRLEIRIDGRFAASCTISDKPGRAGLF

SLRTGAAFSAYAATEHVNLWGAGLRDLGRELHADRRLVIDGGVRSSGVCPVTLELAYPLVSNRFVLDFAGQTSRG
QALLSLGEYRLSGTASSVEFMRNGKSLPSTPEPARLRVFEDNVRRDRSGRAVLTIRIEALNGTMRLHLRGKTWQV
PFADNAARARITLDRASLTGYERTSLESSIEERSASGN

Suitably, the *B. longum* transitional strain comprises one or more genes selected from a GH43_24, GH127, GH30_5, and GH 43_32 gene as defined herein.

Suitably, the *B. longum* transitional strain comprises a GH43_17 gene and one or more selected from a GH43_24, GH127, GH30_5, and GH 43_32 gene as defined herein.

Suitably, the *B. longum* transitional strain comprises a GH43_17, GH43_24, GH127, GH30_5, and GH 43_32 gene as defined herein.

Suitably, the *B. longum* transitional strain comprises a GH43_17, GH43_22, GH43_27, GH43_29, GH121, GH43_24, GH127, GH30_5, and GH 43_32 gene as defined herein.

Suitably, the *B. longum* transitional strain comprises a GH43_17, GH43_22, GH43_27, GH43_29, GH121, GH43_24, GH127, GH30_5, GH 43_32, as defined herein.

### GH43_17 gene cluster

Suitably, the *B. longum* transitional strain may comprise one or more genes encoding for a family 31 glucosidase (GH31), an ABC transporter, a Lac-I type regulator, a MFS transporter and/or an AraC family transcriptional regulator.

Suitably, the present *B. longum* transitional strain comprises a glycosyl hydrolase family 31 (GH31) gene. Suitably, the GH31 gene comprises SEQ ID NO: 27 or a sequence with at least 60% sequence identity to SEQ ID NO: 27. Suitably, the GH31 gene comprises a sequence with at least 60%, at least 70%, at least 80%, at least 90%, at least 95%, at least 98% or at least 99% sequence identity to SEQ ID NO: 27.

SEQ ID NO: 27
ATGACAACTTCATTCACCATCGACGGCAACGCCCTGATCTGGACCGGGGACGGCGAAACCCTGCGCATCGAACCT
TGGGAAGAGAACAGCGTACGTGTACGCGCCACCCGCAACCGTGGCTTCGGCCCGGTCGATTGGGCGCTTCTGGAA
CCGAAGAATGAATCCGGCCGTGTCGCAGACATCGCCGTCGGCGAGGACGGCGAACACGCCAGCCTGACCAACGGC
AGCATCACCGTTAAAGCGGATTCGAATCATGCTCCATTGCTGTCTGCCGGATATGAAACCTTCCGGTGTGACCTG
AGCTTCTGGAACGCCGAAGGCGAACTCCTGTTCCGCGAATATCCACAAGGTGGGTCGCTTTTGCTCAAGGCGCGT
GACTACACTCCGGTGTCCGGTGAAAGCTTCGCCGTGACCACGTCTTTCAGCGCCGATCCCAAAGAACGGCTGTAT
GGCATGGGCGAATACCAACAGGACGTGCTTGACCTCAAAGGCTCCACCTTTGAACTTGCGCACCGTAATTCCCAA
GCCTCCGTGCCGTTCGTCGTCTCCTCCAAGGGGTACGGCTTCCTGTGGCACAATCCGGCTATTGGCCGCGCCACT
TTTGGACGCAACCGAACCGAATGGCGGCTCAGTCCACTGACCAGATTGACTACTGGGTCACCGCCGGTGACTCC
TACGCGCAGATCGAATCGCAATATGCCGACGCCACCGGACATGCGCCAGTCATGCCTGAATGGGGTATGGGCTTC
TGGCAGTGCAAGCTGCGTTACTGGAACCAGGAACAATTGCTTGACGTGGCCCGAGGCTTCAAATCCCGGAACATC
CCGCTAGACCTCATCGTCATTGACTTCTTTCACTGGCCTCATTTGGGCGACTATAAGTTCGAGGACGAATTCTGG
CCTGATCCCGAGGCCATGGTCGCCGAGCTCAACAGCATGGGCGTCAAGCTCATGGTGTCTGTGTGGCCGCAGGTC
TCGGTCTCATCCGAGAACTTCGTGGAGATGAAGCGCAACAACTATCTGGTAAGCGCTGAAGCTGGGCTCAATCTT
GACATGATGTTCGAAGAGCCGTGCGTCAACTATGATCCCACCAACCCGGGAGCTCGCAAATTTGTGTGGGACAAG
TGCAAGGCCAACTATTGGGACAAGGGCGTGCGCGCCTTCTGGCTGGATGAGGCCGAACCCGAATATGGTGTCTAC

GATTTTCGCAACTACCGCTACCACATGGCAGCGACCTCAACGTGGGTAACGTCTATCCGCAGGCTTACAACCGC
GGATTCTACGAGGGGCAGATAGAAGCCGGCATGGAAGGCGAGATCGTTAACCTGACTCGATGTGCGTGGGCTGGA
TCTCAACGTTACGGATCGTTGGTCTGGTCTGGAGACGTTGGCTCCACATTCGCCGATCTGAAATCGCAGATTACC
TGTGCTATTCACATGGGTATGGCTGGCATCCCTTGGTTCACTACAGACATGGGCGGCTTCCATGATGGGGTGATC
GATTCGGATTCATTCAAGGAGCTGCTGGCCCGCTGGTGCGCGTTCTCCTGCTTCCTGCCCGTCATGCGCAACCAT
GGTGACCGCAGCCTGGGGGAGTCGACCGGCAAGCAAACCATCACCAAGGCAACCGGTGAGCACCGTTCGCCTTCG
GGCGCGGACAACGAGCCATGGAGCTATGGCCCTGAAATGGAGTCCATATTCCGTAAATACATCGCCGTGCGCGAG
GTCATGCGCCCGTATACCCGTGAACTGTTCCAGTCTGCCCATGAGCAGGGTCAGCCGTTGGTGCGAGGACTGTTC
TACGAGTTTCCGACCGATGAACACGTGGCCGACATTGCGGACGAATACCTGTACGGTCCTGACATTCTTGTGGCT
CCCGTAGTCGAGGCCGGTGCTGCTTCCCGTAGCGTCTACCTTCCTGGCGATGAGACGACCACTTGGACTGATTTG
CGAGACGGTGCCGTATACGCGGGTGGGCAGAGCATCGAGTCGTCTGCAGCAATCGACACGGTCCCTGCCTTTGCG
CGAGATGGTCGGGACCATGGTTTGATTGGTCTGTTGTAG

Suitably, the GH31 gene may encode a protein shown as SEQ ID NO: 28 or a sequence with at least 80% sequence identity to SEQ ID NO: 28. Suitably, the protein may comprise a sequence with at least 85%, at least 90%, at least 95%, at least 98% or at least 99% sequence identity to SEQ ID NO: 28.

SEQ ID NO: 28

MTTSFTIDGNALIWTGDGETLRIEPWEENSVRVRATRNRGFGPVDWALLEPKNESGRVADIAVGEDGEHASLTNG
SITVKADSNHAPLLSAGYETFRCDLSFWNAEGELLFREYPQGGSLLLKARDYTPVSGESFAVTTSFSADPKERLY
GMGEYQQDVLDLKGSTFELAHRNSQASVPFVVSSKGYGFLWHNPAIGRATFGRNRTEWAAQSTDQIDYWVTAGDS
YAQIESQYADATGHAPVMPEWGMGFWQCKLRYWNQEQLLDVARGFKSRNIPLDLIVIDFFHWPHLGDYKFEDEFW
PDPEAMVAELNSMGVKLMVSVWPQVSVSSENFVEMKRNNYLVSAEAGLNLDMMFEEPCVNYDPTNPGARKFVWDK
CKANYWDKGVRAFWLDEAEPEYGVYDFRNYRYHMGSDLNVGNVYPQAYNRGFYEGQIEAGMEGEIVNLTRCAWAG
SQRYGSLVWSGDVGSTFADLKSQITCAIHMGMAGIPWFTTDMGGFHDGVIDSDSFKELLARWCAFSCFLPVMRNH
GDRSLGESTGKQTITKATGEHRSPSGADNEPWSYGPEMESIFRKYIAVREVMRPYTRELFQSAHEQGQPLVRGLF
YEFPTDEHVADIADEYLYGPDILVAPVVEAGAASRSVYLPGDETTTWTDLRDGAVYAGGQSIESSAAIDTVPAFA
RDGRDHGLIGLL

Suitably, the present *B. longum* transitional strain comprises one or more ABC transporter genes. Suitably, the ABC transporter genes comprise SEQ ID NO: 29-31 or sequences with at least 60% sequence identity to SEQ ID NO: 29-31. Suitably, the ABC transporter gene comprises a sequence with at least 60%, at least 70%, at least 80%, at least 90%, at least 95%, at least 98% or at least 99% sequence identity to SEQ ID NO: 29-31. Suitably, the present *B. longum* transitional strain comprises a gene with at least 60% sequence identity to SEQ ID NO: 29, a gene with at least 60% sequence identity to SEQ ID NO: 30 and a gene with at least 60% sequence identity to SEQ ID NO: 31.
SEQ ID NO: 29 
ATGACGCATCGTAGCACCTGGTGGAAAACCGCTCTCGGCATCATATTGACGCTCATCATGATGTTTCCTGTCTAC
TGGATGATCAACATCTCGTTCACTGGTAAGGCATCCATTCGTTCCGGCGACCTGTGGCCCAAGGATTTCACCTTT
GACAACTACGCCCGCGTAATCGCCGACCAAATGCCCTATCTGGGCACTTCCATCCTCGTAGCGGTATGCTGCGTG
ATTCTAACGCTGGTCATCGCACTGCCTGCCGCCTACGCACTGGCTTTGCTGCGCTGTCCAGGCAGCGGCGCGCTC
AGCTTCCTGCTCATCGTGGCTCAGATGATTCCCGCCGTCGTGATGTCGCTCGGCTTCTACGAGATTTATAACAAC
ATTGGTCTGCTCGATACGTTGCCCGGCCTGATCCTCGCCGACTCGACCATTGCGGTGCCGTTCGCGGTCATGCTC
CTGACTTCTTTCATGGCCGGCATCCCGCGGTCCCTGCTTGAGGCCGCCGAAGTGGATGGAGCCTCACGTACCCGT
CGCTTCTTTTCCATTGTCATCCCGTTATCGCGCAATTCGATCGTGACCGTCTCCCTGTTCGCTTTCCTATGGTCT
TGGAGCGACTTCCTGTTCGCTTCCACCCTTGACTCCGGCGGCGGCAAGATGCGCCCGATCACTATGGGTCTGTAC
AACTATATCGGTGCGCAGACCCAGGAATGGGGGCCGATGATGGCCACCGCAGTGCTTGCATCCATTCCCGCGACC
ATCCTGCTTGTCTTCGCCCAGAAGTACGTCGCCGCAGGCGTGACCGCCGGTGCTGTTAAGGACTAA
SEQ ID NO: 30 
ATGACAGCCTCAACAACAAGCCCCGTTCGCCGGGCAAAGTCCGGCACTCCGGTCCGGGCCAAACTGGCCATCGCC
GGATTCATTGCCCCACTGATTATCTACTTGGTAATCTTTTACGCGTTCCCGCTCATCCAGAACGTGTCAATGAGC
CTGCACCGATACACGCGACGAACCTTCGTTACCGGAGATGCGCTGTTCGTGGGTCTCGACATCTACAAGGAAGTC
ATTTCCTCCGTGGAGTTCTGGCCGGTTGTGGGGCAGACCTTCGTGTTCGTGGTCGTCTCGCTGATATTCCAATAT
GTAATCGGCTTGGCCCTGGCGGTGTTCTTCAACGATAACTTCAAGCTCTCTGGTGTGCTGCGCGGCATCATGCTG
GTTCCGTGGCTGTTGCCGCTGATTGTTTCTGGAACCGTCTGGCAGTGGATGATGGACCCTGACTCCGGCATCCTC
AACATGTTCCTCGGTCTGTTTGACATCGAACCCATCTGGTGGCTCCAGGCGGATAACTCGCTGTGGGCCGTCATC
ATCGCCAACATCTGGCTGGGAATCCCCTTCAACCTCGTGATCCTGTATTCCGGCCTACAGAACATCAGCGGCGAC
CTGTATGAAGCCGCCTCCCTCGATGGCTGCAACGCCTGGCAGCGCTTCTGGAAGATCACCTTCCCTCTCCTGAAG
CCCGTCACTTCGATCACCCTGTTGCTCGGCTTCGTCTATACATTGAAGGTCGTTGACGTGATCTGGATGATGTCC
CAGGGAACCGGCACCTCGCGTACCCTCGCCACCTGGGCCTATTCGATGGCATTTGGCAAGGGAACTTCAATGACT
ATCAAATACTCGGAGGCTTCGGTGCTCGGCACGATTCTCATCATCGTGGCGTTGATTTTCGGACTGATTTACCTG
CGGGTCCAGAAGACCCAGGAAACCTGCTAA
SEQ ID NO: 31

ATGAAGTCCAATACCGCTCTTAAGATAACCGCCGCATTATGCTCCTGCGCCATGCTTGTCGGCGTCAGCGCCTGT
GGTTCGAGCAACAGCACCACGGATGATAAGGTGATCGAATGGTGGATGACTGGACCCGCCACGAGGATGGCTCC
GAGTTCGACAAACTGGTCAAGGCGTGTGCGCCCGAAGGCTACACAATTGAGCGCCAAGCCATCGCCACTTCCGAC
CTGCTCAACAACCTCACCACCGCAATCAAGGAAGACAATGGCCCGGATGTTGCGGTCATCGACAACCCGATGATT
CCGTCCGCCGTCGATGCGGGTTTGGTTGCTGGTTCCGACGAAACTGGTCTTGACGTTTCTGCCTGGGATGAGAAC
CTTGAGGCTCCGGGCGTAGTGGACGGCCAGGCATATGGCGTGCCGCTGGGCGGATCCAACACGTTGGGTCTTATG
TACAACCCCACCATCATTGAGGCAGCCGGTGTGGATGTATCCACCATCACCGATTGGGATTCGCTCAACGCGGCC
ATCAAGAAGGTCGTTGACGCCGGATACAAGGGCATTACGTTCTCGGGCATCTCGGGTGAGGAAGGCGTCTTCCAG
TTCCTGCCTTGGTTCTGGGGCGCAGGTGGTGATCTGTCCAAGCTTGACTCCCAGGCGCAGAAGGACGCCGAAGAC
CTGCTTTCCGGGTGGATCAGCAAGGGATGGGCTCCCAAGTCCGCCACGACCAACACCCAGTCGGCCTCCTGGGAT
CTGTTCCTGGCTGGCGACTACGGATTTGCTGAAATCGGCACCTGGATGCAGTCCGAGGCAGACGAGGCCGGAGCC
AAACTTATTCCGATCCCCGCAAAGGATGGCGGCGTGGCCACCGTGCCGACCGGTGGCGAGTTCGCCATGGTCGCC
TACCACAAGAAGGATGCGGAATCCCACTACAAGCTCGCCAATCAGGTTATCGAATGTCTTTCCGAGGACGAGACT
CTGCTTAAGGTAAGCAACGCTCTGAGCAACCTCGCTGCCAAGAAGGCCGTGCGTGCCGAGCAGCTCGCGGCTAGC
GACGGCTTGGCTCAGTGGAAGGAATCCATCGAGAACGCCGCCGGCCGTACCTCCGACTTGGGTCTCAAATACGAG
GAAGCCTCCGCAAGCATCTCCGAATCCCTGCTGGCGGCCCTTAACGCGGCTTGA

Suitably, the ABC transporter ganes may encode a proteins shown as SEQ ID NO: 32-34 or polypeptide with at least 80% sequence identity to SEQ ID NO: 32-34. Suitably, the gene may encode a polypetide with at least 80%, at least 85%, at least 90%, at least 95%, at least 98% or at least 99% sequence identity to SEQ ID NO: 32. Suitably, the gene may encode a polypeptide with at least 80%, at least 85%, at least 90%, at least 95%, at least 98% or at least 99% sequence identity to SEQ ID NO: 33. Suitably, the gene may encode a polypeptide with at least 80%, at least 85%, at least 90%, at least 95%, at least 98% or at least 99% sequence identity to SEQ ID NO: 34.
SEQ ID NO: 32 
MTHRSTWWKTALGIILTLIMMFPVYWMINISFTGKASIRSGDLWPKDFTFDNYARVIADQMPYLGTSILVAVCCV
ILTLVIALPAAYALALLRCPGSGALSFLLIVAQMIPAVVMSLGFYEIYNNIGLLDTLPGLILADSTIAVPFAVML
LTSFMAGIPRSLLEAAEVDGASRTRRFFSIVIPLSRNSIVTVSLFAFLWSWSDFLFASTLDSGGGKMRPITMGLY
NYIGAQTQEWGPMMATAVLASIPATILLVFAQKYVAAGVTAGAVKD
SEQ ID NO: 33 
MTASTTSPVRRAKSGTPVRAKLAIAGFIAPLIIYLVIFYAFPLIQNVSMSLHRYTRRTFVTGDALFVGLDIYKEV
ISSVEFWPVVGQTFVFVVVSLIFQYVIGLALAVFFNDNFKLSGVLRGIMLVPWLLPLIVSGTVWQWMMDPDSGIL
NMFLGLFDIEPIWWLQADNSLWAVIIANIWLGIPFNLVILYSGLQNISGDLYEAASLDGCNAWQRFWKITFPLLK
 
PVTSITLLLGFVYTLKVVDVIWMMSQGTGTSRTLATWAYSMAFGKGTSMTIKYSEASVLGTILIIVALIFGLIYL
RVQKTQETC
SEQ ID NO: 34

MKSNTALKITAALCSCAMLVGVSACGSSNSTTDDKVIEWWDDWTRHEDGSEFDKLVKACAPEGYTIERQAIATSD
LLNNLTTAIKEDNGPDVAVIDNPMIPSAVDAGLVAGSDETGLDVSAWDENLEAPGVVDGQAYGVPLGGSNTLGLM
YNPTIIEAAGVDVSTITDWDSLNAAIKKVVDAGYKGITFSGISGEEGVFQFLPWFWGAGGDLSKLDSQAQKDAED
LLSGWISKGWAPKSATTNTQSASWDLFLAGDYGFAEIGTWMQSEADEAGAKLIPIPAKDGGVATVPTGGEFAMVA
YHKKDAESHYKLANQVIECLSEDETLLKVSNALSNLAAKKAVRAEQLAASDGLAQWKESIENAAGRTSDLGLKYE
EASASISESLLAALNAA

Suitably, the present *B. longum* transitional strain comprises a Lac-I type regulator gene. Suitably, the Lac-I type regulator gene comprises SEQ ID NO: 35 or a sequence with at least 60% sequence identity to SEQ ID NO: 35. Suitably, the Lac-I type regulator gene comprises a sequence with at least 60%, at least 70%, at least 80%, at least 90%, at least 95%, at least 98% or at least 99% sequence identity to SEQ ID NO: 35.

SEQ ID NO: 35
ATGGTGACCATCAACGACGTGGCGCGGGAGGCAGGAGTCTCCAAAACCACGGTCTCATTCGTGCTTTCGGGCTCG
CGCCCCGTTGCTGCAGCCACCGAACAACGTATCCGTGAGGCAATGGACAGACTCGGCTATACCGTCAATCATGCC
GCCCGCAGCTTGTCCACTTCGAAGACCATGACCATAGCCGTGGTGACCAGCAACCGGCAGGACGCCTACTTTGAC
ATTGCCCGTGGCACATACATCAACGGCTTATCCCGAGCAGCCGCCGAAACCGGCTACGACATGCTCATCACTAAC
GATCCAGACGGCTCCGCTACGGAGAACGCCTGCCAATCACACAAGGCGGATGGGCTGGTTTTTTTAGACGTCAGG
CAGAACGATCCGCGTGTGCCGATTGCCGCTGAATCCGGCATTCCAACAGTCTCGCTAGGAGTCCCAGTCAATCCA
ATGAATCTTGATGTGGTCGACACCGACTTCACGGACATGGCGGCCTCGACCATGCGTACACTGCACGATGCCGGA
CACCGCCGCGTCAGCGTCATCACGCTCAGTAGCCGGGTGATTGCCGAACAACTCAACGACACCGCTCGATTCCTC
AGGGAAATCGAACGTTCCGGAGAACGACTTGGCATGCATGCCACTATCCGACATTGCTCTACAAGGCCCGGAATC
ATCGACACAGACATCGCTCGCATTCTTGACGGTCGAGGTGAGGACACCGCATTCGTCATCCATAATGAATCGGCC
GTATTGGTGTTCAGACGGGCAGTGGAACATCGCGGACTGCGCATCCCCGAGGATATCTCCGTCATCGCCATCAAT
GAAAAGCAGATGTCGGACGCTCTGTATCTGCCATATTCCGCCTACGAAAACGACGTGGAACTGGTCACCCAATCT
GCCGTCAATACGCTTGTGGACCGTATCGAACATCCCGAGCTGACGCCGACACGAACGTTGATCAAGGCCTCGTAC
ATAGATCGAGACTCCGTGGCCAATATCTGA

Suitably, the Lac-I type regulator gene may encode a protein shown as SEQ ID NO: 36 or a sequence with at least 80% sequence identity to SEQ ID NO: 36. Suitably, the protein may comprise a sequence with at least 85%, at least 90%, at least 95%, at least 98% or at least 99% sequence identity to SEQ ID NO: 36.

SEQ ID NO: 36

MVTINDVAREAGVSKTTVSFVLSGSRPVAAATEQRIREAMDRLGYTVNHAARSLSTSKTMTIAVVTSN
RQDAYFDIARGTYINGLSRAAAETGYDMLITNDPDGSATENACQSHKADGLVFLDVRQNDPRVPIAAE
SGIPTVSLGVPVNPMNLDVVDTDFTDMAASTMRTLHDAGHRRVSVITLSSRVIAEQLNDTARFLREIE
RSGERLGMHATIRHCSTRPGIIDTDIARILDGRGEDTAFVIHNESAVLVFRRAVEHRGLRIPEDISVI
AINEKQMSDALYLPYSAYENDVELVTQSAVNTLVDRIEHPELTPTRTLIKASYIDRDSVANI

Suitably, the present *B. longum* transitional strain comprises a facilitator superfamily (MFS) gene. Suitably, the MFS gene comprises SEQ ID NO: 37 or a sequence with at least 60% sequence identity to SEQ ID NO: 37. Suitably, the MFS gene comprises a sequence with at least 60%, at least 70%, at least 80%, at least 90%, at least 95%, at least 98% or at least 99% sequence identity to SEQ ID NO: 37.

SEQ ID NO: 37
ATCGCCGAGTTCCATTACGCTATCGGGCATTTTCATTGTGCCGGTCATCGGATTGGTTGCTCAGGCATTCCCGGA
CAGCTCGCTCTCCAGCGTGCAGATGATTGTTTCGGCATCACTCTGACCGCACTGGTTGGCGCTTGGCTGACCGGC
AAACTCGCCAGCATTCTATCCCGGAAGACCGTGGCACTGATTGGTGCAGGCGGCATGCTGCTGTTCGGTCTGCTG
CCGTACTTCGTGCATTCCAGTCTGGCTGCAGTCATCGCGTTTTCCGCGTTGATGGGCGTATGCCTAGGCTTTATC
AACAACGTGCTGCCTACTTTGATCTCCGTGCACTACGAGGGCGATGAGCGACAGTCGATTATGGGTCAGCAGGTT
GCCGTGGCCAGCATCGGTGCGATGGTGTTCATGACCGTGGCCGGCAAACTCGCCACCGCACAGTGGTATCACGCC
TACCTCATCTACTTGTTCGCCGCCGTGGTGCTGGTGGTCTGCGCATTCACGCTGCCCACCAAGAATGGTGAGACG
GACGAAGCCGGCCGGATTCAGGGAACGGGGCCTTCCGCGTCGATTCGCGAGGTTATGACCGGCAAACTGTGGTTC
TTGGTTGTTGCCGGCTTCTTCTTCCTTCTGGCGAACAATGCCTACAGCAACAACTTGTCCCTGTTGGTCGAGCAG
CGCGGCTTGGGCGATGCCGGAACCGCTGGACTGATTTCCACCATCGGACAGTTCGGCGGACTGCTGGCTGGTTTG
TGCGTCGGTCTTATGGTCCGATTCGTGAAGAACCATTTGCTGATGGTCGGCTTCATTGTCGAGGGCCTGTCTTTG
CTGCTGCTTGGCTGCTCGGCCAGCCTGCCACTGCTCATCATCGGCAGTTTCTTTGCCGGAGCCGGCCTGAGCATC
TACTATGCGCAGGCGCCATTCCTCGTCACCGTCATCGAAAAGCCCTACCTCATCCCGCTGGGCATTGCTGCCATG
ACCACGGCCAACGCACTGGGCGGATTTGCCAGCCCTGTGCTCGTCAACGCGATTAACGGACTGTTTGGTTCGCAC
GCGGCCGGCGCGATGTTCATCGGTGCCGCGATTGCTCTGGCCGGAGCGGTGGCTCTCGGTGTGAGCGGATTCCAA
AAGAAGTGCCTCGAAAGCGCGAAGTGA

Suitably, the MFS gene may encode a protein shown as SEQ ID NO: 38 or a sequence with at least 80% sequence identity to SEQ ID NO: 38. Suitably, the protein may comprise a sequence with at least 85%, at least 90%, at least 95%, at least 98% or at least 99% sequence identity to SEQ ID NO: 38.

SEQ ID NO: 38
MAEFHYAIGHFHCAGHRIGCSGIPGQLALQRADDCFGITLTALVGAWLTGKLASILSRKTVALIGAGGMLLFGLL
PYFVHSSLAAVIAFSALMGVCLGFINNVLPTLISVHYEGDERQSIMGQQVAVASIGAMVFMTVAGKLATAQWYHA
YLIYLFAAVVLVVCAFTLPTKNGETDEAGRIQGTGPSASIREVMTGKLWFLVVAGFFFLLANNAYSNNLSLLVEQ
RGLGDAGTAGLISTIGQFGGLLAGLCVGLMVRFVKNHLLMVGFIVEGLSLLLLGCSASLPLLIIGSFFAGAGLSI
YYAQAPFLVTVIEKPYLIPLGIAAMTTANALGGFASPVLVNAINGLFGSHAAGAMFIGAAIALAGAVALGVSGFQ
KKCLESAK

Suitably, the present *B. longum* transitional strain comprises an AraC family transcriptional regulator gene. Suitably, the AraC gene comprises SEQ ID NO: 39 or a sequence with at least 60% sequence identity to SEQ ID NO: 39. Suitably, the AraC gene comprises a sequence with at least 60%, at least 70%, at least 80%, at least 90%, at least 95%, at least 98% or at least 99% sequence identity to SEQ ID NO: 39.

SEQ ID NO: 39

ATGGAGCGCGATGCTTTCCGGCTGCCGGGCCTCACCGCCGGCGATGACAACCAGTATGCCGATCACACGCTCACC
GGCATGGCAGCCGATGCGGCGAACGTCATAGCCGCAGGCGGTCCCGCCCCGCTGACTAGCTTCGGCACTGTCGCT
CAAGCCGCCCATCTCAATCCAGATGACGGCTTCGGCATCATTGGCCATGATCTTGCACACCCATCGCACCTACAC
CGGCATGACTATATGGAAATCACGCACGCCATCGCCGGTACGGTACTGGTCTGGGTCGAAGGAGAGACCAACGTG
CTGACACAGGGCGGCACCATACTCATCAAGCCTGGAGCCCGTCATCTCATCTCCCCCATCATCGAATACGGGCAA
ACACCACACGAGGCGGACATCCTGATTAAACCCGAGCTCATCAGGCAATGCCGCATTCCGATTCTGGAAGCAGCC
GGCGCCGACCGGATGTTCATTAGCTGGCTTGACGATGACCGGCAGACCCACTGCCTGCTGGCAGCCGGCAAGCAC
CACGCCGGCGAGGCCGCTATCAGCCGCATGTTCATCGCCTACTGCATCAACGCAACCTACAGGCCAGACTTCACC
GTCATCGGCAACCTGCTCGAGCTGTTCCACGAAACGTCCCGAGTCTTGGAACACCAGCCACGTACCGATCCGCTG
ATCGCCGCCATCATCGAAACCATCACGGCAGATCCCGCCACGGCCCACAACCAGGCCATAGCGGACACACTTGGA

TACAGCGTGGGATATCTGTCCCGGTACGCGCGCAAGCACAGCGGGCACACACTCGGCCAACTCATCAACGAGGAA
AGGCTCCGACTCGGCGCCGAACTGCTCGTCACCACCGACGACACCATTGCCGAAATCACCCGAACCATTGGCTAC
GAAAGTCCAGCCTATTTCCATAAACTCTTCCGCAGCCGCTACCTCATTACCCCCGACCGCTACCGCAACGACTTC
CGTATCGCATTACGTTGCGGATGA

Suitably, the AraC gene may encode a protein shown as SEQ ID NO: 40 or a sequence with at least 80% sequence identity to SEQ ID NO: 40. Suitably, the protein may comprise a sequence with at least 85%, at least 90%, at least 95%, at least 98% or at least 99% sequence identity to SEQ ID NO: 40.

SEQ ID NO: 40

MERDAFRLPGLTAGDDNQYADHTLTGMAADAANVIAAGGPAPLTSFGTVAQAAHLNPDDGFGIIGHDLAHPSHLH
RHDYMEITHAIAGTVLVWVEGETNVLTQGGTILIKPGARHLISPIIEYGQTPHEADILIKPELIRQCRIPILEAA
GADRMFISWLDDDRQTHCLLAAGKHHAGEAAISRMFIAYCINATYRPDFTVIGNLLELFHETSRVLEHQPRTDPL
IAAIIETITADPATAHNQAIADTLGYSVGYLSRYARKHSGHTLGQLINEERLRLGAELLVTTDDTIAEITRTIGY
ESPAYFHKLFRSRYLITPDRYRNDFRIALRCG

Suitably, the *B. longum* transitional strain comprises a MFS transporter and an AraC family transcriptional regulator gene.

Suitably, the *B. longum* transitional strain comprises a GH43_17, a MFS transporter and an AraC family transcriptional regulator gene. Suitably, the GH43_17, MFS transporter and AraC family transcriptional regulator genes are comprised in a gene cluster..

As used herein, a 'gene cluster' may refer to a group of genes that are located next to each other in a chromosome.

Suitably, the *B. longum* transitional strain comprises each of a GH31, an ABC transporter, a Lac-I type regulator, a MFS transporter and/or an AraC family transcriptional regulator gene.

Suitably, the *B. longum* transitional strain comprises a GH43_17, a MFS transporter, an AraC, a GH31, an ABC transporter, and a Lac-I type regulator gene. Suitably, the GH43_17, MFS transporter, AraC family transcriptional regulator, GH31, ABC transporter, and Lac-I type regulator genes are comprised in aa gene cluster as described above.

Suitably, the *B. longum* transitional strain further comprises a xylulose kinase gene and/or a xylose isomerase gene. Suitably, the xylulose kinase gene and/or xylose isomerase genes are comprised in a gene cluster as defined above.

Suitably, the xylulose kinase gene comprises SEQ ID NO: 41 or a sequence with at least 60% sequence identity to SEQ ID NO: 41. Suitably, the xylulose kinase gene comprises a sequence with at least 60%, at least 70%, at least 80%, at least 90%, at least 95%, at least 98% or at least 99% sequence identity to SEQ ID NO: 41.

SEQ ID NO: 41
ATGACGAGAGTACTGGTTGCCGGCGTAGATACGTCAACTCAATCAACAAAGGTCCGCATTACGGACGC
CGCCACCGGCGAACAGGTTCGGTTCGGGCAGGCCAAGCACCCGGATGGCACCTCGGTCAACCCGGAAT
TCTGGTGGGAGGCCTTCACCAAGGCCGCCGAGCAGGCCGGCGGGCTTGACGATGTCGCGGCCCTCGCG
GTTGGCGGCCAGCAGCATGGCATGGTCATTCTCGACAAGCAGGGCAACGTGATTCGCGATGCGATGCT
CTGGAATGACACCAGTTCCGCCCCGCAGGCCGCCGCCCTGATCGACAAGCTCGGTGCAACTCCGGCCG
AGGGCGACGAACCGGACGACGTGACCGCCCGCGGCAAGCAGCGCTGGGTCAAGGCCGTCGGGTCCTCC
CCCGTCGCTTCCTACACGCTGACCAAGGTGGCGTGGGTGGCCGAGAACGAGCCTGAGAACGCCAAGAA
GATTGCCGCCGTCTGTCTGCCGCACGATTGGCTGAGCTGGCGTATCGCCGGCTATGGCCCGGTGGCCG
AGGGCGAGGACGCTCATCTCGAAGCCCTGTTCACCGACCGTTCCGACGCTTCCGGCACCATTTACTAC
GATGCCGCGCATGACGAGTACCGCCGCGATCTCATCGCCATGGTGCTGACCCCCGCCGAGGGCGAGGA
AGCCGCCAAGGCCCACGCCGACGCCATTGTGCTGCCCACCGTGCTGGGCCCGCATGAGGCAGCCGCCG
TCAAGGCCGACCCCGCCATTGCCGGCAAGGACGTTGAAGGCGGCTGCATCATCGGCCCCGGCGGCGGA
GACAATGCCATGGCCTCGCTGGGCCTCGGCATGGCCGTGGGCGATGTGTCCGTATCGCTCGGCACCTC
CGGCGTGGCCGCGGCCATCGCTGAAAACCCGGTGTACGACCTGACCGGAGCGATTTCTGGCTTTGCCG
ACTGCACCGGTCATTATCTGCCGCTTGCCTGCACCATCAACGGTTCGCGCATTCTGGACGCCGGTCGC
GCCGCCCTTGGCGTGGACTACGACGAGCTGGCCGAACTGGCCTTTAAGGCCGAGCCGGGTGCCGGCGG
CATCACCCTGGTGCCGTACTTCGACGGCGAGCGTACGCCGAACCGTCCGGACGCCACCGCCTCGCTGA
CTGGCCTGACCCTGCACAACACCACCAAGGAGAATCTGGCTCGTGCGTTCGTCGAAGGCCTGCTGTGT
TCCCAGCGCGACTGCCTCGAGCTGATTCGTTCGCTGGGTGCCGAGATCAACCGCATCCTGCTCATTGG
CGGTGGCGCGAAGTCCGTGGCCATCCGCACGCTGGCCCCCTCAATCCTCGGCATGGACGTGACCCGTC
CGGCCACCGACGAATATGTGGCCATCGGCGCCGCCCGTCAGGCCGCCTGGGTGCTGTCCGGCGAGGCC
GAACCGCTGACCTGGCAACTCACCATCGAGGGCGTGGAGACCGGCGAGCCCACCGAAGCCGTGTACGA
GGCATACGCCAAGGCGCGCGGCTGA

Suitably, the xylulose kinase gene may encode a protein shown as SEQ ID NO: 42 or a sequence with at least 80% sequence identity to SEQ ID NO: 42. Suitably, the protein may comprise a sequence with at least 85%, at least 90%, at least 95%, at least 98% or at least 99% sequence identity to SEQ ID NO: 42.

SEQ ID NO: 42

MTRVLVAGVDTSTQSTKVRITDAATGEQVRFGQAKHPDGTSVNPEFWWEAFTKAAEQAGGLDDVAALAVGGQQHG
MVILDKQGNVIRDAMLWNDTSSAPQAAALIDKLGATPAEGDEPDDVTARGKQRWVKAVGSSPVASYTLTKVAWVA
ENEPENAKKIAAVCLPHDWLSWRIAGYGPVAEGEDAHLEALFTDRSDASGTIYYDAAHDEYRRDLIAMVLTPAEG
EEAAKAHADAIVLPTVLGPHEAAAVKADPAIAGKDVEGGCIIGPGGGDNAMASLGLGMAVGDVSVSLGTSGVAAA
IAENPVYDLTGAISGFADCTGHYLPLACTINGSRILDAGRAALGVDYDELAELAFKAEPGAGGITLVPYFDGERT
PNRPDATASLTGLTLHNTTKENLARAFVEGLLCSQRDCLELIRSLGAEINRILLIGGGAKSVAIRTLAPSILGMD
VTRPATDEYVAIGAARQAAWVLSGEAEPLTWQLTIEGVETGEPTEAVYEAYAKARG

Suitably, the xylose isomerase gene comprises SEQ ID NO: 43 or a sequence with at least 60% sequence identity to SEQ ID NO: 43. Suitably, the xylose isomerase gene comprises a sequence with at least 60%, at least 70%, at least 80%, at least 90%, at least 95%, at least 98% or at least 99% sequence identity to SEQ ID NO: 43.

SEQ ID NO: 43
ATGGGTCTGTGGGATGTTGACAAGATCGAGTACGTCGGCCGCGCCAAAGGACCGAAGGAAGACTTCGCCTTCCAT
TACTACGATGCCGACAAGGTCGTTGCCGGCAAGAAGATGAAGGATTGGCTGCGCTTCGGCGTTGCTTGGTGGCAC
ACCTTCAACCAGGAACTGGTTGATCCGTTCGGCACCGGCACCGCGCACCGCCCGTACTACAAGTACACCGATCCG
ATGGACCAGGCTCTGGCCAAGGTCGACTACGCCTTCGAGCTGTTCCAGAAGCTGGGCGTCGAGTACTTCTGCTTC
CACGATCGTGACATCGCCCCCGAAGGCGACACCCTGCGCGAGACCAACGCCAACCTCGACAAGGTCGTTGACAAG
ATCGACGAGAATATGAAGTCCACCGGTGTCAAGCTGCTGTGGAACACCTCCTCCCTGTTCACCAACCCGCGCTTC

GTGTCCGGCGCCGCCACTTCTCCGTTCGCCGACATCTACGCCTACGCCGGTGGCCAGCTCAAGAAGAGCTTGGAG
ATCGGCAAGCGCCTGGGCGCCGAGAACTACGTGTTCTGGGGTGGCCGCGAAGGCTACGAGAACCTGTGGAACACC
GAGATGAAGCGCGAGACCGACCACATCGCCAAGTTCTTCCACATGTGCGCAGATTACGCCAAGGAAATCGGCTTT
GAGGCCCAGTTCCTGATCGAGCCGAAGCCGAAGGAGCCGACGCTGCACCAGTACGACTTCGATGCCGCCACCGCC
ATCGAGTTCCTGCGCAACCACGACCTGACCGACGTCTTCAAGCTGAACTTGGAAGGCAACCACGCCAACCTGGCC
GGCCACACCTACCAGCACGAGATCCGCGTGGCCCGCGAGTCCGGCTTCCTCGGTTCCCTCGACGCCAACCAGGGC
GACAAGCTCATCGGCTGGGATATGGACGAGTTCCCGACCGATCTGTACGAGACCGTCGCCGTCATGTGGGAAGTC
CTGCAGGCCGGCTCCATCGGACCTCACGGTGGTCTGAACTTCGACGCCAAGCCGCGCCGTACCTCCTTCTACGAG
GAGGACCTGTTCCGCTCCCACATCGCCGGCATGGATGCCTACGCCGCCGGCCTGCTGGTTGCCGACAAGATGAAC
CAGGACGGCTTCATCCAGAATCTTCAGGCCGAGCGCTACAGCTCCTACGACTCCGGCATCGGCAAGGACATCGAC
GAGGGCAACGTCACCTTGGCCGACCTCGAAGCCTACAGCCTCGACAAGCCGCAGTCCGAGCTCATCGCCGCCACC
AAGTCCGATCACCTCGAGTCCGTCAAGGCCACCATCAACAACTACATCATTGATGCCCTGGCTGAGGTCGAGTGA

Suitably, the xylulose isomerase gene may encode a protein shown as SEQ ID NO: 44 or a sequence with at least 80% sequence identity to SEQ ID NO: 44. Suitably, the protein may comprise a sequence with at least 85%, at least 90%, at least 95%, at least 98% or at least 99% sequence identity to SEQ ID NO: 44.

SEQ ID NO: 44

MGLWDVDKIEYVGRAKGPKEDFAFHYYDADKVVAGKKMKDWLRFGVAWWHTFNQELVDPFGTGTAHRPYYKYTDP
MDQALAKVDYAFELFQKLGVEYFCFHDRDIAPEGDTLRETNANLDKVVDKIDENMKSTGVKLLWNTSSLFTNPRF
VSGAATSPFADIYAYAGGQLKKSLEIGKRLGAENYVFWGGREGYENLWNTEMKRETDHIAKFFHMCADYAKEIGF
EAQFLIEPKPKEPTLHQYDFDAATAIEFLRNHDLTDVFKLNLEGNHANLAGHTYQHEIRVARESGFLGSLDANQG
DKLIGWDMDEFPTDLYETVAVMWEVLQAGSIGPHGGLNFDAKPRRTSFYEEDLFRSHIAGMDAYAAGLLVADKMN
QDGFIQNLQAERYSSYDSGIGKDIDEGNVTLADLEAYSLDKPQSELIAATKSDHLESVKATINNYIIDALAEVE

### Human milk oligosaccharide (HMO)

Suitably, the present *B. longum* transitional strain preferentially utilizes 3- fucosyllactose (3-FL) compared to other *B. longum* transitional strainsas demonstrated by a better growth, for example as shown in the present Examples.

Suitably, the present *B. longum* transitional strain may have a growth rate of at least 0.6 k when cultured in the presence of 3-FL. Suitably, the present *B. longum* transitional strain may have a growth rate of at least 0.7 k, at least 0.8 k or at least 0.9 k when cultured in the presence of 3-FL. Growth rate may be calculated by culturing a bacterium on a given substrate, or mixture of substrates, for a period of time and modelling the growth curve using a logistic growth model, to obtain the relative growth rate k. An illustrative method for determining growth rate is provided in the present Examples. Without wishing to be bound by theory, preferential growth on 3-FL is considered to be advantageous as levels of 3-FL rise in human breastmilk during the weaning period. Preferentially growth on 3-FL indicates that the present *B. longum* transitional strain may be particularly adapted to survive and grow in the microbiome during the weaning phase.
uitably, the present B. longum transitional strain comprises a glycosyl hydrolase family 25 (GH25) gene. Suitably, the GH25 gene comprises SEQ ID NO: 45 or a sequence with at least 60% sequence identity to SEQ ID NO: 45. Suitably, the GH25 gene comprises a sequence with at least 60%, at least 70%, at least 80%, at least 90%, at least 95%, at least 98% or at least 99% sequence identity to SEQ ID NO: 45.

SEQ ID NO: 45

ATGAGCAATCCAACAAATGATGGTATCAACTTGAATTACCTCGCAAACGTGCGTCCCTCGTCGCGACAGCTTGTC
TGGCAGCGTATGGAGATGTATGCCTTCATACACTTCGGCATGAATACCATGACAGACAGGGAATGGGGTCTTGGG
CATGAGGATCCGGCGCTGTTCGATCCACAGAATGTAGATGTGGAACAGTGGATGGATGCGCTGGTGGCTGGTGGA
ATGACTGGTGTCATCTTGACGTGCAAGCATCATGATGGATTCTGCCTGTGGCCATCGCGTTACACGCAGCATACC
GTTGCCGCCTCGCCGTGGAGGGACGGAAAAGGGGATCTCGTTCGTGAGGTCAGTGAGTCCGCCAGACGTCATGGA
CTGAAGTTCGGCGTATACCTGTCTCCGTGGGATCGAACCGAAGAATCCTATGGCAAAGGCAAGGCATATGACGAT
TTCTACGTCGGACAATTGACTGAGTTGCTCACCCAGTACGGACCGATTTTCTCCGTATGGCTGGATGGTGCCAAT
GGTGAGGGCAAGAACGGCAAGACTCAGTATTACGACTGGGATCGTTACTACAACGTCATTCGTTCGCTTCAACCC
AATGCGGTGATATCCGTATGCGGTCCCGACGTTCGCTGGGCTGGAAATGAAGCCGGACATGTACGTGACAACGAA
TGGAGTGTCGTGCCCCGACGACTGCGTTCGGCGGAACTGACTATGGAAAATTCACAGCAGGAGGACGATGCGTCC
TTTGCTTCTACGGTTCGCTCTCAAGATGACGACCTTGGAAGTCGTGAGGCGGTTTCCGGATACGGGGATGACGTC
TGTTGGTACCCAGCTGAGGTCGATACCTCCATTCGCCCTGGATGGTTCTATCACAAGTATGAAGACGACAAGGTC
ATGAGCGCAGATCAGCTTTTTGACCTCTGGCTTTCCGCAGTCGGCGGTAATTCGTCTCTTCTGCTCAATATTCCT
CCGTCTCCAGAAGGACTGTTCGCAGAACCGGATGTGGAGTCGCTCAAGGGGCTGGGAAGCCGTATCAATGAATTC
CGCAAAGCATTGGCTTCGTCTTGTTGCGAGGTCAAGACCAGCAGCGCGGACGAAACTGCAATGCGACTTCTCGAT
GGGAATCAGGACACGTATTGGTCTCCTGATGCCAATGACGTGGCCCCTGCCGTCACGCTCACTTTCCCGCAGCTG
ACGACGATCAATGCCGTTGTGGTTGAAGAGGCCATAGAGTATGGGCAGCGCATTGAACATATGCGCGTTACTGGT
GTGCTATCTGATGGTACTGAGTGTGTACTCGGCCAGTTCGGCACAGTGGGATACCGCAGGATACTCCGCTTCGAC
GATGTCGAAGTATCTTCGGTTACCCTACATGTGGATGATTCAAGGTTCACGCCAATGATCAGCCGTGCAGCTGCG
GTGCGGATATAA

Suitably, the GH25 gene may encode a protein shown as SEQ ID NO: 46 or a sequence with at least 80% sequence identity to SEQ ID NO: 46. Suitably, the protein may comprise a sequence with at least 85%, at least 90%, at least 95%, at least 98% or at least 99% sequence identity to SEQ ID NO: 46.

SEQ ID NO: 46

MSNPTNDGINLNYLANVRPSSRQLVWQRMEMYAFIHFGMNTMTDREWGLGHEDPALFDPQNVDVEQWMDALVAGG
MTGVILTCKHHDGFCLWPSRYTQHTVAASPWRDGKGDLVREVSESARRHGLKFGVYLSPWDRTEESYGKGKAYDD
FYVGQLTELLTQYGPIFSVWLDGANGEGKNGKTQYYDWDRYYNVIRSLQPNAVISVCGPDVRWAGNEAGHVRDNE
WSVVPRRLRSAELTMENSQQEDDASFASTVRSQDDDLGSREAVSGYGDDVCWYPAEVDTSIRPGWFYHKYEDDKV
MSADQLFDLWLSAVGGNSSLLLNIPPSPEGLFAEPDVESLKGLGSRINEFRKALASSCCEVKTSSADETAMRLLD
GNQDTYWSPDANDVAPAVTLTFPQLTTINAVVVEEAIEYGQRIEHMRVTGVLSDGTECVLGQFGTVGYRRILRFD
DVEVSSVTLHVDDSRFTPMISRAAAVRI

Suitably, the present B. longum transitional strain comprises a glycosyl hydrolase family 95 (GH95) gene. Suitably, the GH95 gene comprises SEQ ID NO: 47 or a sequence with at least 60% sequence identity to SEQ ID NO: 47. Suitably, the GH95 gene comprises a sequence with at least 60%, at least 70%, at least 80%, at least 90%, at least 95%, at least 98% or at least 99% sequence identity to SEQ ID NO: 47.

SEQ ID NO: 47

ATGAAACTCACATTCGATGGAATCTCTTCGTGCTGGGAAGAAGGCATCCCGCTCGGCAACGGACGCATGGGAGCG
GTCCTGTGTTCCGAACCGGAAACCGACGTGCTGTATCTCAACGACGACACCCTTTGGTCAGGATATCCACACGCG
GAAACCTCGCCGGTGACGCCGGAGATTGTGGCCAAGGCACGCCAGGCGTCGTTGCAGGACGACTACACCGCCGCC
ACGCGAATCATCAAGGAAGCCACACTGCAGGAAAAGGACGAACAGATTTACGAGCCATTCGGAACGGCCCGTATT
CAGTACTCGACCCCTGCAGACGGCCGTGAGAGCATGAAACGCCAGCTGGATCTTGCAAGGGCGCTCGCCGGTGAA
ACATTCCAGATGGGTGATGCCAACGTTCATGTCGACGCATGGTGCAGCGAGCCTGATGACCTGTTGGTCTACAGG
ATGTCATCGGATGCGCCGGTTGATGTGAACATCAGTGTCGCCGGCACTTTCCTCAAACAATCGCGCGCCTCGTTG
GAAACGGTATCCGACGGTCATCGGGCCACACTCGTCGTCATGGGCCGGATGCCTGGACTCAACATCGGGCTCCTC
CCTCATCCTTCCGAACATCCTTGGGAAGATGAGCAGGACGGAACCGGAATGGCGTACGCCGGTGCGTTCTCCCTT
ACCGTCACAGGTGGCGACATCAATGTGGACGACAACAGTCTGCAATGTTCGCACATCACCGGATTATCGCTCCGC
TTCCGCAGTATGAGCGGATTCAAGGGAAGCGACCAGCAGCCGGAACGAAGCATGACGGTTATCGCCGACCATCTG
GAGAAAACCATCGACGAGTGGTCGACCGACCTGCAGACCATGCTCGACCGCCATATCGCGGACTACCGCAGATAT
TTCGACAGGGTGGCCATCCATCTCGGTTCAGCCCATGATGACGATACGGAACTACCGTTCTCGGCGATCCTTCGC
TCGGATGAGAACAAAGAACCGCATCGTCTGGAGATGCTGGCGGAGGCAATGTTCGATTTCGGCCGGTATATGCTT
ATCTCCTCGTCCAGGCCACACACCCAGCCGGCGAATCTGCAGGGGATTTGGAACCATAAGGACTTCCCAAACTGG
TACAGCGCCTACACGACGAACATCAACGTCGAGATGAACTATTGGATGACCGGCCCCTGCGCGCTCAAGGAGCTC
ATCGAGCCGCTCGTCTCCATGAATGAGGAGCTGCTGGCACCGGGGCACGATGCCGCTGACAGGATTCTCGGCTGC
CGAGGATCGGCTGTCTTCCATAATGTCGATCTCTGGCGTAGGGCCCTTCCTGCGAACGGCGATCCGATGTGGGCG
TTCTGGCCGTTCGGCCAGGCATGGATGTGCCGGAACCTGTTCGATGAATATCTGTTCAACCAGGATGCATCGTAC
CTGGCCCGCATCTGGCCGATCATGCGGGACAACGCGCGATTCTGCATGGATTTCCTATCGGAGACAGAGCATGGG
CTGGCCCCGTCCCCTGCAACATCACCGGAGAACTGTTTCCTGGTGAACGGAGAACCGGTATCCGTTGCGCAAAGC
AGTGAGAATGCCACGGCCATCGTGCGTAATCTGCTTGATGATTTGATTCAGGCTTCTCACGATCTGGAAAACCTT
GACGAAGAGGACAGAAATCTGGTCCGTGAAGCGGAATCCGTCCGTTCCCAACTGGCTGAAACGCGATTGGGAGCT
GATGGAAGAGTCCTTGAATGGAACGACGAATTCATCGAATCCGATCCACAGCACCGCCATCTGTCCCACCTTTAC
GAACTGCATCCTGGTGCAGGCATCACGTCTAAGACTCCGCGTCTGGAGGAAGCCGCGAGAAAATCCCTCGAAGTG
CGTGGCGATGATGGTTCCGGTTGGAGCATCGTATGGCGCATGATCATGTGGGCACGTCTGCGTGATGCGGAACAC
GCCAAACGAATCATAGGCATGTTCCTACGGCCGGTGGATGCGAACGCTGAAACCAATCTGCTGGGCGGAGGAGTG
TACGACAGCGGATTATGCGCCCACCCGCCGTTCCAGATCGACGGAACCTTGGATTCCCGGCGGCCTTGTCGGAG
ATGCTCGTCCAAAGCCACGATGGCTGGATTCGCGTTCTTCCGGCCCTGCCGGAGGATTGGCATGAGGGAAGCTTC
CATGCGCTCCGCGCAAGAGGTGGAATCCAAGTGGATGCGACCTGGACGGATCAGACAGTGGAATATACGTTGCGC
TGTTCGAAGCCCACGGAGATTACGCTGAACGTTCTGGGGACTGATATGGACGTGTCGCATTGTCTCCGGATAAG
CCATTCAAGGGAACCATCCGGCGTTAA

Suitably, the GH95 gene may encode a protein shown as SEQ ID NO: 48 or a sequence with at least 80% sequence identity to SEQ ID NO: 48. Suitably, the protein may comprise a sequence with at least 85%, at least 90%, at least 95%, at least 98% or at least 99% sequence identity to SEQ ID NO: 48.

SEQ ID NO: 48

MKLTFDGISSCWEEGIPLGNGRMGAVLCSEPETDVLYLNDDTLWSGYPHAETSPVTPEIVAKARQASLQDDYTAA
TRIIKEATLQEKDEQIYEPFGTARIQYSTPADGRESMKRQLDLARALAGETFQMGDANVHVDAWCSEPDDLLVYR
MSSDAPVDVNISVAGTFLKQSRASLETVSDGHRATLVVMGRMPGLNIGLLPHPSEHPWEDEQDGTGMAYAGAFSL
TVTGGDINVDDNSLQCSHITGLSLRFRSMSGFKGSDQQPERSMTVIADHLEKTIDEWSTDLQTMLDRHIADYRRY
FDRVAIHLGSAHDDDTELPFSAILRSDENKEPHRLEMLAEAMFDFGRYMLISSSRPHTQPANLQGIWNHKDFPNW
YSAYTTNINVEMNYWMTGPCALKELIEPLVSMNEELLAPGHDAADRILGCRGSAVFHNVDLWRRALPANGDPMWA
FWPFGQAWMCRNLFDEYLFNQDASYLARIWPIMRDNARFCMDFLSETEHGLAPSPATSPENCFLVNGEPVSVAQS
SENATAIVRNLLDDLIQASHDLENLDEEDRNLVREAESVRSQLAETRLGADGRVLEWNDEFIESDPQHRHLSHLY
ELHPGAGITSKTPRLEEAARKSLEVRGDDGSGWSIVWRMIMWARLRDAEHAKRIIGMFLRPVDANAETNLLGGGV
YDSGLCAHPPFQIDGNLGFPAALSEMLVQSHDGWIRVLPALPEDWHEGSFHALRARGGIQVDATWTDQTVEYTLR
CSKPTEITLNVLGTDMGRVALSPDKPFKGTIRR

Suitably, the present *B. longum* transitional strain may comprise a GH25 and a GH95 gene as defined herein.

### EMBODIMENTS

The present invention provides the embodiments according to the following numbered clauses:
1. A *Bifidobacterium longum* transitional microorganism strain deposited with Collection nationale de cultures de micro-organismes (CNCM) under deposit number CNCM I-5942 or a *B. longum* transitional strain having an identifying characteristic of the *B. longum* transitional strain deposited under deposit number CNCM I-5942.
2. A *B. longum* transitional microorganism strain which has an Average Nucleotide Identity (ANI) of at least 99% to a *B. longum* strain deposited with the CNCM under deposit number CNCM I-5942.
3. A *B. longum* transitional strain according to clause 1 which has an ANI of at least 98.1% to a *B. longum* transitional strain deposited under deposit number CNCM I-5942.
4. A *B. longum* transitional strain according to any preceding clause wherein the *B. longum* transitional strain is not resistant to any one of tetracycline and erythromycin.
5. A *B. longum* transitional strain according to any preceding clause wherein the *B. longum* transitional strain is not resistant to any one of tetracycline, erythromycin, clindamycin and ampicillin.
6. A *B. longum* transitional strain according to any preceding clause wherein the *B. longum* transitional strain is not resistant to any of tetracycline, erythromycin, clindamycin, ampicillin, gentamycin, streptomycin, chloramphenicol and vancomycin.
7. A *B. longum* transitional strain according to any preceding clause wherein:
   (i) lack of resistance to tetracycline is due to absence of a tetracycline resistance gene, suitably a *tetW* gene which encodes a protein with at least 80% sequence identity to SEQ ID NO: 1 and/or a *tetQ* which encodes a protein with at least 80% sequence identity to SEQ ID NO: 2;
   (ii) lack of resistance to erythromycin is due to absence of a erythromycin resistance gene, suitably a *Erm49* gene which encodes a protein with at least 80% sequence identity to SEQ ID NO: 3;
   (iii) lack of resistance to erythromycin and/or clindamycin is due to absence of a corresponding resistance gene, suitably a *Erm(X)* gene which encodes a protein with at least 80% sequence identity to SEQ ID NO: 4; and/or
   (iv) lack of resistance to chloramphenicol is due to absence of a chloramphenicol resistance gene, suitably a *CrmX* gene which encodes a protein with at least 80% sequence identity to SEQ ID NO: 6.
8. A *B. longum* transitional strain according to any preceding clause wherein the *B. Longum* transitional strain comprises a glycosyl hydrolase family 43_17 (GH43_17) gene; suitably wherein the GH43_17 gene comprises SEQ ID NO: 7 or a sequence with at least 60% sequence identity to SEQ ID NO: 7.
9. A *B. longum* transitional strain according to any preceding clause, which further comprises a major facilitator superfamily (MFS) gene; suitably wherein the MFS gene comprises SEQ ID NO: 37 or a sequence with at least 60% sequence identity to SEQ ID NO: 37.
10. A *B. longum* transitional strain according to any preceding clause, which further comprises an AraC gene; suitably wherein the AraC gene comprises SEQ ID NO: 39 or a sequence with at least 60% sequence identity to SEQ ID NO: 39.
11. A *B. longum* transitional strain according to clause 10, wherein the GH43_17, MFS and AraC genes are comprised in a gene cluster.
12. A *B. longum* transitional strain according to any of clauses 8 to 11, wherein the *B. longum* transitional strain further comprises one or more of a GH31 gene, and a LacI gene; preferably further comprising a xylulose kinase gene and a xylose isomerase gene.
13. A *B. longum* transitional strain according clause 12, wherein the GH43_17, MFS, AraC, GH31, and LacI genes are comprised in a gene cluster; preferably wherein the GH43_17, MFS, AraC, GH31, LacI, xylulose kinase and xylose isomerase genes are comprised in a gene cluster.
14. A *B. longum* transitional strain according to any preceding clause, which further comprises one or more genes encoding for one or more glycoside hydrolases selected from GH43_17, GH43_22, GH43_27, GH43_29, GH121, GH43_24, GH127, GH30_5, GH 43_32 and GH30.
15. A *B. longum* transitional strain according to any preceding clause, which further comprises GH29 and GH95 genes.
16. A *B. Longum* transitional strain according to any preceding clause wherein the *B. Longum* transitional strain preferentially utilizes 3-fucosyllactose (3-FL).
17. A *B. Longum* transitional strain according to any preceding clause wherein the *B. longum* transitional strain has a growth rate of at least 0.6 k when cultured in the presence of 3-FL.
18. A composition comprising a *B. longum* transitional strain according to any preceding clause; preferably wherein the composition is a probiotic composition.
19. A composition comprising a *B. longum* transitional strain according to any preceding clause and at least one further probiotic and/or prebiotic.
20. Use of a *B. longum* transitional strain or a composition according to any preceding clause as a dietary supplement; suitably wherein the *B. longum* transitional strain or composition is administered to a subject in combination with a fiber containing diet or fiber containing foodstuffs.
21. Use of a *B. longum* transitional strain or a composition according to any of clauses 1 to 19 to promote and/or assist the metabolism of a fiber containing diet or fiber containing foodstuffs by a subject.
22. A method for promoting and/or assisting the metabolism of a fiber containing diet or fiber containing foodstuffs by a subject; the method comprising administering a *B. longum* transitional strain or a composition according to any of clauses 1 to 19 to the subject.
23. Use of a *B. longum* transitional strain or a composition according to any of clauses 1 to 19 to promote and/or assist the transition from a milk-based diet to solid food in an infant and/or in a young child.
24. A method of promoting and/or assisting the transition from a milk-based diet to solid food in an infant and/or in a young child, the method comprising administering to the infant and/or to the young child a *B. longum* transitional strain or a composition according to any of clauses 1 to 19.
25. The use according to any of clauses 20, 21 or 23 or the method according to clause 22 or 24, wherein the *B. longum* transitional strain is administered in combination with a prebiotic.
26. The composition according to clause 19 or the use or method according to clause 25, wherein the prebiotic is a fibre and/or a human milk oligosaccharide (HMO); suitably wherein the HMO is 3-FL.

### EXAMPLES

The invention is further described with reference to the following examples. It will be appreciated that the invention as claimed is not intended to be limited in any way by these examples.

### Example 1 - Isolation and phylogenetic identity of NCC 5025 strain

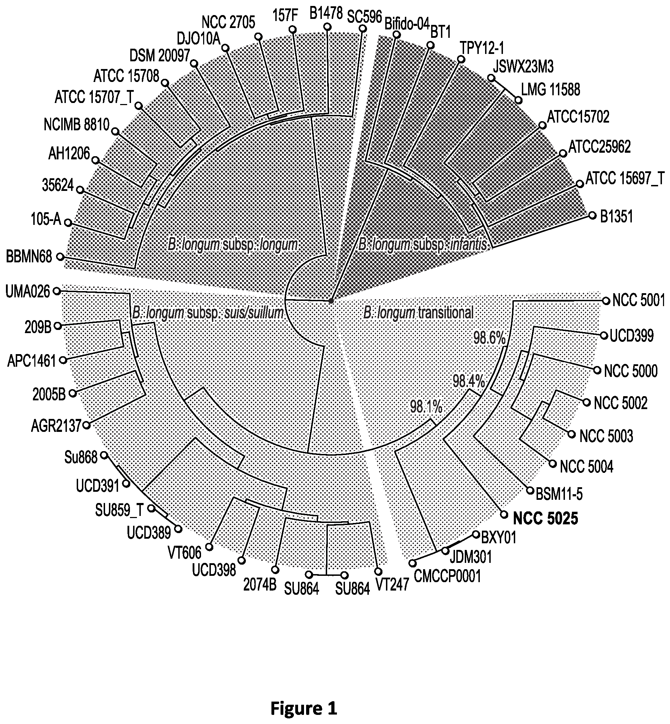

The *B. longum* transitional strain NCC 5025 was isolated at Nestlé Research from the feces of a weaning infant aged (between 6 to 12 months old). It was obtained from stool samples by cultivation on Eugon Tomato agar, followed by preliminary identification using MALDI-ToF MS (Biotyper, Bruker Scientific Instruments, Billerica, USA) and confirmed by sequencing. The isolate was deposited in the Nestlé Culture Collection (Nestlé Research, Lausanne, Switzerland) under NCC 5025 and was further deposited at the "Collection Nationale de Culture Microorganismes" (CNCM, Paris, France) under CNCM I-5942. PacBio sequencing of NCC 5025 was performed according to supplier's recommendations. The sequencing data was further assembled using the Hierarchical Genome Assembly Process (HGAP4) de novo assembly analysis application available through the SMRT Link portal (Pacific Biosciences, Menlo Park, USA). Obtained sequences were compared to publicly available *B. longum* genomes by Average Nucleotide Identity (ANI) computed using OrthoANIu v1.2 (Yoon et al., 2017). The generated matrix of pairwise genome similarities was further used to build a UPGMA phylogenetic tree using the BioNumerics software (v8.0, bioMérieux SA, Marcy l'Etoile, France). Analysis revealed that the NCC 5025 belongs to the *B. longum* transitional group as it clustered with others strains of this putative newly described subspecies (Vatanen et al.;Cell; 2022 Nov 10;185(23):4280-4297.e12) . Phylogenetically, NCC 5025 lies in between strains isolated from China (e.g. JDM301; CMCC P001) and strains isolated from Bangladesh (NCC 5000-NCC5004), sharing an Average Nucleotide Identity (ANI) of 98.4% with this latter group of strains (see Figure 1).

### Example 2 - Antibiotic Resistance Profiling

Phenotypic antibiotic testing of *B. longum* transitional strains was performed according to the recommendations made by EFSA (EFSA J 16, e05206, doi:10.2903/j.efsa.2018.5206 (2018)) following the official method ISO 10932. As required in the ISO method 10932 *B. longum* ATCC 15707 was used as internal control. Minimal Inhibitory Concentration (MICs) obtained for this control strain were within the range determined for this strain (see Annex of the ISO method). Obtained MICs were compared to the EFSA applicable thresholds (*EFSA Journal* 2012 Guidance on the assessment of bacterial susceptibility to antimicrobials of human and veterinary importance) determining the sensitivity or resistance phenotype to the list of relevant antibiotic. MICs obtained for *B. longum* transitional NCC 5000, 5001, 5002, 5003, 5004 and 5025 are depicted in Table 1.

Results showed that most of the strains were considered resistant to several antibiotic considered of importance for EFSA. *B. longum* transitional NCC 5000 and NCC 5001 are considered resistant to erythromycin and clindamycin. NCC 5003 is considered resistant to tetracycline, erythromycin and clindamycin. NCC 5004 is considered resistant to tetracycline, erythromycin, clindamycin and ampicillin. As well, *B. longum* transitional NCC 5002 showed a resistance for tetracycline.

*B. longum* transitional NCC 5025 was the only strain sensitive to all antibiotics considered relevant by EFSA, namely gentamycin, streptomycin, tetracyclin, erythromycin, clindamycin, ampicillin and vancomycin.

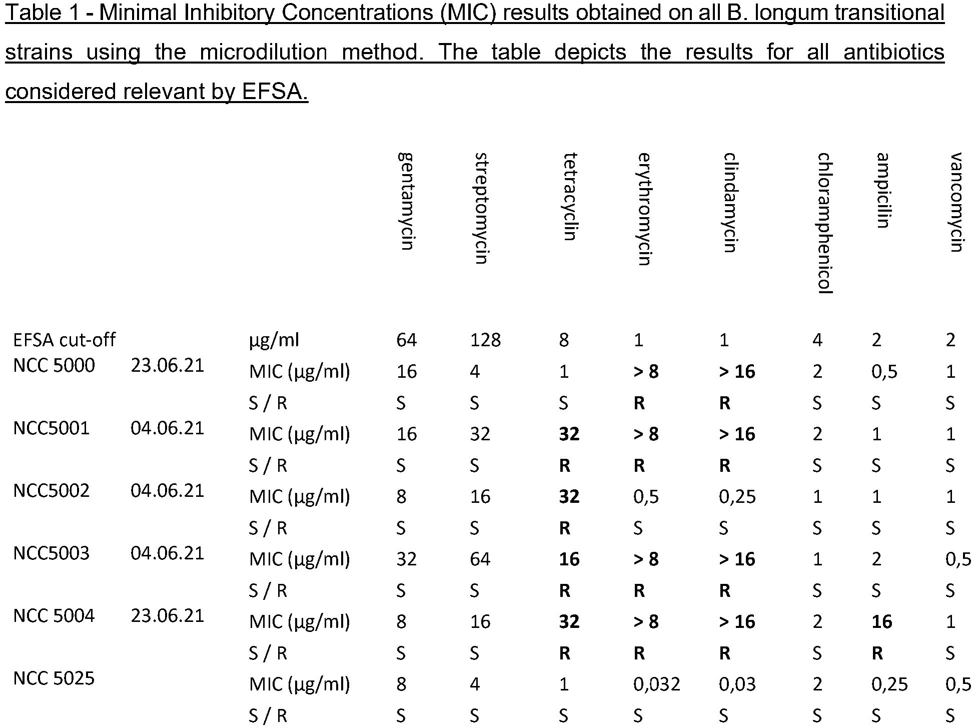

### Example 3 - Carbohydrate Active Enzyme (CaZy) profiling

Strains of *B. longum* transitional were annotated to CAZymes combining dbCAN3 (Yin et al., 2012; Zhang et al., 2018) tools and databases HMMdb (v10) and DIAMOND (v 2.0.14). Query sequences with > 0.50 coverage and e-value < 1e-15 were annotated with HMMER according to the dbCAN CAZyme domain HMM database. DIAMOND was also used to annotate query sequences with hits in the CAZy database (Lombard et al., 2014) (http://www.cazy.org/) with > 0.90 identity, and e-value < 1e-100. HMMER annotation was prioritized and used in instances of mismatched CAZyme annotations of query sequences between HMMER and DIAMOND tools. Only CAZyme families and subfamilies encoding Glycoside Hydrolases (GHs) and Polysaccharide Lyases (PLs) were used for comparative analyses of *B. longum* clades.

All genes predicted as CAZymes by dbCAN3 were further analysed for the putative extracellular activity. To this end, signal peptides were predicted using DeepSig (Savojardo et al; Bioinformatics, 34(10), 2018, 1690-1696), a Deep Convolutional Neural Network trained on the well-known SignalP (v4.0) (Petersen et al.; Nat Methods; 2011 Sep 29;8(10):785-6). and tested on UniproKB. The network takes the N-terminus of the input sequence with a threshold of 21 residues. The input is then forwarded to the feature extraction module, to then output a binary output (absence, presence) for the prediction of signal peptides.

The analysis revealed that NCC 5025 harbors a unique enzyme setup compared to other genomes in the *B. longum* transitional clade. In particular, it is the only strain to have a glycosyl hydrolase (GH) family 43 subfamily 17 (GH43_17), that encodes for an enzyme with both α-L-arabinofuranosidase (EC 3.2.1.55) and endo-β-1,4-xylanase (EC 3.2.1.8) activities, with capacity to breakdown complex carbohydrates like arabinan, arabinogalactan, and arabinoxylan. To date, the only characterized enzymatic activity of GH43_17 come from *Bacteroides intestinalis* (Pereira et al.; Nat Commun. 2021 Jan; 12(1):459.) and *Caldicellulosiruptor owensensis* (Helbert *et al;* 2019; 116(13); 6063-6068).

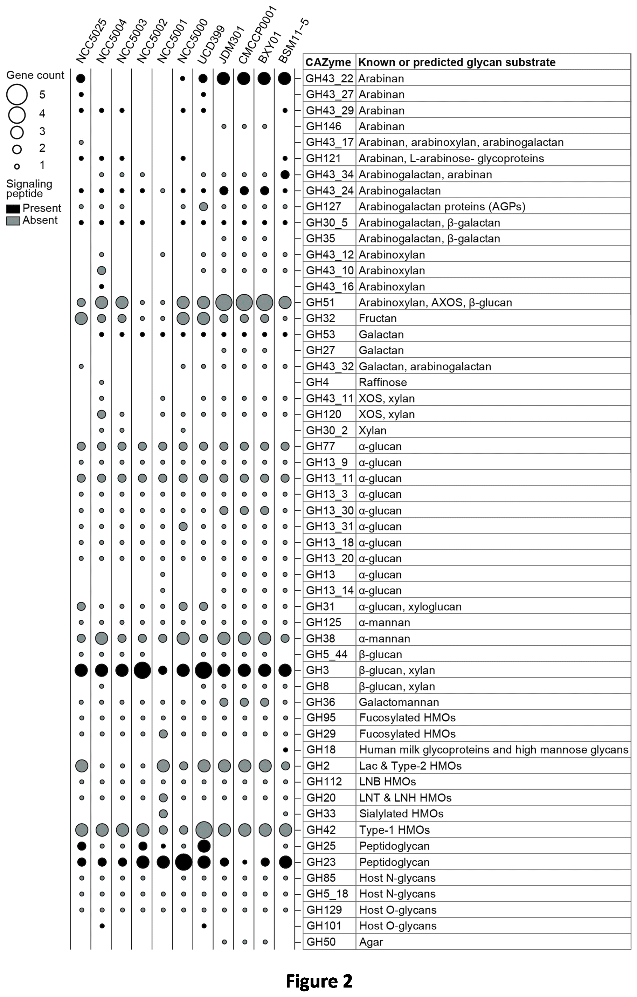

Altogether, amongst *B. longum* transitional, NCC 5025 possesses a unique CAZyme profile. Its genome encodes five different CAZymes that target arabinans (GH43_22, GH43_27, GH43_29, GH121, and the exclusive GH43_17), compared to UCD399 and BSM11-5, which encode four and the rest of the *B. longum* transitional genomes which encode either three or fewer of these CAZymes (Figure 2). In addition, four of the five arabinan-degrading CAZymes present in NCC 5025 have a signal peptide, which grants this bacterium an advantage as a primary degrader of complex structures of arabinan when present in high molecular weight, usually in the diet (Figure 2).

*B. longum* transitional NCC5025 also encodes five different CAZymes that target arabinogalactans (GH43_24, GH127, GH30_5, GH43_32, and GH43_17), two of which have signal peptides. Additionally, NCC5025 contains three genes that encode an inulin-degrading CAZyme (GH32), similar to NCC5000 and UCD399. The other *B*. *longum* transitional possess two or less number of genes that encode GH32, which grants them less efficiency to utilize inulin from the environment (Figure 2).

B. longum transitional NCC 5025 contains as well several enzymes implicated in the degradation and metabolization of Human Milk Oligosaccharides (HMO). It contains a GH20 (lacto-N-biosidase) enzyme and a GH112 (lacto-N-biose phosphorylase) and several GH42 (β-galactosidase), implicated in the degradation and metabolization of Lacto-N-tetraose (LNT) and its subcomponents. The strain also possess a GH29 and a GH95 (fucosidases) encoding genes which are implicated in the degradation and metabolization of fucosylated human milk oligo-saccharides, such as 2'FL, 3'FL or diFL. Taken together, *B. longum* transitional NCC 5025 can serve as a primary degrader of arabinan, arabinogalactans and inulin thanks to their unique CAZyme repertoire, including the exclusive presence of GH43_17. Carbohydrate blends that contain arabinan in combination with arabinogalactans, inulin or fucosylated HMOs, etc., may grant NCC 5025 advantage for growing and producing beneficial metabolites.

### Example 4 - GH43 17 encoding gene cluster of NCC 5025

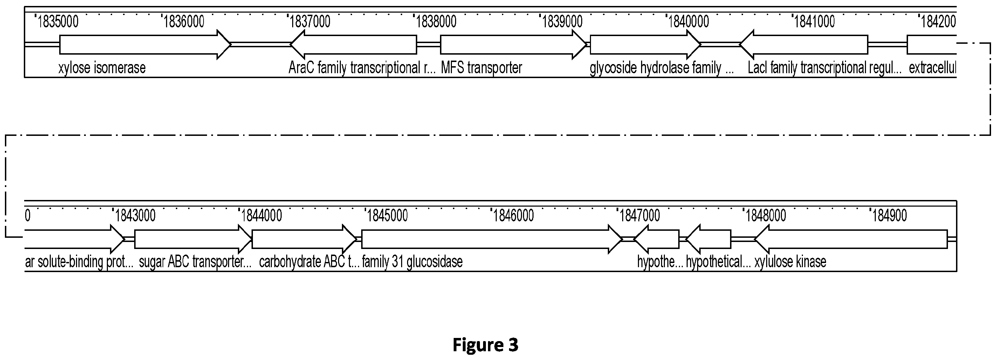

By aligning all available genomes of *B*. *longum* transitional using the BioNumerics software (v8.0, bioMérieux SA, Marcy I'Etoile, France), it was determined that the GH43_17 gene of NCC 5025 was located in a genetic region unique to that strain. This unique region contains a family 31 glucosidase (GH31; NCC5025_001581), followed by an ABC transporter (NCC5025_001580-001578), a Lac-I type regulator (NCC5025_001577), a GH43_17 enzyme (NCC5025_001576), a MFS transporter (NCC5025 _001575) and an AraC family transcriptional regulator (NCC5025_001574) (see Figure 3 and Table 2).

**Table 2 - Summary of the genes found in the GH43 17 encoding region of NCC 5025. Genome annotation was performed with the PGAP annotation pipeline available at NCBI.**

| | **LENGTH (BP)** | **ANNOTATION** |
|---|---|---|
| **NCC5025_001584** | 1521 | xylulose kinase |
| **NCC5025_001583** | 357 | hypothetical protein |
| **NCC5025_001582** | 345 | hypothetical protein |
| **NCC5025_001581** | 2064 | family 31 glucosidase |
| **NCC5025_001580** | 816 | carbohydrate ABC transporter permease |
| **NCC5025_001579** | 930 | sugar ABC transporter permease |
| **NCC5025_001578** | 1179 | extracellular solute-binding protein |
| **NCC5025_001577** | 1005 | LacI family transcriptional regulator |
| **NCC5025_001576** | 876 | glycoside hydrolase family 43 protein |
| **NCC5025_001575** | 1152 | MFS transporter |
| **NCC5025_001574** | 999 | AraC family transcriptional regulator |
| **NCC5025_001573** | 1350 | xylose isomerase |

Further BLASTn analysis of all the genes contained in this region using the BioNumerics software (v8.0, bioMérieux SA, Marcy l'Etoile, France) revealed that the Lac-I type regulator (CDS_000417), the GH43_17 enzyme (CDS_000418), the MFS transporter (CDS_000419) and the AraC family transcriptional regulator (CDS_000420) had no homologues in other strains of *B. l.* subsp *juvenis*. Homologues with relatively low degree of similarity (max 80% coverage / 60% identity) to the family 31 glucosidase (GH31; CDS000413) and the ABC transporter (CDS_000414-00416) encoding genes were found in the three closely related isolates JDM301, BXY01 and CMCC P001.

### Example 5 - NCC 5025 has a high growth rate on 3-FL

*B. longum* transitional strains were retrieved from the Nestlé Culture Collection and were reactivated from a freeze-dried stock, using two successive culturing steps (16h, 37°C, anaerobiosis) in MRS supplemented with 0.05 % cysteine (MRSc). Reactivated cultures were then centrifuged, washed and resuspended in 1 volume of PBS. Washed cells were used to inoculate MRS based medium without a carbon source (MRSc-C) (10 g L-1 of bacto proteose peptone n°3, 5 g L-1 bacto yeast extract, 1 g L-1 Tween 80, 2 g L-1 di-ammonium hydrogen citrate, 5 g L-1 sodium acetate, 0.1 g L-1 magnesium sulphate, 0.05 g L-1 manganese sulfate, 2 g L-1 di-sodium phosphate, 0.5 g L-1 cysteine) in which 3-FL was added as unique carbon source and at a final concentration of 0.5%. Growth was then performed in a 96 well microplate, with a volume of 200 µl per well. Incubation was performed in anaerobiosis for 46h, and optical density was measured over this period in a spectrophotometer at 580 nm. The growth curve was then modelled using a logistic growth model, to obtain the relative growth rate k for each variant.

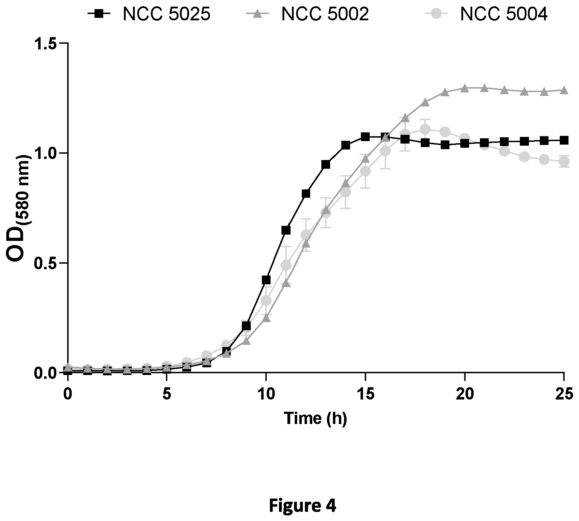

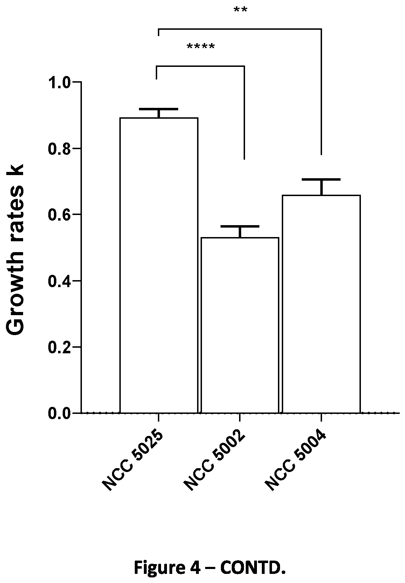

Amongst all *B. longum* transitional strains tested, NCC 5025 has the highest growth rate on 3FL, indicating that this strain is the best adapted to this substrate (see Figure 4). It has been shown that 3-FL is the human milk oligosaccharide that shows the greatest increase in the human breast milk during the period of transition between milk-based diet and solid food (Plows, J.F., et al., Longitudinal Changes in Human Milk Oligosaccharides (HMOs) Over the Course of 24 Months of Lactation. J Nutr, 2021. 151(4): p. 876-882), hence these results show an advantage of NCC 5025 for an application during this period.

### Example 6 - growth of NCC 5025 on high molecular weight food fibers

The inventors tested if the NCC 5025 strain had the capacity to grow on related high molecular weight fibers. For that purpose, selected *B. longum* transitional strains (NCC 5002, NCC 5004, NCC 5025, respectively) were grown on the above mentioned MRSc medium without sugar, to which 5 g/L% of arabinan (arabinan from Sugar-beet pulp from Megazyme) or Inulin (Orafti HSI from Beneo,) was added. Growth assays were performed in a BioLector XT microbioreactor system (m2p-labs GmbH, Baesweiler, Germany), using 48 flowerplate inserted in an anaerobic chamber for 50h (2ml volume per well, agitation at 600 rpm, CO₂ atmosphere, 37°C). Growth was followed over time by continuous measurement of the scattered light at 620 nm.

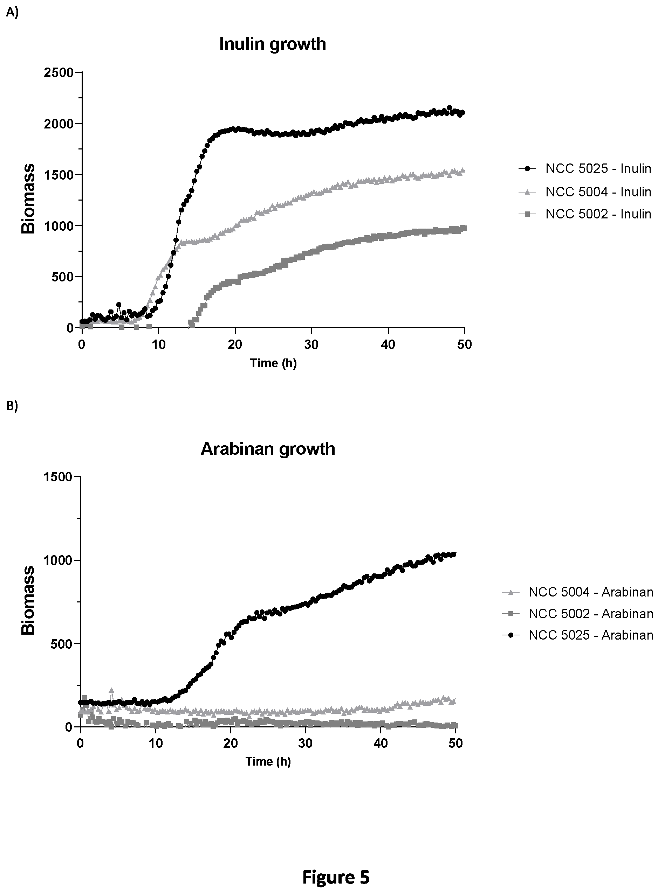

Surprisingly, results demonstrated that amongst the tested strains, *B. longum* transitional NCC 5025 had a particular ability to grow on inulin (average size of DP6-8, Tsatsaragkou et al.; Foods 2021, 10(5), 951) and high molecular weight arabinan. As compared to other *B. longum* transitional NCC 5002 and NCC 5004, *B. longum* transitional NCC 5025 grew faster (faster doubling time) and to a higher final yield. On the high molecular weight arabinan, amongst all tested strains, the NCC 5025 strain was the only to grow (see Figure 5).

### Conclusions

The data provided demonstrate that:
*a) B. longum* transitional NCC 5025 is clearly distinguished from previously isolated *B. I. juvenis* strains, and shares 98.4% ANI to the strains previously isolated from Bangladeshi infants (Vatanen et al. 2022; as above);
b) This is the only *B. longum* transitional strain to date to be free of antibiotic resistance to the set of antibiotics considered relevant by EFSA;
*c) B. longum* transitional NCC 5025 has a unique Carbohydrate Active EnZyme (CaZy) profile, including the presence of a GH43 subfamily 17 enzyme, that was not characterized to date in the *B. longum* species.
*d) B. longum* transitional NCC 5025 grows particularly well on 3-FL;
*e) B. longum* transitional NCC 5025 grows the well on a set of food derived fibers (e.g. inulin and arabinan).

Overall, the data suggest that this strain is particularly adapted to the weaning period and may perform in this environment better than other *B. longum* transitional strains. As well, our data suggest that on a diet containing food derived fiber (e.g. in adulthood), this strain may as well perform better than other *B. longum* transitional strains.

Although the invention has been described by way of example, it should be appreciated that variations and modifications may be made without departing from the scope of the invention as defined in the claims. Furthermore, where known equivalents exist to specific features, such equivalents are incorporated as if specifically referred in this specification.

## Claims

1. A *Bifidobacterium longum* transitional microorganism strain deposited with Collection nationale de cultures de micro-organismes (CNCM) under deposit number CNCM I-5942 or a *B. longum* transitional strain having an identifying characteristic of the *B. longum* transitional strain deposited under deposit number CNCM I-5942.

2. A *B. longum* transitional microorganism strain which has an Average Nucleotide Identity (ANI) of at least 99% to a *B. longum* strain deposited with the CNCM under deposit number CNCM I-5942.

3. A *B. longum* transitional strain according to claim 1 which has an ANI of at least 98.1% to a *B. longum* transitional strain deposited under deposit number CNCM I-5942.

4. A *B. longum* transitional strain according to any preceding claim wherein the *B. longum* transitional strain is not resistant to any one of tetracycline, erythromycin, clindamycin and ampicillin; preferably wherein the *B. longum* transitional strain is not resistant to any of tetracycline, erythromycin, clindamycin, ampicillin, gentamycin, streptomycin, chloramphenicol and vancomycin.

5. A *B. longum* transitional strain according to any preceding claim wherein the *B. Longum* transitional strain comprises a glycosyl hydrolase family 43_17 (GH43_17) gene; suitably wherein the GH43_17 gene comprises SEQ ID NO: 7 or a sequence with at least 60% sequence identity to SEQ ID NO: 7.

6. A *B. longum* transitional strain according to any preceding claim, which further comprises a major facilitator superfamily (MFS) gene; suitably wherein the MFS gene comprises SEQ ID NO: 37 or a sequence with at least 60% sequence identity to SEQ ID NO: 39 and/or an AraC gene; suitably wherein the AraC gene comprises SEQ ID NO: 39 or a sequence with at least 60% sequence identity to SEQ ID NO: 39; preferably wherein the GH43_17, MFS and AraC genes are comprised in a gene cluster.

7. A *B. longum* transitional strain according to claim 6, wherein the *B. longum* transitional strain further comprises one or more of a GH31 gene and a LacI gene; preferably wherein the *B. longum* transitional strain further comprises a xylulose kinase gene and a xylose isomerase gene; and further preferably wherein the GH43_17, MFS, AraC, GH31, LacI, xylulose kinase and xylose isomerase genes are comprised in a gene cluster.

8. A *B. longum* transitional strain according to any preceding claim, which further comprises one or more genes encoding for one or more glycoside hydrolases selected from GH43_17, GH43_22, GH43_27, GH43_29, GH121, GH43_24, GH127, GH30_5, GH 43_32 and GH30.

9. A *B. Longum* transitional strain according to any preceding claim wherein the *B. Longum* transitional strain preferentially utilizes 3-fucosyllactose (3-FL); preferably wherein the *B. longum* transitional strain has a growth rate of at least 0.6 k when cultured in the presence of 3-FL.

10. A probiotic composition comprising a *B. longum* transitional strain according to any preceding claim.

11. A composition comprising a *B. longum* transitional strain according to any preceding claim and at least one further probiotic and/or prebiotic.

12. Use of a *B. longum* transitional strain or a composition according to any preceding claim as a dietary supplement; suitably wherein the *B. longum* transitional strain or composition is administered to a subject in combination with a fiber containing diet or fiber containing foodstuffs.

13. Use of a *B. longum* transitional strain or a composition according to any of claims 1 to 9 to promote and/or assist the metabolism of a fiber containing diet or fiber containing foodstuffs by a subject.

14. Use of a *B. longum* transitional strain or a composition according to any of claims 1 to 9 to promote and/or assist the transition from a milk-based diet to solid food in an infant and/or in a young child.
